# EUROPEAN PATENT SPECIFICATION

(11) **EP 4 712 997 B1**
(45) Date of publication and mention of the grant of the patent: **18.11.2026**
(21) Application number: 24728188.4
(22) Date of filing: 17.05.2024
(51) Int. Cl.: A61K 38/17, A61P 27/02

(54) **ANNEXIN A5 PROTEIN AS A COMBINATION THERAPY WITH AN ANTI-VEGF AGENT FOR USE IN THE TREATMENT OF MACULAR OEDEMA OR RETINAL VEIN OCCLUSION**
ANNEXIN-A5-PROTEIN ALS KOMBINATIONSTHERAPIE MIT EINEM ANTI-VEGF-MITTEL ZUR VERWENDUNG BEI DER BEHANDLUNG VON MAKULAÖDEM ODER NETZHAUTVENENOKKLUSION
ANNEXINE A5 MONOMÈRE DESTINÉE À ÊTRE UTILISÉE DANS LE TRAITEMENT D'UN OEDÈME MACULAIRE OU D'UNE OCCLUSION VEINEUSE RÉTINIENNE

(30) Priority: 18.05.2023 WO PCT/EP2023/063441
(43) Date of publication of application: 25.03.2026
(60) Divisional application: 26206422.3
(73) Proprietor: Annexin Pharmaceuticals AB, 111 60 Stockholm (SE)
(72) Inventor: FROSTEGÅRD, Anna, 111 60 Stockholm (SE); HAEGERSTRAND, Anders, 111 60 Stockholm (SE)
(74) Representative: Potter Clarkson
(86) International application number: PCT/EP2024/063794
(87) International publication number: WO 2024/236190

(56) References cited:
- EP-A1- 3 453 721
- CHOLLET P ET AL: "Inhibition of intraocular fibrin formation with annexin V.", vol. 76, no. 8, 1 August 1992 (1992-08-01), GB, pages 450 - 452, XP093090062, ISSN: 0007-1161, Retrieved from the Internet <URL:https://www.ncbi.nlm.nih.gov/pmc/articles/PMC504314/pdf/brjopthal00056-0002.pdf> DOI: 10.1136/bjo.76.8.450
- MARK M EWING ET AL: "Annexin AS Therapy Attenuates Vascular Inflammation and Remodeling and Improves Endothelial Function in Mice", ARTERIOSCLEROSIS, THROMBOSIS, AND VASCULAR BIOLOGY, HIGHWIRE PRESS, PHILADELPHIA, PA, US, vol. 31, 1 January 2011 (2011-01-01), pages 95 - 101, XP007922101, ISSN: 1524-4636, DOI: LO.1161/ATVBAHA.LL0.216747
- ANNEXIN PHARMACEUTICALS: "Positive results from Annexin Pharmaceuticals' phase-1 study - Annexin Pharmaceuticals", 18 September 2021 (2021-09-18), XP093090054, Retrieved from the Internet <URL:https://www.annexinpharma.com/en/positive-results-from-annexin-pharmaceuticals-phase-1-study/> [retrieved on 20231010]
- SACCONI RICCARDO ET AL: "Emerging therapies in the management of macular edema: a review", vol. 8, 12 August 2019 (2019-08-12), pages 1413, XP093090161, Retrieved from the Internet <URL:https://www.ncbi.nlm.nih.gov/pmc/articles/PMC6694451/pdf/f1000research-8-21038.pdf> DOI: 10.12688/f1000research.19198.1

## Description

### Field of the Invention

The invention relates to novel methods, uses and compositions for enhancing anti-VEGF therapies and/or reducing complications associated with administration of an anti-VEGF agent. Particular areas of interest include the prophylaxis or the treatment of macular oedema (also referred to herein as macular edema or ME), the prophylaxis or the treatment of retinal vein occlusion, and conditions associated with either.

### Background to the Invention

Studies of vascular endothelial-derived growth factor (VEGF) has revolutionized the understanding of vasculogenesis and angiogenesis during development and physiological homeostasis (Apte et al, 2019, Cell, 176(6): 1248-1264). The central role of VEGF in the pathogenesis blinding eye diseases has also become evident.

For example, macular oedema is the pathological accumulation of fluid in the central retina. It is a complication of many retinal diseases, including diabetic retinopathy (DR), retinal vascular occlusions (RVO) and uveitis (UV), among others (Haydinger et al, 2023, Frontiers in Medicine, 10: 1128811). Macular oedema causes decreased visual acuity and, when chronic or refractory, can cause severe and permanent visual impairment and blindness. In combination with the underlying pathology, the oedema can progress to cause irreversible tissue damage with death of retinal cells, and permanent visual impairment.

In most instances, macular oedema develops due to dysregulation of the blood-retinal barrier (BRB), a set of structures that, in health, strictly regulates the passage of proteins, salts, metabolites and other solutes between the blood and the retinal tissue. Dysregulation of the BRB permits infiltration of the retinal tissue by proteins and other solutes that are normally retained in the blood (Haydinger *et al,* 2023, *supra).* The increase in osmotic pressure in the tissue drives fluid accumulation.

Current treatments target blood-retinal barrier (BRB) dysfunction, and include vascular endothelial growth factor (VEGF) blockers, corticosteroids, and non-steroidal antiinflammatory drugs (Haydinger *et al,* 2023, *supra;* Ip & Hendrick, 2018, Asia-Pacific Journal of Ophthalmology, 7(1): 40-45). These treatments target vasoactive and inflammatory mediators that cause disruption to the blood-retinal barrier. Other treatments include targeted laser photocoagulation (Haydinger *et al,* 2023, *supra;* lp & Hendrick, 2018, *supra).*

The front-line therapy of macular oedema associated with diabetic retinopathy (DR) or retinal vascular occlusions (RVO), as well as the treatment of RVO itself, is anti-VEGF therapy, which is sometimes augmented with intravitreal corticosteroid and/or retinal laser photocoagulation (Ehlers et al. 2022, Ophthalmology, 129:88-99; Ehlers et al, 2017, Ophthalmology, 124:1412-23; and Yeh et al, 2015, Ophthalmology, 122:769-78). The treatment of macular oedema secondary to neovascular AMD also relies heavily on the use of anti-VEGF therapy (Flaxel et al, 2020, Ophthalmology, 127:P1-P65).

Sacconi et al., 2019, F1000Research, vol. 8, page 1413, is entitled "Emerging therapies in the management of macular edema*".* It notes that introduction into clinical practice of anti-vascular endothelial growth factor injections (ranibizumab and aflibercept) and dexamethasone implants has revolutionized the treatment of macular edema (ME) secondary to DR and RVO, but also notes that new drugs are needed in the treatment of resistant forms of ME secondary to DR and RVO, noting that a fluocinolone acetonide implant has been approved by the US FDA for the treatment of diabetic ME but not for RVO, and that brolucizumab and abicipar pegol have been shown to be effective in preliminary studies and have the chance to be approved soon for diabetic ME treatment. It further notes that in ME secondary to uveitis, a crucial role is played by corticosteroids and non-biologic immunomodulatory drugs.

The established standard of care for anti-VEGF therapy of macular oedema and/or RVO, and indeed in multiple retinal diseases, is by delivery of the therapeutic agents directly into the eye by intravitreal injection (Haydinger *et al,* 2023, *supra;* lp & Hendrick, 2018, *supra;* Cox et al., 2021, J. Clin. Med., 10(5): 981; Hattenbach et al., 2023, Ophthalmol Sci., 3(3): 100302). Such medications are injected directly into the eye because the retina is located at the back of the eye and is surrounded by several protective layers, including the cornea, the iris, and the lens. These layers can make it difficult for medications to reach the retina when they are administered by other routes. When medications are injected into the eye, they are delivered directly to the retina and can act locally to target the source of the problem. For example, anti-VEGF medications are injected into the eye to block the action of VEGF-A, a protein that promotes the growth of abnormal blood vessels in the retina. By reducing the levels of VEGF-A, these medications can help to prevent the formation of new blood vessels and reduce inflammation and swelling in the eye. Injections into the eye are typically performed in an ophthalmologist's office or an outpatient surgical center. After the injection, patients are typically monitored for a short period to ensure that there are no adverse effects from the medication.

Targeting VEGF with intravitreal injections of monoclonal antibodies or high-affinity decoy receptors are effective means of treating most diseases complicated by macular oedema, including wet age-related macular degeneration (Heier et al., 2012, Ophthalmology, 119:2537-48), diabetic macula oedema (Nguyen et al., 2012, Ophthalmology, 119:789-801; Korobelnik et al, 2014, Ophthalmology, 121:2247-54), and macular oedema caused by central RVO (CRVO) (Brown et al., 2010, Ophthalmology, 117:1124-33), branch RVO (BRVO) (Campochiaro et al., 2010, Ophthalmology, 117:1102-12) and uveitis (Mackensen et al., 2008, Retina, 28:41-5).

However, there are concerns of ocular and systemic toxicity in anti-VEGF therapy, and long-term frequent reinjection is required (Nguyen et al., 2010, Ophthalmology, 117:2146-51; Campochiaro et al., 2010, Ophthalmology, 117:2387-94; Saint-Geniez et al, 2008, PLoS One., 3:e3554). Potential complications associated with intravitreal injections of anti-VEGF therapy include infectious endophthalmitis, sterile intraocular inflammation, and retinal vasculitis particularly with brolucizumab (Cox *et al.,* 2021, *supra)* and transient intraocular pressure (IOP) spikes (Levin et al., 2021, J Glaucoma, 30:1019-26).

Infectious endophthalmitis remains one of the most devastating complications associated with anti-VEGF therapy. The reported incidence post-injection varies from 0.008 to 0.092%, and despite its low incidence, the high volume of repeated anti-VEGF injections means that they account for a large proportion of infectious endophthalmitis cases (Cox *et* al., 2021, *supra).*

Sterile Intraocular Inflammation (SII), also termed pseudoendophthalmitis, is characterized by acute-onset intraocular inflammation without infection that resolves without antibiotic treatment. The reported incidence of anti-VEGF associated SII varies by study from 0.02 to 0.37%, wherein presentation usually occurs between 24 h and seven days from the inciting injection. Symptoms can consist of blurred vision, floaters, pain, and photophobia. Pain is present in up to 46% of patients, and its presence is significantly associated with severe vitreous or anterior chamber (AC) inflammation. Photophobia occurs in 4 to 19% of patients. Additionally, visual acuity is frequently substantially diminished from its baseline at presentation, but it often returns to pre-injection levels after the inflammation resolves. Intraocular inflammation is present on exam in virtually every case. This may consist of vitritis, AC reaction, or most commonly, both Additional, less common exam findings include hypopyon, fibrin, keratic precipitates, corneal oedema, conjunctival injection, and chemosis (Cox *et al.,* 2021, *supra).*

Serial anti-VEGF therapy by intravitreal injection is the current standard of care for treatment of macular oedema (Ip & Hendrick, 2018, *supra).* Hattenbach *et al.,* 2023, *supra,* reports that although efficacious, some patients with RVO require frequent long-term injections and monitoring to maintain the gains achieved during initial intensive treatment. For example, a well established approach is for monthly intravitreal injections of anti-VEGF, repeated over the course of several months or over several years (Apte et al., 2019, *supra).*

However, Hattenbach *et al.,* 2023, *supra,* reports that, in contrast to the clinical trial findings, data from clinical-setting studies suggest many patients with RVO (both BRVO and CRVO) do not achieve the gains observed in clinical trials, at least partially due to suboptimal injection frequency with anti-VEGF; and that in a recent analysis, patients with CRVO from clinical setting studies were found, on average, to be monitored less frequently, to receive fewer anti-VEGF injections, and to not have achieved the vision gains observed in patients with CRVO from clinical trials. This decreased effectiveness in clinical settings is reported to be likely to be a reflection of the high burden associated with RVO treatment (and consequent decreased adherence), including the need for regular anti-VEGF injections and appointments, and the associated costs.

Nevertheless, it is recognised that indications for intravitreal injections are expanding, with more and more patients likely to be impacted by the acute and chronic effects of intravitreal injections (Levin et al., 2021, *supra).* However, no treatment to date for macular oedema or RVO has demonstrated reliable methods for directly improving the perfusion. Instead, current strategies focus on minimizing fallout from the consequences of RVO to minimize vision loss from macular oedema and neovascularization (Ip & Hendrick, 2018, *supra).*

Apte et al., 2019, *supra* reports that therapeutic approaches that complement VEGF-based strategies are of high interest and relevance, with a desired goal of multitarget approaches to find a combination that can reduce the high treatment burden of frequent intraocular injections, improve visual outcomes, and prevent continued vision loss from atrophic neurodegeneration. However, Apte et al., 2019, *supra* reports that several strategies that have targeted novel pathways have so far failed to demonstrate efficacy. Likewise, Hattenbach *et al.,* 2023, *supra,* reports that there is an unmet need for more efficacious and durable treatment options for RVO to decrease the treatment burden without visual compromise.

It is against this background that there is a need in the art for improved therapies for macular oedema, and associated conditions, including RVO. In particular, there is a need for effective therapies that can be effectively administered to a subject without the requirement for direct injection (in particular, multiple repeated injections) into the eye(s) of the subject.

### Summary of the invention

Surprisingly, against this background, it has been determined that Annexin A5 is effective in enhancing forms of anti-VEGF therapies, and in reducing the burden for repeated anti-VEGF administrations, thereby providing opportunities to reduce complications and/or side-effects associated with the repeated administration of anti-VEGF therapeutics. Moreover, by reducing the requirement of repeated anti-VEGF administrations, the opportunity for a subject to acquire reduced response and/or resistance to ongoing anti-VEGF therapeutic regimes may be reduced.

Additionally, the applicants have determined that, when administered to a subject systemically (such as by intravenous administration), the Annexin A5 protein is able to preferentially localise to the site of an occlusion in a subject with retinal vein occlusion (RVO). It has also been determined that Annexin A5 provides biological activity that modulates the factors contributing to dysregulation of the blood-retinal barrier (BRB) in subjects with macular oedema and/or RVO, as well as contributing other therapeutic benefits in subjects with macular oedema and/or RVO.

Accordingly, in a first aspect of the present invention, there is provided molecule comprising, or consisting of, Annexin A5 protein for use in the prophylaxis or the treatment of macular oedema, or a condition associated therewith, in a subject,
wherein the use is as a combination therapy with an anti-vascular endothelial growth factor (anti-VEGF) agent; and
wherein the condition associated with macular oedema is selected from the group consisting of: retinal vascular occlusion (RVO), dysregulation of the blood-retinal barrier (BRB), such as dysregulation of the inner BRB and/or the outer BRB, dysfunction of barriers between the vitreous and blood vessels at the ciliary body, macular edema-associated ocular diseases, selected from the group consisting of diabetic retinopathy, uveitis, post-cataract surgery inflammation (pseudophakic macular edema or Irvine-Gass syndrome), retinal dystrophy, drug reactions, intraocular tumors, serous central chorioretinopathy, radiation retinopathy and other retinal vascular abnormalities including retinal arterial macroaneurysms and retinal telangiectasia, changes to subretinal neovascular membranes observed in conditions selected from the group consisting of age-related macular degeneration (AMD), metamorphopsia, micropsia, blurred vision, central scotoma and reduction of contrast or colour sensitivity. Optionally, the molecule consists of monomeric Annexin A5.

In an alternative embodiment of the first aspect of the present invention, there is provided an anti-VEGF agent for use in the prophylaxis or the treatment of macular oedema, or a condition associated therewith, in a subject,
wherein the use is as a combination therapy with a molecule comprising, or consisting of, Annexin A5 protein; and
wherein the condition associated with macular oedema is selected from the group consisting of: retinal vascular occlusion (RVO), dysregulation of the blood-retinal barrier (BRB), such as dysregulation of the inner BRB and/or the outer BRB, dysfunction of barriers between the vitreous and blood vessels at the ciliary body, macular edema-associated ocular diseases, selected from the group consisting of diabetic retinopathy, uveitis, post-cataract surgery inflammation (pseudophakic macular edema or Irvine-Gass syndrome), retinal dystrophy, drug reactions, intraocular tumors, serous central chorioretinopathy, radiation retinopathy and other retinal vascular abnormalities including retinal arterial macroaneurysms and retinal telangiectasia, changes to subretinal neovascular membranes observed in conditions selected from the group consisting of age-related macular degeneration (AMD), metamorphopsia, micropsia, blurred vision, central scotoma and reduction of contrast or colour sensitivity, optionally, wherein the molecule consists of monomeric Annexin A5.

In some embodiments of the first aspect of the present invention, the molecule comprising, or consisting of, Annexin A5 protein or the composition comprising said molecule, is presented in the same composition as the anti-VEGF agent.

In other embodiments of the first aspect of the present invention, the molecule comprising, or consisting of, Annexin A5 protein or the composition comprising said molecule is formulated separately from the anti-VEGF agent. In that case, the anti-VEGF and Annexin A5 components may be administered to the subject simultaneously or sequentially.

In the case that the molecule comprising, or consisting of, Annexin A5 protein (or the composition comprising said molecule) is formulated separately from the anti-VEGF agent, the anti-VEGF agent may be administered directly to the eye. For example, the anti-VEGF agent may be administered by intravitreal injection.

In the case of sequential administration of the Annexin A5 component and the anti-VEGF component then, in some instances, the molecule comprising, or consisting of, Annexin A5 protein ("the Annexin A5 component") is administered to the subject, as a first round of administration of the Annexin A5 component, prior to the commencement of anti-VEGF therapy. In other instances, the molecule comprising, or consisting of, Annexin A5 protein may be administered to the subject, as a first round of administration of the Annexin A5 component, after the anti-VEGF therapy has commenced, but typically prior to the end of the anti-VEGF therapy. For example in the case of a form of anti-VEGF therapy that (in the absence of Annexin A5 co-therapy) would require multiple rounds of anti-VEGF administration for the prophylaxis or treatment of macular oedema, or the condition associated therewith, in the subject, the Annexin A5 component may be administered to the subject, as a first round of administration of the Annexin A5 component, prior to the commencement of anti-VEGF therapy, or after the anti-VEGF therapy has commenced but before any one or more of the subsequent multiple round of administration of the anti-VEGF component.

In some embodiments of the first aspect of the present invention, the subject may also receive a second round of administration of the of the Annexin A5 component. In some instances, the Annexin A5 component is administered to the subject, as a first round of administration of the Annexin A5 component, and subsequently as a second round of administration of the Annexin A5 component, prior to the commencement of anti-VEGF therapy. In other instances, the Annexin A5 component is administered to the subject, as a first round of administration of the Annexin A5 component prior to the commencement of anti-VEGF therapy, and the Annexin A5 component is administered to the subject, as a second round of administration of the Annexin A5, after the anti-VEGF therapy has commenced, but typically prior to the end of the anti-VEGF therapy. In other instances, the Annexin A5 component is administered to the subject, as a first round of administration of the Annexin A5 component, and subsequently as a second round of administration of the Annexin A5 component, wherein both of the first and second rounds of administration of the Annexin A5 component occurs after the anti-VEGF therapy has commenced, but typically prior to the end of the anti-VEGF therapy. In some options, there are no rounds of anti-VEGF therapy between the first and second rounds of administration of the Annexin A5 component. In other options, there may be a single round of anti-VEGF therapy between the first and second rounds of administration of the Annexin A5 component. In other options, there may be more than one round of anti-VEGF therapy between the first and second rounds of administration of the Annexin A5 component.

In some embodiments of the first aspect of the present invention, the subject may also receive a third round of administration of the of the Annexin A5 component. In some instances, the Annexin A5 component is administered to the subject, as a first round of administration of the Annexin A5 component, and subsequently as a second round of administration of the Annexin A5 component, and further subsequently as a third round of administration of the Annexin A5 component, all prior to the commencement of anti-VEGF therapy. In other instances, the Annexin A5 component is administered to the subject, as a first round of administration of the Annexin A5 component, and subsequently as a second round of administration of the Annexin A5 component, both prior to the commencement of anti-VEGF therapy, and the Annexin A5 component is administered to the subject, as a third round of administration of the Annexin A5, after the anti-VEGF therapy has commenced, but typically prior to the end of the anti-VEGF therapy. In other instances, the Annexin A5 component is administered to the subject, as a first round of administration of the Annexin A5 component prior to the commencement of anti-VEGF therapy, and the Annexin A5 component is administered to the subject, as a second round of administration of the Annexin A5, and further subsequently as a third round of administration of the Annexin A5 component, where both the second and third rounds of administration occur after the anti-VEGF therapy has commenced, but typically prior to the end of the anti-VEGF therapy. In other instances, the Annexin A5 component is administered to the subject, as a first round of administration of the Annexin A5 component, and subsequently as a second round of administration of the Annexin A5 component, and further subsequently as a third round of administration, wherein all three of the first, second and third rounds of administration of the Annexin A5 component occurs after the anti-VEGF therapy has commenced, but typically prior to the end of the anti-VEGF therapy. In some options, there are no rounds of anti-VEGF therapy between the first and second rounds of administration of the Annexin A5 component and/or between the second and third rounds of administration of the Annexin A5 component. In other options, there may be a single round of anti-VEGF therapy between the first and second rounds of administration of the Annexin A5 component and/or between the second and third rounds of administration of the Annexin A5 component. In other options, there may be more than one round of anti-VEGF therapy between the first and second rounds of administration of the Annexin A5 component and/or between the second and third rounds of administration of the Annexin A5 component.

Although described above with respect to one, two, or three rounds of administration of the Annexin A5 component, it will be appreciated that one or more additional rounds of administration of the Annexin A5 component may be used beyond the third round. In some instances, all rounds of administration of the Annexin A5 component occur prior to the commencement of anti-VEGF therapy. In other instances, any of the first, second, third or any subsequent round of administration of the Annexin A5 component may occur prior to the commencement of anti-VEGF therapy, and one or more subsequent rounds of administration the Annexin A5 component may administered to the subject after the anti-VEGF therapy has commenced, but typically prior to the end of the anti-VEGF therapy. In other instances, rounds of administration of the Annexin A5 component occur after the anti-VEGF therapy has commenced, but typically prior to the end of the anti-VEGF therapy. In some options, there are no rounds of anti-VEGF therapy between the first and second rounds of administration of the Annexin A5 component, between the second and third rounds of administration of the Annexin A5 component, between the third round and a subsequent round of administration of the Annexin A5 component and/or between any of the multiple subsequent rounds of administration of the Annexin A5 component. In other options, there may be a single round of anti-VEGF therapy between any one or more of the first and second rounds of administration of the Annexin A5 component, the second and third rounds of administration of the Annexin A5 component, the third round and a subsequent round of administration of the Annexin A5 component and/or any of the multiple subsequent rounds of administration of the Annexin A5 component. In other options, there may be more than one round of anti-VEGF therapy between any one or more of the first and second rounds of administration of the Annexin A5 component, the second and third rounds of administration of the Annexin A5 component, the third round and a subsequent round of administration of the Annexin A5 component and/or any of the multiple subsequent rounds of administration of the Annexin A5 component.

The subject to whom the co-therapy is applied in accordance with the first aspect of the present invention is preferably observably responsive to the first, and/or any subsequent, round of administration of the Annexin A5 component part of the co-therapy. That is to say, it is preferred that the subject demonstrates an observable reduction in worsening, stabilisation, or improvement in the condition of the macular oedema, and/or condition associated therewith, as a result of any one or more of any of the rounds of administration of the Annexin A5 component of the co-therapy. Such an observable reduction in worsening, stabilisation, or improvement may, for example, be in respect of any one, two, three or four of the following characteristics:
i. visual acuity, optionally assessed by Best-Corrected Visual Acuity (BCVA) test;
ii. macular retinal thickness;
iii. macular volume; and
iv. Retinal Area of Non-Perfusion (RANP).

Typically, the (or each) observable reduction in worsening, stabilisation, or improvement may be observable, and/or observed, before the subject receives a subsequent administration of anti-VEGF agent.

As a consequence of the administration of the Annexin A5 component in the first, and/or any subsequent round of administration of the Annexin A5 component, the frequency and/or number of subsequent rounds of administration of the anti-VEGF component that follow the initial administration of the anti-VEGF component to the subject can be reduced, or even entirely avoided, for a defined period without substantially reducing the prophylactic or therapeutic effectiveness of the anti-VEGF therapy in respect of the macular oedema, or a condition associated therewith. This may be compared, for example, to a suitable control such as a subject, or cohort of subjects, who has/have not been treated with a molecule comprising, or consisting of, Annexin A5 protein, but who receive(s) multiple administrations of the anti-VEGF component (such as successive administrations approximately every 28 days (e.g. ± 7, 6, 5, 4, 3, 2, or 1 days) for the same defined period. The defined period may, for example, be about, up to, or at least 2, 3, 4, 5, 6, 7, 8, 9, 10, or 11 months, or about, up to, or at least 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period. The defined period may be until the end of the subject's therapeutic or prophylactic need. A cohort, can in some embodiments, be two or more subjects, as such about, at least, or greater than 3, 4, 5, 10, 15, 20, 25 30, 40, 50 60, 70, 80 90 or 100 subjects.

The prophylactic or therapeutic effectiveness of the anti-VEGF therapy in respect of the macular oedema, or a condition associated therewith, can be assessed with respect to any one, two, three or four of the following characteristics:
v. visual acuity, optionally assessed by Best-Corrected Visual Acuity (BCVA) test;
vi. macular retinal thickness;
vii. macular volume; and
viii. Retinal Area of Non-Perfusion (RANP).

In the embodiment of the first aspect of the present invention in which the molecule comprising, or consisting of, Annexin A5 protein is administered to the subject prior to the commencement of anti-VEGF therapy, a reduced frequency of administration of the anti-VEGF component that follows the initial administration of the Annexin A5 component may, for example, refer to a period of time between the initial administration of the Annexin A5 component to the subject and a first administration of the anti-VEGF component to the subject, that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years, provided that the subject receives at least one administration of the anti-VEGF component during the therapy. In this context, the term "about" may refer to a period that ± 50%, 40%, 30%, 20%, 10%, 5%, 4%, 3%, 2%, 1% or less than 1% of the stated time period.

For example, in accordance with the first aspect of the present invention, and following the initial round of administration of the Annexin A5 component (and/or any subsequent round of administration of the Annexin A5 component) to the subject, the anti-VEGF component may be subsequently administered, to the subject:
i. fewer than 12, 11, 10, 9, 8, 7, 6, 5, 4, 3, 2 or 1 times within a period of twelve months from the start of treatment with the Annexin A5 molecule or composition;
ii. fewer than 10, 9, 8, 7, 6, 5, 4, 3, 2 or 1 times within a period of ten months from the start of treatment with the Annexin A5 molecule or composition;
iii. fewer than 8, 7, 6, 5, 4, 3, 2 or 1 times within a period of eight months from the start of treatment with the Annexin A5 molecule or composition; and/or
iv. fewer than 6, 5, 4, 3, 2 or 1 times within a period of six months from the start of treatment with the Annexin A5 molecule or composition.

Additionally or alternatively, a reduced frequency of the subsequent rounds of administration of the anti-VEGF component that follow the initial administration of the anti-VEGF component, in the subject that receives co-therapy with Annexin A5 in accordance with the first aspect of the present invention, may, for example, refer to a period of time between the initial administration of the anti-VEGF component to the subject and a second administration of the anti-VEGF component to the subject that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period.

Additionally, or alternatively, a reduced frequency of the subsequent rounds of administration of the anti-VEGF component that follow the initial administration of the anti-VEGF component, in the subject that receives co-therapy with Annexin A5 in accordance with the first aspect of the present invention, may, for example, refer to a period of time between a second round and/or any subsequent round of administration of the anti-VEGF component, and the round of anti-VEGF administration that follows it, that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period.

For example, in accordance with the first aspect of the present invention, and following the initial administration of the anti-VEGF component to the subject, the anti-VEGF agent may be subsequently administered, to the subject that receives co-therapy with Annexin A5:
i. fewer than 12, 11, 10, 9, 8, 7, 6, 5, 4, 3, 2 or 1 times within a period of twelve months from the start of treatment with the Annexin A5 molecule or composition;
ii. fewer than 10, 9, 8, 7, 6, 5, 4, 3, 2 or 1 times within a period of ten months from the start of treatment with the Annexin A5 molecule or composition;
iii. fewer than 8, 7, 6, 5, 4, 3, 2 or 1 times within a period of eight months from the start of treatment with the Annexin A5 molecule or composition; and/or
iv. fewer than 6, 5, 4, 3, 2 or 1 times within a period of six months from the start of treatment with the Annexin A5 molecule or composition.

Preferably, in accordance with the first aspect of the present invention, the molecule comprising, or consisting of, Annexin A5 protein (such as a molecule consisting of monomeric Annexin A5) is administered to the subject systemically, preferably intravenously, subcutaneously or intramuscularly, or may be administered by suprachoroidal injection (e.g. as discussed in Wu *et al,* 2023, *Pharmaceuticals,* **16**(9): 1241). Where it is administered intravenously, then the molecule comprising, or consisting of, Annexin A5 protein, or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2. In this context, the term "about" when applied to a pH value that is stated to the nearest integer may refer to a value that is ± 0.5, 0.4, 0.3, 0.2, or 0.1 pH units of the stated value; whereas when the term "about" is applied to a pH value that is stated to one decimal place, then it may refer to a value that is ± 0.05, 0.04, 0.03, 0.02, or 0.01 pH units of the stated value.

Preferably, in accordance with the first aspect of the present invention, for each round of administration of the Annexin A5 component to the subject, a dose of the molecule comprising, or consisting of, the Annexin A5 protein is administered to the subject for two consecutive days or more, such as for three, four, five, six, seven or more consecutive days, and/or or for at least two, three, four, five six or seven days within each 7-day (one week) of 8-day period, such as for a period of at least one, two, three, four weeks or more.

Optionally, in accordance with the first aspect of the present invention, for each round of administration of the Annexin A5 component to the subject, the daily dose of the molecule comprising, or consisting of, Annexin A5 protein (such as a molecule consisting of monomeric Annexin A5) that is administered to the subject is: a) a total daily dose of about 5 mg, about 4 mg, about 3mg, about 2mg, or less than about 2 mg, 0.1 to 1.5 mg or about 1 mg; and/or b) within a range selected from the group consisting of about 50-100 µg, about 40-80 µg, about 30-60 µg, about 20-40 µg, 1 µg to 30 µg per kg, or less than about 20 µg, per kg body weight of the subject. The term "about" as used herein may be used herein to mean a range of ±50%, ±40%, ±30%, ±20%, ±10%, ±5%, ±4%, ±3%, ±2% or ±1% of the value mentioned.

In a second aspect of the present invention, there is provided a molecule comprising, or consisting of, Annexin A5 protein for use in the prophylaxis or the treatment of retinal vein occlusion (RVO), or a condition associated therewith, in a subject,
wherein said condition associated with RVO is selected from the group consisting of macular oedema, retinal ischemia, iris neovascularization (optionally, iris neovascularization characterised by an increase in cytokines, such as an increase in VEGF), retinal neovascularization (optionally, retinal neovascularization characterised by an increase in cytokines, such as an increase in VEGF), neovascular glaucoma, vitreous haemorrhage, rubeosis, and retinal detachment,
wherein the use is as a combination therapy with an anti-vascular endothelial growth factor (anti-VEGF) agent, and
wherein a dose of the molecule comprising, or consisting of, Annexin A5 protein is administered to the subject systemically or by suprachoroidal injection, daily for two or more consecutive days. Optionally, the molecule consists of monomeric Annexin A5.

In an alternative embodiment of the second aspect of the present invention, there is provided an anti-VEGF agent for use in the prophylaxis or the treatment of retinal vein occlusion (RVO), or a condition associated therewith, in a subject,
wherein said condition associated with RVO is selected from the group consisting of macular oedema, retinal ischemia, iris neovascularization (optionally, iris neovascularization characterised by an increase in cytokines, such as an increase in VEGF), retinal neovascularization (optionally, retinal neovascularization characterised by an increase in cytokines, such as an increase in VEGF), neovascular glaucoma, vitreous haemorrhage, rubeosis, and retinal detachment,
wherein the use is as a combination therapy with a molecule comprising, or consisting of, Annexin A5 protein, and
wherein a dose of the molecule comprising, or consisting of, Annexin A5 protein is administered to the subject systemically or by suprachoroidal injection, daily for two or more consecutive days. Optionally, the molecule consists of monomeric Annexin A5.

In some embodiments of the second aspect of the present invention, the molecule comprising, or consisting of, Annexin A5 protein or the composition comprising said molecule, is presented in the same composition as the anti-VEGF agent.

In other embodiments of the second aspect of the present invention, the molecule comprising, or consisting of, Annexin A5 protein or the composition comprising said molecule is formulated separately from the anti-VEGF agent. In that case, the anti-VEGF and Annexin A5 components may be administered to the subject simultaneously or sequentially.

In the case that the molecule comprising, or consisting of, Annexin A5 protein (or the composition comprising said molecule) is formulated separately from the anti-VEGF agent, the anti-VEGF agent may be administered directly to the eye. For example, the anti-VEGF agent may be administered by intravitreal injection.

In the case of sequential administration, in some instances of the Annexin A5 component and the anti-VEGF component then, the molecule comprising, or consisting of, Annexin A5 protein ("the Annexin A5 component") is administered to the subject, as a first round of administration of the Annexin A5 component, prior to the commencement of anti-VEGF therapy. In other instances, the molecule comprising, or consisting of, Annexin A5 protein may be administered to the subject, as a first round of administration of the Annexin A5 component, after the anti-VEGF therapy has commenced, but typically prior to the end of the anti-VEGF therapy. For example in the case of a form of anti-VEGF therapy that (in the absence of Annexin A5 co-therapy) would require multiple rounds of anti-VEGF administration for the prophylaxis or treatment of RVO, or a condition associated therewith, in the subject, the Annexin A5 component may be administered to the subject, as a first round of administration of the Annexin A5 component, prior to the commencement of anti-VEGF therapy, or after the anti-VEGF therapy has commenced but before any one or more of the subsequent multiple round of administration of the anti-VEGF component.

In some embodiments of the second aspect of the present invention, the subject may also receive a second round of administration of the of the Annexin A5 component. In some instances, the Annexin A5 component is administered to the subject, as a first round of administration of the Annexin A5 component, and subsequently as a second round of administration of the Annexin A5 component, prior to the commencement of anti-VEGF therapy. In other instances, the Annexin A5 component is administered to the subject, as a first round of administration of the Annexin A5 component prior to the commencement of anti-VEGF therapy, and the Annexin A5 component is administered to the subject, as a second round of administration of the Annexin A5, after the anti-VEGF therapy has commenced, but typically prior to the end of the anti-VEGF therapy. In other instances, the Annexin A5 component is administered to the subject, as a first round of administration of the Annexin A5 component, and subsequently as a second round of administration of the Annexin A5 component, wherein both of the first and second rounds of administration of the Annexin A5 component occurs after the anti-VEGF therapy has commenced, but typically prior to the end of the anti-VEGF therapy. In some options, there are no rounds of anti-VEGF therapy between the first and second rounds of administration of the Annexin A5 component. In other options, there may be a single round of anti-VEGF therapy between the first and second rounds of administration of the Annexin A5 component. In other options, there may be more than one round of anti-VEGF therapy between the first and second rounds of administration of the Annexin A5 component.

In some embodiments of the second aspect of the present invention, the subject may also receive a third round of administration of the of the Annexin A5 component. In some instances, the Annexin A5 component is administered to the subject, as a first round of administration of the Annexin A5 component, and subsequently as a second round of administration of the Annexin A5 component, and further subsequently as a third round of administration of the Annexin A5 component, all prior to the commencement of anti-VEGF therapy. In other instances, the Annexin A5 component is administered to the subject, as a first round of administration of the Annexin A5 component, and subsequently as a second round of administration of the Annexin A5 component, both prior to the commencement of anti-VEGF therapy, and the Annexin A5 component is administered to the subject, as a third round of administration of the Annexin A5, after the anti-VEGF therapy has commenced, but typically prior to the end of the anti-VEGF therapy. In other instances, the Annexin A5 component is administered to the subject, as a first round of administration of the Annexin A5 component prior to the commencement of anti-VEGF therapy, and the Annexin A5 component is administered to the subject, as a second round of administration of the Annexin A5, and further subsequently as a third round of administration of the Annexin A5 component, where both the second and third rounds of administration occur after the anti-VEGF therapy has commenced, but typically prior to the end of the anti-VEGF therapy. In other instances, the Annexin A5 component is administered to the subject, as a first round of administration of the Annexin A5 component, and subsequently as a second round of administration of the Annexin A5 component, and further subsequently as a third round of administration, wherein all three of the first, second and third rounds of administration of the Annexin A5 component occurs after the anti-VEGF therapy has commenced, but typically prior to the end of the anti-VEGF therapy. In some options, there are no rounds of anti-VEGF therapy between the first and second rounds of administration of the Annexin A5 component and/or between the second and third rounds of administration of the Annexin A5 component. In other options, there may be a single round of anti-VEGF therapy between the first and second rounds of administration of the Annexin A5 component and/or between the second and third rounds of administration of the Annexin A5 component. In other options, there may be more than one round of anti-VEGF therapy between the first and second rounds of administration of the Annexin A5 component and/or between the second and third rounds of administration of the Annexin A5 component.

Although described above with respect to one, two, or three rounds of administration of the Annexin A5 component, it will be appreciated that one or more additional rounds of administration of the Annexin A5 component may be used beyond the third round. In some instances, all rounds of administration of the Annexin A5 component occur prior to the commencement of anti-VEGF therapy. In other instances, any of the first, second, third or any subsequent round of administration of the Annexin A5 component may occur prior to the commencement of anti-VEGF therapy, and one or more subsequent rounds of administration the Annexin A5 component may administered to the subject after the anti-VEGF therapy has commenced, but typically prior to the end of the anti-VEGF therapy. In other instances, rounds of administration of the Annexin A5 component occur after the anti-VEGF therapy has commenced, but typically prior to the end of the anti-VEGF therapy. In some options, there are no rounds of anti-VEGF therapy between the first and second rounds of administration of the Annexin A5 component, between the second and third rounds of administration of the Annexin A5 component, between the third round and a subsequent round of administration of the Annexin A5 component and/or between any of the multiple subsequent rounds of administration of the Annexin A5 component. In other options, there may be a single round of anti-VEGF therapy between any one or more of the first and second rounds of administration of the Annexin A5 component, the second and third rounds of administration of the Annexin A5 component, the third round and a subsequent round of administration of the Annexin A5 component and/or any of the multiple subsequent rounds of administration of the Annexin A5 component. In other options, there may be more than one round of anti-VEGF therapy between any one or more of the first and second rounds of administration of the Annexin A5 component, the second and third rounds of administration of the Annexin A5 component, the third round and a subsequent round of administration of the Annexin A5 component and/or any of the multiple subsequent rounds of administration of the Annexin A5 component.

The subject to whom the co-therapy is applied in accordance with the second aspect of the present invention is preferably observably responsive to the first, and/or any subsequent, round of administration of the Annexin A5 component part of the co-therapy. That is to say, it is preferred that the subject demonstrates an observable reduction in worsening, stabilisation, or improvement in the condition of the RVO, and/or condition associated therewith, as a result of any one or more of any of the rounds of administration of the Annexin A5 component of the co-therapy. Such an observable reduction in worsening, stabilisation, or improvement may, for example, be in respect of any one, two, three or four of the following characteristics:
ix. visual acuity, optionally assessed by Best-Corrected Visual Acuity (BCVA) test;
x. macular retinal thickness;
xi. macular volume; and
xii. Retinal Area of Non-Perfusion (RANP).

Typically, the (or each) observable improvement may be observable, and/or observed, before the subject receives a subsequent administration of anti-VEGF agent.

As a consequence of the administration of the Annexin A5 component in the first, and/or any subsequent round of administration of the Annexin A5 component, the frequency and/or number of subsequent rounds of administration of the anti-VEGF component that follow the initial administration of the anti-VEGF component to the subject can be reduced, or even entirely avoided, for a defined period without substantially reducing the prophylactic or therapeutic effectiveness of the anti-VEGF therapy in respect of the RVO, or a condition associated therewith. This may be compared, for example, to a suitable control such as a subject, or cohort of subjects, who has/have not been treated with a molecule comprising, or consisting of, Annexin A5 protein, but who receive(s) multiple administrations of the anti-VEGF component (such as successive administrations approximately every 28 days (e.g. ± 7, 6, 5, 4, 3, 2, or 1 days) for the same defined period. The defined period may, for example, be about, up to, or at least 2, 3, 4, 5, 6, 7, 8, 9, 10, or 11 months, or about, up to, or at least 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period. The defined period may be until the end of the subject's therapeutic or prophylactic need. A cohort, can in some embodiments, be two or more subjects, as such about, at least, or greater than 3, 4, 5, 10, 15, 20, 25 30, 40, 50 60, 70, 80 90 or 100 subjects.

The prophylactic or therapeutic effectiveness of the anti-VEGF therapy in respect of the RVO, or a condition associated therewith, can be assessed with respect to any one, two, three or four of the following characteristics:
xiii. visual acuity, optionally assessed by Best-Corrected Visual Acuity (BCVA) test;
xiv. macular retinal thickness;
xv. macular volume; and
xvi. Retinal Area of Non-Perfusion (RANP).

In the embodiment of the second aspect of the present invention in which the molecule comprising, or consisting of, Annexin A5 protein is administered to the subject prior to the commencement of anti-VEGF therapy, a reduced frequency of administration of the anti-VEGF component that follows the initial administration of the Annexin A5 component may, for example, refer to a period of time between the initial administration of the Annexin A5 component to the subject and a first administration of the anti-VEGF component to the subject, that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years, provided that the subject receives at least one administration of the anti-VEGF component during the therapy. In this context, the term "about" may refer to a period that ± 50%, 40%, 30%, 20%, 10%, 5%, 4%, 3%, 2%, 1% or less than 1% of the stated time period.

For example, in accordance with the second aspect of the present invention, and following the initial round of administration of the Annexin A5 component (and/or any subsequent round of administration of the Annexin A5 component) to the subject, the anti-VEGF component may be subsequently administered, to the subject:
i. fewer than 12, 11, 10, 9, 8, 7, 6, 5, 4, 3, 2 or 1 times within a period of twelve months from the start of treatment with the Annexin A5 molecule or composition;
ii. fewer than 10, 9, 8, 7, 6, 5, 4, 3, 2 or 1 times within a period of ten months from the start of treatment with the Annexin A5 molecule or composition;
iii. fewer than 8, 7, 6, 5, 4, 3, 2 or 1 times within a period of eight months from the start of treatment with the Annexin A5 molecule or composition; and/or
iv. fewer than 6, 5, 4, 3, 2 or 1 times within a period of six months from the start of treatment with the Annexin A5 molecule or composition.

Additionally or alternatively, a reduced frequency of the subsequent rounds of administration of the anti-VEGF component that follow the initial administration of the anti-VEGF component, in the subject that receives co-therapy with Annexin A5 in accordance with the second aspect of the present invention, may, for example, refer to a period of time between the initial administration of the anti-VEGF component to the subject and a second administration of the anti-VEGF component to the subject that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period.

Additionally, or alternatively, a reduced frequency of the subsequent rounds of administration of the anti-VEGF component that follow the initial administration of the anti-VEGF component, in the subject that receives co-therapy with Annexin A5 in accordance with the second aspect of the present invention, may, for example, refer to a period of time between a second round and/or any subsequent round of administration of the anti-VEGF component, and the round of anti-VEGF administration that follows it, that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period.

For example, in accordance with the second aspect of the present invention, and following the initial administration of the anti-VEGF component to the subject, the anti-VEGF agent may be subsequently administered, to the subject that receives co-therapy with Annexin A5:
i. fewer than 12, 11, 10, 9, 8, 7, 6, 5, 4, 3, 2 or 1 times within a period of twelve months from the start of treatment with the Annexin A5 molecule or composition;
ii. fewer than 10, 9, 8, 7, 6, 5, 4, 3, 2 or 1 times within a period of ten months from the start of treatment with the Annexin A5 molecule or composition;
iii. fewer than 8, 7, 6, 5, 4, 3, 2 or 1 times within a period of eight months from the start of treatment with the Annexin A5 molecule or composition; and/or
iv. fewer than 6, 5, 4, 3, 2 or 1 times within a period of six months from the start of treatment with the Annexin A5 molecule or composition.

Preferably, in accordance with the second aspect of the present invention, the mode of systemic administration of the molecule comprising, or consisting of, Annexin A5 protein (such as a molecule consisting of monomeric Annexin A5 protein) is selected from intravenous, subcutaneous or intramuscular administration, or may be administered by suprachoroidal injection (e.g. as discussed in Wu et al, 2023, Pharmaceuticals, 16(9): 1241). Where it is administered intravenously, then the molecule comprising, or consisting of, Annexin A5 protein, or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2. In this context, the term "about" when applied to a pH value that is stated to the nearest integer may refer to a value that is ± 0.5, 0.4, 0.3, 0.2, or 0.1 pH units of the stated value; whereas when the term "about" is applied to a pH value that is stated to one decimal place, then it may refer to a value that is ± 0.05, 0.04, 0.03, 0.02, or 0.01 pH units of the stated value.

More preferably, a dose of the molecule comprising, or consisting of, the Annexin A5 protein is systemically administered (such as by intravenous, subcutaneous or intramuscular administration), or by suprachoroidal injection, daily to the subject, for two or more consecutive days.

In an embodiment of the second aspect of the present invention, for each round of administration of the Annexin A5 component to the subject, a dose of the molecule comprising, or consisting of, Annexin A5 protein is administered to the subject daily for three, four, five, six, seven or more consecutive days and/or or for at least two, three, four, five six or seven days within each 7-day (one week) or 8-day period, such as for a period of at least one, two, three, four weeks or more, and/or or for at least two, three, four, five six or seven days within each 7-day (one week) of 8-day period, such as for a period of at least one, two, three, four weeks or more.

Optionally, in accordance with the second aspect of the present invention, for each round of administration of the Annexin A5 component to the subject, the daily dose of the molecule comprising, or consisting of, Annexin A5 protein (such as monomeric Annexin A5 protein) that is administered to the subject is: a) a total daily dose of about 5 mg, about 4 mg, about 3mg, about 2mg, or less than about 2 mg, 0.1 to 1.5 mg or about 1 mg; and/or b) within a range selected from the group consisting of about 50-100 µg, about 40-80 µg, about 30-60 µg, about 20-40 µg, 1 µg to 30 µg per kg, or less than about 20 µg, per kg body weight of the subject. The term "about" as used herein may be used herein to mean a range of ±50%, ±40%, ±30%, ±20%, ±10%, ±5%, ±4%, ±3%, ±2% or ±1% of the value mentioned.

The inventors have also determined that the treatment and prophylaxis of macular oedema, retinal vein occlusion, and/or one or more conditions associated therewith, in accordance with the first and/or second aspects of the present invention may be achieved with substantially lower doses of a molecule comprising, or consisting of, Annexin A5 protein (such as monomeric Annexin A5 protein).

For example, for each round of administration of the Annexin A5 component to the subject, the subject may be administered the molecule comprising, or consisting of, the Annexin A5 protein at a dose of about 0.01 µg/kg to about 10 µg/kg, or about 0.01 µg/kg to about 1 µg/kg, and/or wherein the subject is administered the molecule at a dose of about 0.001 mg to about 1 mg, or 1 µg to about 100 µg. The term "about" as used herein may be used herein to mean a range of ±50%, ±40%, ±30%, ±20%, ±10%, ±5%, ±4%, ±3%, ±2% or ±1% of the value mentioned.

Optionally, in accordance with the second aspect of the present invention, the molecule comprising, or consisting of, the Annexin A5 protein may be for use, as a combination therapy with an anti-vascular endothelial growth factor (anti-VEGF) agent, in the prophylaxis or treatment of a condition in a subject that has (for example has been diagnosed as having), is suspected of having, or is at risk of having, retinal vein occlusion (RVO), wherein said condition is selected from the group consisting of macular oedema, retinal ischemia, iris neovascularization (optionally, iris neovascularization characterised by an increase in cytokines, such as an increase in VEGF), retinal neovascularization (optionally, retinal neovascularization characterised by an increase in cytokines, such as an increase in VEGF), neovascular glaucoma, vitreous haemorrhage, rubeosis, and retinal detachment.

Optionally, in accordance with the first and/or second aspects of the present invention, the retinal vein occlusion may, for example, be selected from: central retinal vein occlusion (CRVO); branch retinal vein occlusion (BRVO); or hemi-retinal vein occlusion (HRVO).

Optionally, in accordance with the first and/or second aspects of the present invention, the retinal vein occlusion can optionally be acute retinal vein occlusion, for example: acute central retinal vein occlusion (CRVO); acute branch retinal vein occlusion (BRVO); or acute hemi-retinal vein occlusion (HRVO).

Optionally, in accordance with one embodiment of the first and/or second aspects of the present invention, the use or method can also (or alternatively) be for the prophylaxis or the treatment of one or more diseases, disorders or conditions associated with, and/or caused (directly or indirectly) by, macular oedema and/or retinal vein occlusion.

Optionally, in accordance with the first and/or second aspects of the present invention, the subject may be characterised as having about 1.0% to about 2% of erythrocytes that are Annexin A5 positive. In this context, the term "about" when applied to a value that is stated to the nearest integer may refer to a value that is ± 0.5, 0.4, 0.3, 0.2, or 0.1 of the stated value; whereas when the term "about" is applied to a value that is stated to one decimal place, then it may refer to a value that is ± 0.05, 0.04, 0.03, 0.02, or 0.01 of the stated value.

Optionally, in accordance with the first and/or second aspects of the present invention, the subject may be characterised as having a mean number of monomeric Annexin A5 binding sites of about 400 to about 1000 *per* erythrocyte. In this context, the term "about" may refer to a value that ± 50%, 40%, 30%, 20%, 10%, 5%, 4%, 3%, 2%, 1% or less than 1% of the stated value.

Optionally, in accordance with the first and/or second aspects of the present invention, the subject is typically a human subject, such as a human male and/or human female. The subject may be an adult or, less typically, a juvenile, child or neonate.

Optionally, in accordance with the first and/or second aspects of the present invention, the subject is a human adult and/or has a body weight of about 50 kg to about 150 kg. The term "about" as used herein may be used herein to mean a range of ±50%, ±40%, ±30%, ±20%, ±10%, ±5%, ±4%, ±3%, ±2% or ±1% of the value mentioned.

Optionally, in accordance with the first and/or second aspects of the present invention, the subject may, additionally, or alternatively, be characterised by one or more of the following:
a. the subject is aged about 50 years or more;
b. the subject does not have, or is not suspected as having, a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sarcoidoisis, Wegener's Granulomatosis and/or Goodpasture's Syndrome), although preferably excluding diabetes and diabetes mellitus);
c. the subject does not have a medical history of having a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sarcoidoisis, Wegener's Granulomatosis and/or Goodpasture's Syndrome), although preferably excluding diabetes and diabetes mellitus);
d. the subject does not have a familial history of having a cardiovascular disease (such as a myocardial infarction, a stroke, atherosclerosis, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behgets disease, polyarteritis nodosa, sarcoidoisis, Wegener's Granulomatosis and/or Goodpasture's Syndrome), although preferably excluding diabetes and diabetes mellitus); and/or
e. the subject does not have, or is not suspected as having, an oncological condition, such as a solid tumour, a lymphoma and/or a blood cancer, for example one or more condition selected from breast cancer, endometrial cancer, cervix cancer, ovary cancer, lung cancer, head and/or neck cancer, brain cancer, thyroid cancer, oesophagus cancer, stomach cancer, colon and/or rectal cancer, liver cancer, pancreatic cancer, skin cancer, kidney cancer, bladder cancer, prostate cancer, testis cancer, bone cancer, lymphoma (such as Hodgkin lymphoma and/or non-Hodgkin lymphoma) and/or blood cancer (such as acute lymphoblastic leukemia, acute myeloid leukemia and/or chronic myeloid leukemia).

In one embodiment of the first and/or second aspects of the present invention, the dose of the molecule comprising, or consisting of, the Annexin A5 protein is administered to the subject once, or more, *per* day. The dose of the Annexin A5 molecule may optionally be administered to the subject once, or more, *per* day, for a period of one, two, three, four, five or six consecutive days or more, and/or or for at least two, three, four, five six or seven days within each 7-day (one week) of 8-day period, such as for a period of at least one, two, three, four weeks or more.

In one embodiment of the first and/or second aspect of the present invention, the first, and/or any subsequent, round of administration of the molecule comprising, or consisting of, the Annexin A5 protein is administered to the vascular system of the subject, for example, as a systemic administration such as by intravenous administration, or may be administered by suprachoroidal injection. Where it is administered intravenously, then the molecule comprising, or consisting of, the Annexin A5 protein, or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2. In this context, the term "about" when applied to a pH value that is stated to the nearest integer may refer to a value that is ± 0.5, 0.4, 0.3, 0.2, or 0.1 pH units of the stated value; whereas when the term "about" is applied to a pH value that is stated to one decimal place, then it may refer to a value that is ± 0.05, 0.04, 0.03, 0.02, or 0.01 pH units of the stated value.

Also disclosed as a treatment option herein in respect of the first and/or second aspects of the present invention (and also any of the other aspects of the present invention), the molecule comprising, or consisting of, monomeric Annexin A5 protein to be administered directly to the eye for the first round, and/or (if relevant) any or all subsequent rounds, of administration of the Annexin A5 component of the therapy. For example, the Annexin A5 molecule may be administered by suprachoroidal injection or intravitreal injection. Such injections may be in the form of a pharmaceutical formulation, or in the form of a medical device, such as implantable device. Such formulations or devices may, for example, be slow release, or continuous release, and/or resorbable formulations or devices. A slow or continuous drug release may, for example, have the ability to achieve a therapeutic drug concentration and therapeutic effect on the affected eye over a period between about 3 to about 180 days (for example, about 4, 5, or 6 days, about 1, 2 or 3 weeks, about 1, 2, 3, 4, 5 or 6 months), or even longer. In another option, the Annexin A5 molecule may be administered directly to the retinal vein (such as the central retinal vein, the branch retinal vein and/or the hemi-retinal vein) of the subject, for example by catherisation. However, it may be preferred that this is avoided.

The Annexin A5 protein for use in the first and/or second aspects of the present invention may comprise, or consist of, the Annexin A5 protein, and optionally comprises, or consists of, monomeric Annexin A5 protein. Preferably, it consists of monomeric Annexin A5 protein. Optionally it is selected from one or more of the following:
a. a protein comprising, consisting essentially of, or consisting of the sequence of human Annexin A5 protein (such as a human monomeric Annexin A5 protein and/or optionally, a protein comprising, or consisting of, the sequence of SEQ ID NO:1, either with or without the N-terminal methionine);
b. a recombinant human Annexin A5 protein, such as a recombinant human monomeric Annexin A5 protein ;
c. a mammalian orthologue of human Annexin A5 protein, such as of human monomeric Annexin A5 protein;
d. an allelic or genetic variant of a), b) or c);
e. a functional analogue or variant of a Annexin A5 protein (such as a monomeric Annexin A5 protein) which is a protein which comprises, or consists of, an amino acid sequence that is more than 50%, 60%, 70%, 75%, such as more than 80%, 85%, more than 90%, or even more preferably more than 95% or 99% identical to human Annexin A5 protein (such as a monomeric Annexin A5 protein) having the sequence defined by SEQ ID NO:1 either with, or without, the N-terminal methionine; and
f. a biologically active fragment of any of a) to e).

In some embodiments, the molecule comprising the Annexin A5 protein for use in the first and/or second aspects of the present invention may be a conjugate in which the Annexin A5 protein (such as a monomeric Annexin A5 protein) is conjugated to moiety, such as a non-proteinaceous moiety. Without limitation, said moiety may for example be a therapeutic, prophylactic, diagnostic, prognostic, or theragnostic moiety. In some embodiments, the moiety is a drug (such as a non-proteinaceous drug) and/or a radioactive moiety (for example, wherein the conjugate is suitable for use in radioimmunotherapy ("RIT")).

Also provided herein is a composition comprising the molecule that comprises, or consists of, the Annexin A5 protein, as described herein, for use in accordance with the first and/or second aspects of the present invention. Said composition may, for example, be a unit dosage composition. The unit dosage composition may, for example, comprise: a total dose of about 5 mg, about 4 mg, about 3mg, about 2mg, or less than about 2 mg, such as about 0.1-1.5 mg, about 1 mg or less of the Annexin A5 protein. In this context, the term "about" when applied to a unit dosage value in mg that is stated to the nearest integer may refer to a value that is ± 0.5, 0.4, 0.3, 0.2, or 0.1 mg of the stated value; whereas when the term "about" is applied to a unit dosage value in mg that is stated to one decimal place, then it may refer to a value that is ± 0.05, 0.04, 0.03, 0.02, or 0.01 mg of the stated value.

Optionally, the composition is a pharmaceutical composition, and further optionally, the composition further comprises one or more pharmaceutically acceptable carriers. In some embodiments, the composition consists of the molecule comprising, or consisting of, the Annexin A5 protein and one or more pharmaceutically acceptable carriers. Optionally, in addition to the use as a combination therapy with an anti-VEGF agent, the composition is for use as a combination therapy with one or more other agents. Optionally, the Annexin A5 molecule, or the composition thereof, is used, with or without the one or more other agents, as a first line of treatment for the prophylaxis or treatment of macular oedema and/or retinal vein occlusion (RVO), and/or one or more diseases, disorders or conditions associated with, and/or caused (directly or indirectly) by, macular oedema and/or retinal vein occlusion.

In accordance with the first and/or second aspects of the present invention, in some embodiments the molecule comprising, or consisting of, the Annexin A5 protein or the composition comprising said molecule, is for use, and/or is used, as a combination therapy with the one or more other agents, wherein the subject is administered the Annexin A5 molecule in the same composition as, separately from, simultaneously with a separate formulation containing, or sequentially with, the one or more other agents.

Optionally, in accordance with the first and/or second aspect of the present invention, the molecule comprising, or consisting of, the Annexin A5 protein and the anti-VEGF agent are administered in combination, simultaneous in separate administrations, or sequentially in separate administrations. In some embodiments, if the administrations are separate and sequential, then the period of time between the sequential administrations of the Annexin A5 molecule and the anti-VEGF agent is about 28 days or less, such as about 3 weeks or less, about 2 weeks or less, about 1 week or less, about 6, 5, 4, 3, 2 or 1 day or less, such as about 24, 28, 12, 6, 5, 4, 3, 2 or 1 hours or less. However, the reported studies in the present application have demonstrated that an Annexin A5 dosage can delay the requirement for anti-VEGF therapy for much longer periods, or even entirely remove the need for anti-VEGF therapy. As such, in other embodiments, if the administrations are separate and sequential, then the period of time between the sequential administrations of the Annexin A5 molecule and the anti-VEGF agent is greater than about 28 days. For example, as noted above, any given round of administration of the Annexin A5 may occur over multiple days and may include multiple repeated administrations to the subject. For example, for any given round of administration of the Annexin A5 molecule, it may be administered to the subject for two consecutive days or more, such as for three, four, five, six, seven or more consecutive days, and/or or for at least two, three, four, five six or seven days within each 7-day (one week) of 8-day period, such as for a period of at least one, two, three, four weeks or more. In the case that the Annexin A5 administration occurs over multiple days then, in some embodiments, said time periods between the sequential administrations of the Annexin A5 molecule and the anti-VEGF agent are calculated relative to the start date of the Annexin A5 administration. In other embodiments, said time periods are calculated relative to the end date of the Annexin A5 administration.

In a third aspect, the present invention provides a composition (such as a unit dosage composition) for use in accordance with the first and/or second aspects of the present invention, the composition comprising the molecule that comprises, or consists of, the Annexin A5 protein as defined above with respect to the first and/or second aspects of the present invention,
optionally wherein the composition comprises the Annexin A5 molecule (such as the molecule consisting of monomeric Annexin A5 protein) in an amount of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of a subject, and/or
optionally wherein the composition comprises the Annexin A5 molecule (such as the molecule consisting of monomeric Annexin A5 protein) in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg.

The term "about" as used herein may be used herein to mean a range of ±50%, ±40%, ±30%, ±20%, ±10%, ±5%, ±4%, ±3%, ±2% or ±1% of the value mentioned.

In one embodiment of the third aspect of the present invention, the composition is a unit dosage composition and further optionally the treatment and/or prophylaxis can comprise or consist of administering the complete unit dosage to the subject, for example in a single administration.

The composition used in accordance with the third aspect of the invention may be a pharmaceutical composition.

Optionally, the composition of the third aspect of the invention further comprises one or more pharmaceutically acceptable carriers.

For example, the composition may consist of the Annexin A5 molecule (such as a molecule consisting of monomeric Annexin A5 protein) and one or more pharmaceutically acceptable carriers.

In an embodiment of the third aspect of the present invention, the prophylactically or therapeutically effective dose of the anti-VEGF agent is administered directly to an eye of the subject. For example, the anti-VEGF agent may be administered by intravitreal injection.

Also described herein is a sterile injectable intravitreal composition (such as a unit dosage sterile injectable intravitreal composition) comprising a molecule that comprises, or consists of, Annexin A5 protein (such as a molecule that consists of monomeric Annexin A5),
typically wherein the composition is presented in a sterile syringe, and more preferably wherein the syringe contains the composition in a volume of about or less than 200 µL, preferably a volume of about or less than 100 µL, and more preferably wherein the volume is about 10 to about 90 µL, about 20 to about 80 µL, about 30 to about 70 µL, about 40 to about 60 µL or about 50 µL (wherein the term "about" in this context refers to a volume that is ± 4, 3, 2 or 1 µL of the stated value)
optionally, wherein the composition comprises the molecule consisting of monomeric Annexin A5 protein: (i) 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of the subject, and/or (ii) in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg, wherein term "about" as used in this context may be used herein to mean a range of ±50%, ±40%, ±30%, ±20%, ±10%, ±5%, ±4%, ±3%, ±2% or ±1% of the value mentioned, and
optionally wherein the composition is for use in the prophylaxis or the treatment of macular oedema and/or retinal vein occlusion (RVO), and/or one or more diseases, disorders or conditions associated with, and/or caused (directly or indirectly) by, macular oedema and/or retinal vein occlusion, in the subject, and said use is as a combination therapy with an anti-vascular endothelial growth factor (anti-VEGF) agent.

The sterile injectable intravitreal composition may be presented in the form of a kit comprising the composition and one or more additional components.

For example, the kit may comprise the composition presented in a sterile syringe and, affixed thereto or included separately in the kit, one or more sterile needles. Preferably, the or each needle may be a gauge selected from gauge 27, 28, 29, or more preferably 30 or 31. It may be preferred that the or each needle has a length of from about 0.5 to 0.62 inches (12.7 to 15.75 mm) in length. Longer needles may increase risk of retinal injury if the patient accidentally moves forward during the intravitreal injection procedure.

In another example, the kit may comprise one or more further compositions, each comprising one or more antibiotics, for administration to the patient for and/or after intravitreal injection.

In an embodiment thereof, the anti-VEGF agent is administered directly to an eye of the subject. For example, the anti-VEGF agent may be administered by intravitreal injection.

In a fourth aspect of the present invention, there is provided a molecule comprising, or consisting of, Annexin A5 protein (such as the molecule consisting of monomeric Annexin A5 protein or a conjugate thereof) for use in:
a) the prophylaxis or the treatment of a disease, disorder or condition associated with VEGF that is selected from the group consisting of macular oedema, RVO, a retinopathy associated with a blinding eye disease selected from the group consisting of age-related macular degeneration (AMD), diabetic retinopathy and hypertensive retinopathy in a subject;
b) enhancing an anti-VEGF therapy; and/or
c) reducing complications associated with administration of an anti-VEGF agent selected from the group consisting of reducing or preventing the subject developing a reduced response, and/or resistance, to ongoing anti-VEGF therapy; reducing or preventing ocular and/or systemic toxicity in anti-VEGF therapy; and/or reducing or preventing one or more of infectious endophthalmitis, sterile intraocular inflammation, retinal vasculitis and/or transient intraocular pressure (IOP) spikes.

In some embodiments of the fourth aspect of the present invention, the disease, disorder or condition is selected from macular oedema, RVO, or conditions associated with macular oedema or RVO selected from a retinopathy associated with a blinding eye disease, such as age-related macular degeneration (AMD), diabetic retinopathy and hypertensive retinopathy. These, and other diseases, disorders and/or conditions associated with VEGF, are also described in Apte *et al,* 2019 (*supra*)*.*

In some embodiments, the nature of the enhancement of the anti-VEGF therapy provided by the fourth aspect of the present invention can, for example, be to achieve a stronger or more complete prophylactic or therapeutic effect, or prophylactic or therapeutic endpoint, than the effect that can be achieved by the anti-VEGF therapy alone. This may be, for example, relative to a subject receiving the same dosage, frequency and/or period of anti-VEGF administrations, but without having received any administration of the Annexin A5 molecule.

Additionally, or alternatively, the nature of the enhancement of the anti-VEGF therapy provided by the fourth aspect of the present invention can, for example, be to reduce the frequency of repeat anti-VEGF administrations during the course of the anti-VEGF treatment, to reduce the dosage requirement for anti-VEGF therapy, and/or reduce the period during which anti-VEGF therapy is required, in order to obtain the same prophylactic or therapeutic endpoint as achieved by the anti-VEGF therapy alone. This may be relative to a subject receiving anti-VEGF therapy alone, for example when comparing subjects treated in accordance with the fourth aspect of the present invention and subjects treated with anti-VEGF therapy alone, that have reached the same prophylactic or therapeutic endpoint.

In an embodiment of the fourth aspect of the present invention, the anti-VEGF agent is administered directly to an eye of the subject. For example, the anti-VEGF agent may be administered by intravitreal injection.

In the case of macular oedema, RVO, the conditions associated with macular oedema or RVO, and other ocular conditions, the prophylactic or therapeutic effect, or prophylactic or therapeutic endpoint, may be determined by assessing any one, two, three or four of the following characteristics:
i. improvement in visual acuity, optionally assessed by Best-Corrected Visual Acuity (BCVA) test;
ii. increase in macular retinal thickness;
iii. increase in macular volume; and
iv. increase in Retinal Area of Non-Perfusion (RANP).

In some embodiments, the reduction of complications associated with administration of an anti-VEGF agent include a reduction in, or avoidance of, the subject developing a reduced response and/or resistance to ongoing anti-VEGF therapy. Such effects may, for example, be relative to a subject receiving anti-VEGF therapy alone, for example when comparing subjects treated in accordance with the fourth aspect of the present invention and subjects treated with anti-VEGF therapy alone, that have reached the same prophylactic or therapeutic endpoint.

In some embodiments, the reduction of complications associated with administration of an anti-VEGF agent include a reduction in, or avoidance of, ocular and/or systemic toxicity in anti-VEGF therapy. This may be relative to a subject receiving anti-VEGF therapy alone, for example when comparing subjects treated in accordance with the fourth aspect of the present invention and subjects treated with anti-VEGF therapy alone, that have reached the same prophylactic or therapeutic endpoint.

In some embodiments, the reduction of complications associated with administration of an anti-VEGF agent include a reduction in, or avoidance of, complications associated with intravitreal injections of anti-VEGF therapy selected from infectious endophthalmitis, sterile intraocular inflammation, retinal vasculitis and/or transient intraocular pressure (IOP) spikes. This may be relative to a subject receiving anti-VEGF therapy alone, for example when comparing subjects treated in accordance with the fourth aspect of the present invention and subjects treated with anti-VEGF therapy alone, that have reached the same prophylactic or therapeutic endpoint.

Optionally, in accordance with the third, or fourth aspects of the present invention, the subject is typically a human subject, such as a human male and/or human female. The subject may be an adult or, less typically, a juvenile, child or neonate.

Optionally, in accordance with the third, or fourth aspects of the present invention, the subject is a human adult and/or has a body weight of about 50 kg to about 150 kg. The term "about" as used herein may be used herein to mean a range of ±50%, ±40%, ±30%, ±20%, ±10%, ±5%, ±4%, ±3%, ±2% or ±1% of the value mentioned.

Optionally, in accordance with the third, or fourth aspects of the present invention, the subject may, additionally, or alternatively, be characterised by one or more of the following:
a. the subject is aged about 50 years or more;
b. the subject does not have, or is not suspected as having, a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus);
c. the subject does not have a medical history of having a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sarcoidoisis, Wegener's Granulomatosis and/or Goodpasture's Syndrome), although preferably excluding diabetes and diabetes mellitus);
d. the subject does not have a familial history of having a cardiovascular disease (such as a myocardial infarction, a stroke, atherosclerosis, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus); and/or
e. the subject does not have, or is not suspected as having, an oncological condition, such as a solid tumour, a lymphoma and/or a blood cancer, for example one or more condition selected from breast cancer, endometrial cancer, cervix cancer, ovary cancer, lung cancer, head and/or neck cancer, brain cancer, thyroid cancer, oesophagus cancer, stomach cancer, colon and/or rectal cancer, liver cancer, pancreatic cancer, skin cancer, kidney cancer, bladder cancer, prostate cancer, testis cancer, bone cancer, lymphoma (such as Hodgkin lymphoma and/or non-Hodgkin lymphoma) and/or blood cancer (such as acute lymphoblastic leukemia, acute myeloid leukemia and/or chronic myeloid leukemia).

In one embodiment of the third, or fourth aspects of the present invention, the first dose of the molecule comprising, or consisting of, the Annexin A5 protein (and/or any subsequent round of administration of the Annexin A5 component) is administered to the subject once, or more, *per* day. For any given round of administration, the dose of the Annexin A5 molecule may optionally be administered to the subject once, or more, *per* day, for a period of one, two, three, four, five or six consecutive days or more, and/or or for at least two, three, four, five six or seven days within each 7-day (one week) of 8-day period, such as for a period of at least one, two, three, four weeks or more.

In some embodiments of the third, or fourth aspects of the present invention, the molecule comprising, or consisting of, Annexin A5 protein or the composition comprising said molecule, is presented in the same composition as the anti-VEGF agent.

In other embodiments of the third, or fourth aspects of the present invention, the molecule comprising, or consisting of, Annexin A5 protein or the composition comprising said molecule is formulated separately from the anti-VEGF agent. In that case, the anti-VEGF and Annexin A5 components may be administered to the subject simultaneously or sequentially.

In the case of sequential administration of the Annexin A5 component and the anti-VEGF component then, in some instances of the third, or fourth aspects of the present invention, the molecule comprising, or consisting of, Annexin A5 protein ("the Annexin A5 component") is administered to the subject, as a first round of administration of the Annexin A5 component, prior to the commencement of anti-VEGF therapy. In other instances, the molecule comprising, or consisting of, Annexin A5 protein may be administered to the subject, as a first round of administration of the Annexin A5 component, after the anti-VEGF therapy has commenced, but typically prior to the end of the anti-VEGF therapy. For example in the case of a form of anti-VEGF therapy that (in the absence of Annexin A5 co-therapy) would require multiple rounds of anti-VEGF administration to achieve the prophylactic or therapeutic effect or endpoint in the subject, the Annexin A5 component may be administered to the subject, as a first round of administration of the Annexin A5 component, prior to the commencement of anti-VEGF therapy, or after the anti-VEGF therapy has commenced but before any one or more of the subsequent multiple round of administration of the anti-VEGF component.

In some embodiments of the third and/or fourth aspects of the present invention, the subject may also receive a second round of administration of the of the Annexin A5 component. In some instances, the Annexin A5 component is administered to the subject, as a first round of administration of the Annexin A5 component, and subsequently as a second round of administration of the Annexin A5 component, prior to the commencement of anti-VEGF therapy. In other instances, the Annexin A5 component is administered to the subject, as a first round of administration of the Annexin A5 component prior to the commencement of anti-VEGF therapy, and the Annexin A5 component is administered to the subject, as a second round of administration of the Annexin A5, after the anti-VEGF therapy has commenced, but typically prior to the end of the anti-VEGF therapy. In other instances, the Annexin A5 component is administered to the subject, as a first round of administration of the Annexin A5 component, and subsequently as a second round of administration of the Annexin A5 component, wherein both of the first and second rounds of administration of the Annexin A5 component occurs after the anti-VEGF therapy has commenced, but typically prior to the end of the anti-VEGF therapy. In some options, there are no rounds of anti-VEGF therapy between the first and second rounds of administration of the Annexin A5 component. In other options, there may be a single round of anti-VEGF therapy between the first and second rounds of administration of the Annexin A5 component. In other options, there may be more than one round of anti-VEGF therapy between the first and second rounds of administration of the Annexin A5 component.

In some embodiments of the third and/or fourth aspects of the present invention, the subject may also receive a third round of administration of the of the Annexin A5 component. In some instances, the Annexin A5 component is administered to the subject, as a first round of administration of the Annexin A5 component, and subsequently as a second round of administration of the Annexin A5 component, and further subsequently as a third round of administration of the Annexin A5 component, all prior to the commencement of anti-VEGF therapy. In other instances, the Annexin A5 component is administered to the subject, as a first round of administration of the Annexin A5 component, and subsequently as a second round of administration of the Annexin A5 component, both prior to the commencement of anti-VEGF therapy, and the Annexin A5 component is administered to the subject, as a third round of administration of the Annexin A5, after the anti-VEGF therapy has commenced, but typically prior to the end of the anti-VEGF therapy. In other instances, the Annexin A5 component is administered to the subject, as a first round of administration of the Annexin A5 component prior to the commencement of anti-VEGF therapy, and the Annexin A5 component is administered to the subject, as a second round of administration of the Annexin A5, and further subsequently as a third round of administration of the Annexin A5 component, where both the second and third rounds of administration occur after the anti-VEGF therapy has commenced, but typically prior to the end of the anti-VEGF therapy. In other instances, the Annexin A5 component is administered to the subject, as a first round of administration of the Annexin A5 component, and subsequently as a second round of administration of the Annexin A5 component, and further subsequently as a third round of administration, wherein all three of the first, second and third rounds of administration of the Annexin A5 component occurs after the anti-VEGF therapy has commenced, but typically prior to the end of the anti-VEGF therapy. In some options, there are no rounds of anti-VEGF therapy between the first and second rounds of administration of the Annexin A5 component and/or between the second and third rounds of administration of the Annexin A5 component. In other options, there may be a single round of anti-VEGF therapy between the first and second rounds of administration of the Annexin A5 component and/or between the second and third rounds of administration of the Annexin A5 component. In other options, there may be more than one round of anti-VEGF therapy between the first and second rounds of administration of the Annexin A5 component and/or between the second and third rounds of administration of the Annexin A5 component.

Although described above with respect to one, two, or three rounds of administration of the Annexin A5 component, it will be appreciated that one or more additional rounds of administration of the Annexin A5 component may be used beyond the third round. In some instances, all rounds of administration of the Annexin A5 component occur prior to the commencement of anti-VEGF therapy. In other instances, any of the first, second, third or any subsequent round of administration of the Annexin A5 component may occur prior to the commencement of anti-VEGF therapy, and one or more subsequent rounds of administration the Annexin A5 component may administered to the subject after the anti-VEGF therapy has commenced, but typically prior to the end of the anti-VEGF therapy. In other instances, rounds of administration of the Annexin A5 component occur after the anti-VEGF therapy has commenced, but typically prior to the end of the anti-VEGF therapy. In some options, there are no rounds of anti-VEGF therapy between the first and second rounds of administration of the Annexin A5 component, between the second and third rounds of administration of the Annexin A5 component, between the third round and a subsequent round of administration of the Annexin A5 component and/or between any of the multiple subsequent rounds of administration of the Annexin A5 component. In other options, there may be a single round of anti-VEGF therapy between any one or more of the first and second rounds of administration of the Annexin A5 component, the second and third rounds of administration of the Annexin A5 component, the third round and a subsequent round of administration of the Annexin A5 component and/or any of the multiple subsequent rounds of administration of the Annexin A5 component. In other options, there may be more than one round of anti-VEGF therapy between any one or more of the first and second rounds of administration of the Annexin A5 component, the second and third rounds of administration of the Annexin A5 component, the third round and a subsequent round of administration of the Annexin A5 component and/or any of the multiple subsequent rounds of administration of the Annexin A5 component.

As a consequence of the administration of any one, or more, rounds of administration of the the Annexin A5 component, the frequency and/or number of subsequent rounds of administration of the anti-VEGF component that follow the initial administration of the anti-VEGF component to the subject can be reduced, or even entirely avoided, for a defined period without substantially reducing the prophylactic or therapeutic effectiveness of the anti-VEGF therapy. This may be compared, for example, to a suitable control such as a subject, or cohort of subjects, who has/have not been treated with a molecule comprising, or consisting of, Annexin A5 protein, but who receive(s) multiple administrations of the anti-VEGF component (such as successive administrations approximately every 28 days (e.g. ± 7, 6, 5, 4, 3, 2, or 1 days) for the same defined period. The defined period may, for example, be about, up to, or at least 2, 3, 4, 5, 6, 7, 8, 9, 10, or 11 months, or about, up to, or at least 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period. The defined period may be until the end of the subject's therapeutic or prophylactic need. A cohort, can in some embodiments, be two or more subjects, as such about, at least, or greater than 3, 4, 5, 10, 15, 20, 25 30, 40, 50 60, 70, 80 90 or 100 subjects.

In an embodiment of the third or fourth aspect of the present invention, the molecule comprising, or consisting of, Annexin A5 protein can be administered to the subject prior to the commencement of anti-VEGF therapy, in order to reduce the frequency of administration of the anti-VEGF component that follows the initial administration of the Annexin A5 component. This may, for example, refer to a period of time between the initial administration of the Annexin A5 component to the subject and a first administration of the anti-VEGF component to the subject, that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years, provided that the subject receives at least one administration of the anti-VEGF component during the therapy. In this context, the term "about" may refer to a period that ± 50%, 40%, 30%, 20%, 10%, 5%, 4%, 3%, 2%, 1% or less than 1% of the stated time period.

Additionally, or alternatively, a reduced frequency of the subsequent rounds of administration of the anti-VEGF component that follow the initial administration of the anti-VEGF component, in the subject that receives co-therapy with Annexin A5 in accordance with the third or fourth aspects of the present invention, may, for example, refer to a period of time between the initial administration of the anti-VEGF component to the subject and a second administration of the anti-VEGF component to the subject that is a period of time that is about, or greater than, 1, 2, 3, 4, 5, 6, 7, 8, 9, 10, or 11 months, or greater than 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period.

Additionally, or alternatively, a reduced frequency of the subsequent rounds of administration of the anti-VEGF component that follow the initial administration of the anti-VEGF component, in the subject that receives co-therapy with Annexin A5 in accordance with the third or fourth aspects of the present invention, may, for example, refer to a period of time between a second round and/or any subsequent round of administration of the anti-VEGF component, and the round of anti-VEGF administration that follows it, that is a period of time that is greater than 1, 2, 3, 4, 5, 6, 7, 8, 9, 10, or 11 months, or greater than 1, 1.5, 2, 2.5, 3, 3.5, 4, 4.5, or 5 years. In this context, the term "about" may refer to a period that ± 50%, 40%, 30%, 20%, 10%, 5%, 4%, 3%, 2%, 1% or less than 1% of the stated time period.

For example, in accordance with the third or fourth aspects of the present invention, and following the initial administration of the anti-VEGF component to the subject, the anti-VEGF agent may be subsequently administered, to the subject that receives co-therapy with Annexin A5, fewer than:
i. 12, 11, 10, 9, 8, 7, 6, 5, 4, 3, 2 or 1 times within a period of twelve months from the start of treatment with the Annexin A5 molecule or composition;
ii. 10, 9, 8, 7, 6, 5, 4, 3, 2 or 1 times within a period of ten months from the start of treatment with the Annexin A5 molecule or composition;
iii. 8, 7, 6, 5, 4, 3, 2 or 1 times within a period of eight months from the start of treatment with the Annexin A5 molecule or composition; and/or
iv. 6, 5, 4, 3, 2 or 1 times within a period of six months from the start of treatment with the Annexin A5 molecule or composition.

In one embodiment of the third or fourth aspects of the present invention, the molecule comprising, or consisting of, the Annexin A5 protein is administered to the vascular system of the subject, for example, as a systemic administration such as by intravenous administration. Where it is administered intravenously, then the molecule comprising, or consisting of, the Annexin A5 protein, or a composition comprising the molecule may, for example, be used in the form of a sterile aqueous solution which may contain other substances, for example, enough salts or glucose to make the solution isotonic with blood. The sterile aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2. In this context, the term "about" when applied to a pH value that is stated to the nearest integer may refer to a value that is ± 0.5, 0.4, 0.3, 0.2, or 0.1 pH units of the stated value; whereas when the term "about" is applied to a pH value that is stated to one decimal place, then it may refer to a value that is ± 0.05, 0.04, 0.03, 0.02, or 0.01 pH units of the stated value.

Although disclosed as a treatment option herein in respect of the third or fourth aspects of the present invention, it is less preferred for the molecule comprising, or consisting of, monomeric Annexin A5 protein to be administered directly to the eye when treating ocular conditions, such as macular oedema, RVO and/or conditions associated with macular oedema and/or RVO. For example, the Annexin A5 molecule may be administered by intravitreal injection. In another option, the Annexin A5 molecule may be administered directly to the retinal vein (such as the central retinal vein, the branch retinal vein and/or the hemi-retinal vein ) of the subject, for example by catherisation. However, it is preferred that this is avoided.

The Annexin A5 protein for use in the third or fourth aspects of the present invention may be as described above for the first and/or second aspects of the present invention.

In some embodiments of the third or fourth aspects of the present invention, the molecule comprising the Annexin A5 protein for use in the first and/or second aspects of the present invention may be a conjugate in which the Annexin A5 protein (such as a monomeric Annexin A5 protein) is conjugated to moiety, such as a non-proteinaceous moiety. Without limitation, said moiety may for example be a therapeutic, prophylactic, diagnostic, prognostic, or theragnostic moiety. In some embodiments, the moiety is a drug (such as a non-proteinaceous drug) and/or a radioactive moiety (for example, wherein the conjugate is suitable for use in radioimmunotherapy ("RIT")).

Also provided herein is a composition comprising the molecule that comprises, or consists of, the Annexin A5 protein, as described herein, for use in accordance with the third or fourth aspects of the present invention. Said composition may, for example, be a unit dosage composition. The unit dosage composition may, for example, comprise: a total dose of about 5 mg, about 4 mg, about 3mg, about 2mg, or less than about 2 mg, such as about 0.1-1.5 mg, about 1 mg or less of the Annexin A5 protein. In this context, the term "about" when applied to a unit dosage value in mg that is stated to the nearest integer may refer to a value that is ± 0.5, 0.4, 0.3, 0.2, or 0.1 mg of the stated value; whereas when the term "about" is applied to a unit dosage value in mg that is stated to one decimal place, then it may refer to a value that is ± 0.05, 0.04, 0.03, 0.02, or 0.01 mg of the stated value.

Optionally, the composition is a pharmaceutical composition, and further optionally, the composition further comprises one or more pharmaceutically acceptable carriers. In some embodiments, the composition consists of the molecule comprising, or consisting of, the Annexin A5 protein and one or more pharmaceutically acceptable carriers. Optionally, in addition to the use as a combination therapy with an anti-VEGF agent, the composition is for use as a combination therapy with one or more other agents. Optionally, the Annexin A5 molecule, or the composition thereof, is used, with or without the one or more other agents, as a first line of treatment for the disease, disorder or conditions to be treated.

In accordance with the third or fourth aspects of the present invention, in some embodiments the molecule comprising, or consisting of, the Annexin A5 protein or the composition comprising said molecule, is for use, and/or is used, as a combination therapy with the one or more other agents, wherein the subject is administered the Annexin A5 molecule in the same composition as, separately from, simultaneously with a separate formulation containing, or sequentially with, the one or more other agents.

Optionally, in accordance with the third or fourth aspects of the present invention, the molecule comprising, or consisting of, the Annexin A5 protein and the anti-VEGF agent are administered in combination, simultaneous in separate administrations, or sequentially in separate administrations. In some embodiments, if the administrations are separate and sequential, then the period of time between the sequential administrations of the Annexin A5 molecule and the anti-VEGF agent is about 28 days or less, such as about 3 weeks or less, about 2 weeks or less, about 1 week or less, about 6, 5, 4, 3, 2 or 1 day or less, such as about 24, 28, 12, 6, 5, 4, 3, 2 or 1 hours or less. However, the reported studies in the present application have demonstrated that an Annexin A5 dosage can delay the requirement for anti-VEGF therapy for much longer periods. As such, in other embodiments, if the administrations are separate and sequential, then the period of time between the sequential administrations of the Annexin A5 molecule and the anti-VEGF agent is greater than about 28 days. For example, as noted above, the Annexin A5 administration may occur over multiple days and may include multiple repeated administrations to the subject. For example, the Annexin A5 molecule may be administered to the subject for two consecutive days or more, such as for three, four, five, six, seven or more consecutive days, and/or or for at least two, three, four, five six or seven days within each 7-day (one week) of 8-day period, such as for a period of at least one, two, three, four weeks or more. In the case that the Annexin A5 administration occurs over multiple days then, in some embodiments, said time periods between the sequential administrations of the Annexin A5 molecule and the anti-VEGF agent are calculated relative to the start date of the Annexin A5 administration. In other embodiments, said time periods are calculated relative to the end date of the Annexin A5 administration.

The present application also provides a molecule comprising, or consisting of, Annexin A5 protein (such as a molecule consisting of monomeric Annexin A5) or composition comprising said molecule, for use, or use, or method as substantially described herein, with reference to the description.

### Definitions

The terms "a molecule comprising, or consisting of, Annexin A5 protein" and "a molecule consisting of monomeric Annexin A5 protein" as used herein is defined further below in section B of the detailed description of the invention. It optionally further includes the meaning of a composition comprising, consisting essentially of, or consisting of, the molecule comprising, or consisting of, Annexin A5 protein (such as the molecule consisting of monomeric Annexin A5 protein). Said composition may, for example, be a pharmaceutically acceptable, or veterinarially acceptable, composition.

A "pharmaceutically acceptable" composition may be a composition that is safe for administration to a subject, such as a human subject, by injection, such as intravenous, subcutaneous or intramuscular injection. Most preferably, the injection is an intravenous injection.

A "veterinarially acceptable" composition may be a composition that is safe for administration to an animal subject, such as by injection, for example by intravenous, subcutaneous or intramuscular injection. Most preferably, the injection is an intravenous injection.

A pharmaceutical composition, or veterinary composition, according to the invention may thus comprise, consist essentially of, or consist of, a molecule consisting of monomeric Annexin A5 protein in admixture with a pharmaceutically or veterinarially acceptable adjuvant, diluent and/or carrier, which will typically be selected with regard to the intended route of administration and standard pharmaceutical practice. The composition may be in the form of immediate-, delayed- or controlled-release applications. The formulation may be a unit dosage, for example a unit dosage containing a daily dose or unit, daily sub-dose or an appropriate fraction thereof, of the active ingredient.

A "sterile injectable intravitreal" composition may be a composition that is safe for administration to a subject, particularly a human subject, such by direct injection into the vitreous of the subject's eye.

A "sterile injectable intravitreal composition according to the invention may thus comprise, consist essentially of, or consist of, a molecule consisting of monomeric Annexin A5 protein in admixture with an adjuvant, diluent and/or carrier, selected for compatibility and/or tolerance when introduced directly into the vitreous of the eye. The composition may be in the form of immediate-, delayed- or controlled-release applications. The formulation may be a unit dosage, for example a unit dosage containing a daily dose or unit of the active ingredient.

The phrases "pharmaceutically acceptable", "pharmacologically acceptable" and "veterinarially acceptable" refer to compositions that do not produce an adverse, allergic or other untoward reaction when administered to an animal, such as, for example, a human, as appropriate. The same considerations apply to sterile injectable intravitreal compositions. The preparation of such compositions are known to those of skill in the art in light of the present disclosure, as exemplified by Remington's Pharmaceutical Sciences, 18th Ed. Mack Printing Company, 1990. Moreover, for animal (*e.g*., human) administration, it will be understood that preparations should meet sterility, pyrogenicity, general safety and purity standards, such as those as required by FDA Office of Biological Standards.

As used herein, "pharmaceutically acceptable carrier" and "veterinarially acceptable carrier" include any and all solvents, dispersion media, coatings, surfactants, antioxidants, preservatives (e.g., antibacterial agents, antifungal agents), isotonic agents, salts, preservatives, drugs, drug stabilizers, excipients, disintegration agents, such like materials and combinations thereof, as would be known to one of ordinary skill in the art. Except insofar as any conventional carrier is incompatible with the active ingredient, its use in the therapeutic or pharmaceutical compositions is contemplated.

The molecule comprising, or consisting of, Annexin A5 protein (such as a molecule consisting of monomeric Annexin A5 protein), or a composition comprising the molecule (such as in admixture with one or more pharmaceutically acceptable carrier, or veterinarially acceptable carriers) can, for example, be administered to the subject parenterally, for example, intravenously, intra-arterially, intraperitoneally, intrathecally, intraventricularly, intrasternally, intracranially, intra-muscularly or subcutaneously, or may be administered by infusion techniques.

In accordance with one option, the administration avoids direct administration to the eye, such as by intravitreal injection.

In another option, the molecule consisting of monomeric Annexin A5 protein may be administered directly to the eye. For example, in one optionally preferred embodiment, the molecule consisting of monomeric Annexin A5 protein may be administered to one, or both, of the eyes of the subject by intravitreal injection. In accordance with this embodiment, the monomeric Annexin A5 protein may be administered to the patient in the form of a sterile injectable intravitreal composition, for example as defined in accordance with the fourth aspect of the present invention.

In another option, the molecule comprising, or consisting of, Annexin A5 protein (such as a molecule consisting of monomeric Annexin A5 protein) may be administered directly to a retinal vein (such as the central retinal vein, the branch retinal vein and/or the hemi-retinal vein) of one, or both, of the eyes of the subject, for example by catheterisation.

Systemic administration, such as Intravenous administration, is a preferred embodiment for the first, second, third and fourth aspects of the present invention. The molecule comprising, or consisting of, Annexin A5 protein (such as a molecule consisting of monomeric Annexin A5 protein), or a composition comprising the molecule may, for example, be used in the form of a sterile aqueous solution which may contain other substances, for example, enough salts or glucose to make the solution isotonic with blood. The aqueous solution should be suitably buffered (preferably to a pH of from 3 to 9), if necessary. For example, optionally, the aqueous solution is buffered at a pH within a range selected from about pH 4 to about pH 8, about pH 5 to about pH 8, about pH 6 to about pH 8, about pH 6.5 to about pH 7.9, about pH 6.8 to about pH 7.7, about pH 7.0 to about pH 7.5, about pH 7.1 to about pH 7.4, about pH 7.1 to about pH 7.4, and optionally about pH 7.1, 7.2, 7.3 or 7.4, most preferably about pH 7.2. In this context, the term "about" when applied to a pH value that is stated to the nearest integer may refer to a value that is ± 0.5, 0.4, 0.3, 0.2, or 0.1 pH units of the stated value; whereas when the term "about" is applied to a pH value that is stated to one decimal place, then it may refer to a value that is ± 0.05, 0.04, 0.03, 0.02, or 0.01 pH units of the stated value. The preparation of suitable parenteral formulations under sterile conditions is readily accomplished by standard pharmaceutical techniques well-known to those skilled in the art.

Formulations suitable for parenteral administration typically include aqueous and non-aqueous sterile injection solutions which may contain anti-oxidants, buffers, bacteriostats and solutes which render the formulation isotonic with the blood of the intended recipient; and aqueous and non-aqueous sterile suspensions which may include suspending agents and thickening agents. The formulations may be presented in unit-dose or multi-dose containers, for example sealed ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules and tablets of the kind previously described.

In one embodiment, the pharmaceutically acceptable, or veterinarially acceptable, composition (for example, as may be used in any of the first, second, third and/or fourth aspects of the present invention) and/or a sterile injectable intravitreal composition (for example as defined in accordance with the fourth aspect of the present invention) may be defined as being safe for injection if it contains no, or substantially no, endotoxin. Endotoxin is often used synonymously with the term lipopolysaccharide, which is a major constituent of the outer cell wall of Gram-negative bacteria. It consists of a polysaccharide (sugar) chain and a lipid moiety, known as lipid A, which is responsible for the toxic effects observed with endotoxin. The polysaccharide chain is highly variable among different bacteria and determines the serotype of the endotoxin and the lipid components are also highly variable such that a single endotoxin sample may contain 10's to 100's of distinct molecular species. Endotoxin is approximately 10 kDa in size but can form large aggregates up to 1000 kDa. Endotoxin is typically harmful and pyrogenic in therapeutic compositions and regulatory authorities have imposed strict limitations on the allowable levels of endotoxin within a pharmaceutical composition. Accordingly, the level of endotoxin in a composition according to the present invention should be minimised and may be less than 100 endotoxin units (EU) per dose, such as less than 90, 80, 70, 60, 50 40, 30, 20, 10, 5, 4, 3, 2, 1 or less EU per dose. The concentration of endotoxin in a composition according to the present invention may be less than 200 EU/m3, such as less than 150, 100, 90, 80, 70, 60, 50, 40, 30, 20, 10, 5, 4, 3, 2, 1 EU/m3. Methods for measuring endotoxin levels, such as the limulus amoebocyte assay (LAL) method, are well known in the art.

A composition comprising the molecule comprising, or consisting of, Annexin A5 protein (such as a molecule consisting of monomeric Annexin A5 protein) may optionally comprise the Annexin A5 molecule as the sole active agent. To put in another way, in one embodiment the composition (or pharmaceutical composition) may consist of a molecule comprising, or consisting of, Annexin A5 protein (such as a molecule consisting of monomeric Annexin A5 protein) and one or more pharmaceutically acceptable carriers or one or more or veterinarially acceptable carriers. For example, it may optionally exclude compositions in which Annexin A5 is formulated with, conjugated to, or expressed as a fusion protein with, one or more further protein or non-protein agents that are capable of providing an additional therapeutic effect. Said additional therapeutic effect may either be in the context of macular oedema and/or retinal vein occlusion or in the context of a therapeutic effect that is not relevant to macular oedema and/or retinal vein occlusion treatment. For the avoidance of doubt, the molecule consisting of monomeric Annexin A5 protein is not a dimeric Annexin A5 (commonly referred to in the art as "Diannexin"), and is not a PEGylated Annexin A5.

### Other Definitions:

The term "treatment" would be understood by those skilled in medicine. The term "treatment" as used herein may include any treatment of a condition, (for example, wherein the condition is macular oedema , retinal vein occlusion, a condition associated with macular oedema or retinal vein occlusion), in a subject, particularly a human or other mammal, and optionally includes one or more of the following effects:
(i) inhibiting the condition (for example, macular oedema or retinal vein occlusion and/or other disease, disorder or condition associated with, and/or caused by, the macular oedema or RVO), i.e., slowing, reducing or arresting the development of the condition;
(ii) relieving the condition (for example, macular oedema or retinal vein occlusion and/or other disease, disorder or condition associated with, and/or caused by, the macular oedema or RVO), i.e., causing regression of the condition in a subject having the condition; or
(iii) curing the condition (for example, macular oedema or retinal vein occlusion and/or other disease, disorder or condition associated with, and/or caused by, the macular oedema or RVO), i.e. returning a subject having the condition, to a state of health in which the condition, is no longer detectable.

The term "prophylaxis" would be understood by those skilled in medicine. The term "prophylaxis" as used herein may include any prophylactic treatment of a condition (for example, wherein the condition is macular oedema, retinal vein occlusion, a condition associated with macular oedema or RVO) in a subject, particularly a human or other mammal, and optionally includes one or more of the following effects:
(i) preventing the condition (for example, macular oedema or retinal vein occlusion and/or other disease, disorder or condition associated with, and/or caused by, the macular oedema or RVO) from occurring in a subject (for example, a subject which may be predisposed to, or at risk of, developing the condition, but has not yet been diagnosed as having it) i.e. stopping the subject from developing the condition;
(ii) delaying the onset of the condition (for example, macular oedema or retinal vein occlusion and/or other disease, disorder or condition associated with, and/or caused by, the macular oedema or RVO) in a subject, i.e. delaying a subject from developing the condition until later in the life of the subject;
(iii) limiting the occurrence of the condition (for example, macular oedema or retinal vein occlusion and/or other disease, disorder or condition associated with, and/or caused by, the macular oedema or RVO) in a subject, for example reducing the extent to which a subject is affected by the condition; or
(iv) preventing one or more symptoms of the condition (for example, macular oedema or retinal vein occlusion and/or other disease, disorder or condition associated with, and/or caused by, the macular oedema or RVO) in a subject, i.e. stopping the subject from developing one or more symptoms of the condition.

In the case of prophylaxis of another disease, disorder or condition associated with, and/or caused by, the macular oedema or RVO, the subject may optionally already be suffering from, suspected of suffering from, and/or be diagnosed as suffering from, the macular oedema or RVO.

The terms "optionally", "preferably", "such as", "for example" and the like denote that the subsequently described feature, characteristic, event or circumstances may or may not occur, and that the description includes instances where said feature, characteristic, event or circumstances occurs and instances in which it does not occur. For the avoidance of doubt, it is to be understood that, in some embodiments of the invention, any feature, characteristic, event or circumstance described in the context of "optionally", "preferably", "such as", "for example" and the like may be disclaimed from the scope of the claims.

The term "subject" as used herein includes the meaning of any living human or animal to which the treatment of the present invention is applied. Without limitation, exemplary subjects are defined further below in section C of the detailed description of the invention. Most preferably, the subject is a human subject.

The term "elevated" as used herein includes the meaning that the substance (for example, a cell type such as red blood cells (*i.e.* erythrocytes), or a marker on a cell such as Annexin A5 binding sites on red blood cells) is present in a higher amount in a subject with macular oedema and/or retinal vein occlusion when compared to the amount present in a subject, or an average of a group of subjects (e.g. at least 10,000 or 1000 subjects), without macular oedema and/or retinal vein occlusion.

The term "distal" as used here includes the meaning that administration of the molecule consisting of monomeric Annexin A5 protein is not administration directly in, or to, the eye which has been diagnosed with macular oedema and/or retinal vein occlusion.

The term "therapeutically effective amount" refers to the amount of a drug, compound, protein (for example the molecule comprising, or consisting of, the Annexin A5 protein as defined herein) or pharmaceutical composition that is sufficient to effect beneficial or desired results including clinical results associated with the treatment or prophylaxis of macular oedema, retinal vein occlusion, a disease, disorder or condition associated with, and/or caused by, the macular oedema and/or retinal vein occlusion. An effective amount can be administered in one or more administrations. For purposes of this invention, an effective amount of drug, compound, protein (for example the molecule comprising, or consisting of, the Annexin A5 protein as defined herein), pharmaceutical composition or veterinary composition is an amount sufficient to treat, ameliorate, reduce the intensity of and/or prevent macular oedema and/or retinal vein occlusion and/or other disease, disorder or condition associated with, and/or caused by, the macular oedema and/or retinal vein occlusion. As is understood in the clinical context, an effective amount of a drug, compound, protein (for example the molecule comprising, or consisting of, the Annexin A5 protein as defined herein) or pharmaceutical composition may or may not be achieved when administered in conjunction (such as separately, sequentially or simultaneously) with another drug, compound, protein or pharmaceutical, such as those described elsewhere in this application. Thus, an "effective amount" may be considered in the context of administering one or more therapeutic agents, and a single agent may be considered to be given in an effective amount if, in conjunction with one or more other agents, a desirable result may be or is achieved.

### Description of the Figures:

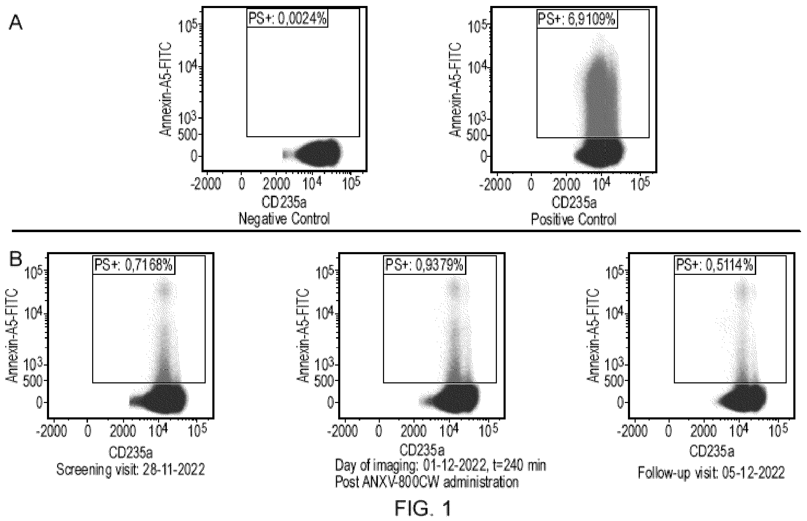

**Figure 1** shows the results of a phosphatidylserine (PS)-assay in RBC from a patient with RVO. This flow cytometry in vitro assay uses FITC-Annexin A5 ("ANXV") and PE-anti-CD235a as a positive marker for RBCs. Panel A: In the negative control Ca²⁺ is absent, whereas in all other samples Ca²⁺ is present to enable FITC-Annexin A5 binding in vitro. Panel B: shows the PS-externalization on RBC at the following time points: screening, imaging day, follow-up.

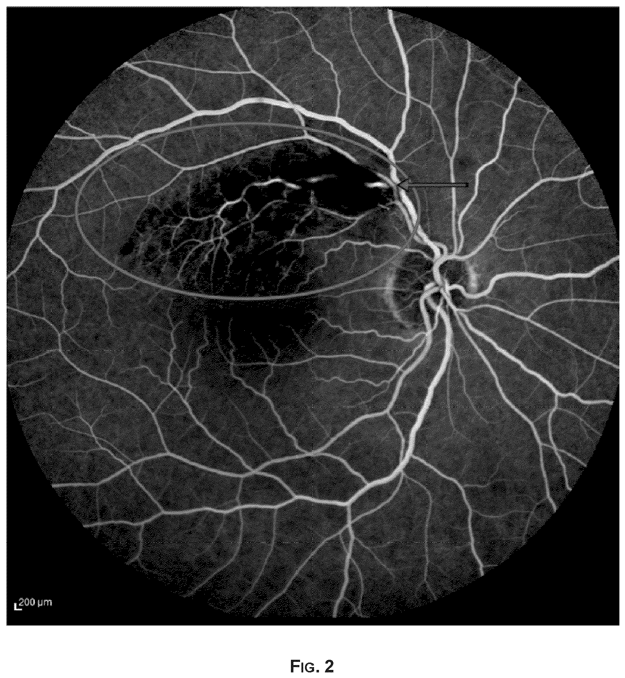

**Figure 2** shows fluorescein angiography (FA) performed using intravenously administered fluorescein prior to systemic administration of a 1 mg flat-dose ANXV-800CW. The red arrow marks the area of occlusion. The darker area, marked by the red oval, corresponds to the damaged area with bleeding and ischemia being present.

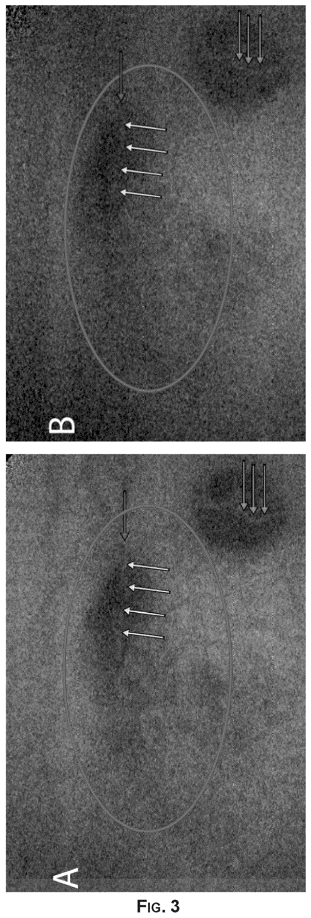

**Figure 3** shows near-infrared (NIR) fluorescence image made at t=5 minutes after systemic injection of 1.0 mg ANXV-800CW (Panel B). NIR mage a t=229 minutes (Panel A). The red arrow indicates the site of the occlusion, while the yellow arrows mark the vein before the site of the occlusion in the damaged area where bleeding is present. The light blue arrow indicates a large vein in the optic nerve. The darker area, surrounded by the red oval corresponds to the damaged area with bleeding present. This oval marks approximately the same anatomical location as the red oval in Figure 2. The used settings of the Heidelberg Spectralis camera system are similar for panel A and B.

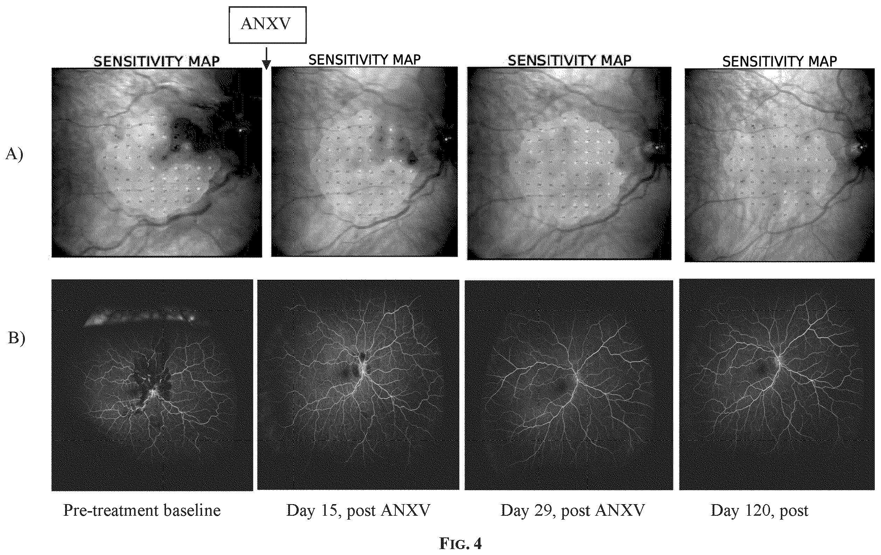

**Figure 4: (A)** shows a microperimetry (MMP) acquired retinal sensitivity map for a treatment-naive RVO patient (subject F), treated with ANXV therapy as a first line therapy. The findings demonstrate clear improvement at 15 days post treatment, through to complete recovery by 120 days post-treatment, without having received anti-VEGF therapy during the study period. **(B)** shows the outcome of imaging of blood flow in the retinal vasculature by fluorescein angiography (FA) for subject F during the same study period, and demonstrates clear improvement using ANXV therapy at 15 days post treatment, through to complete recovery by 29 or 120 days post-treatment.

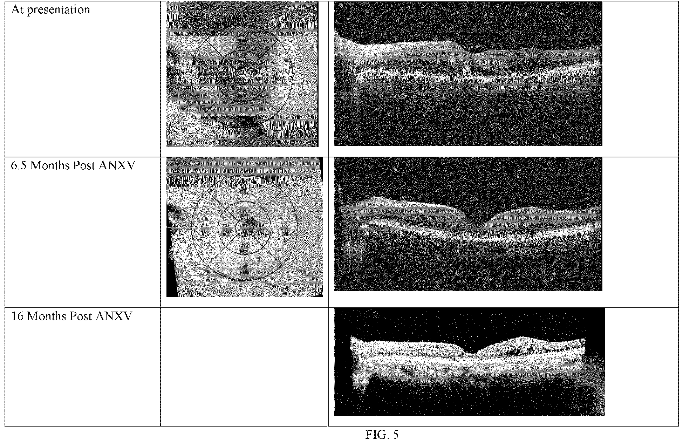

**Figure 5** shows the OCT (optical coherence tomography) measurement of macular retinal thickness for a treatment-naïve RVO patient (subject A), when followed over a study period of 16 months, when treated with ANXV therapy as a first line therapy at the start of the study, and receiving a single anti-VEGF injection about 32 days into the study. At 16 months, visual acuity was found to be stable as 20/60, despite having received only a single anti-VEGF treatment during the study period.

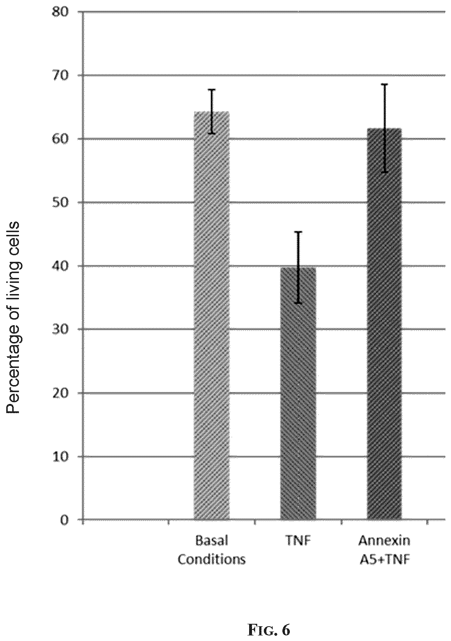

**Figure 6** shows that the application of TNFα led to a reduction in cell viability (column two) compared to control cells to which TNFα had not been applied (column one), whereas the application of Annexin A5 in addition to TNFα inhibits cell death (column three), leading to a cell viability equivalent to levels seen in the control.

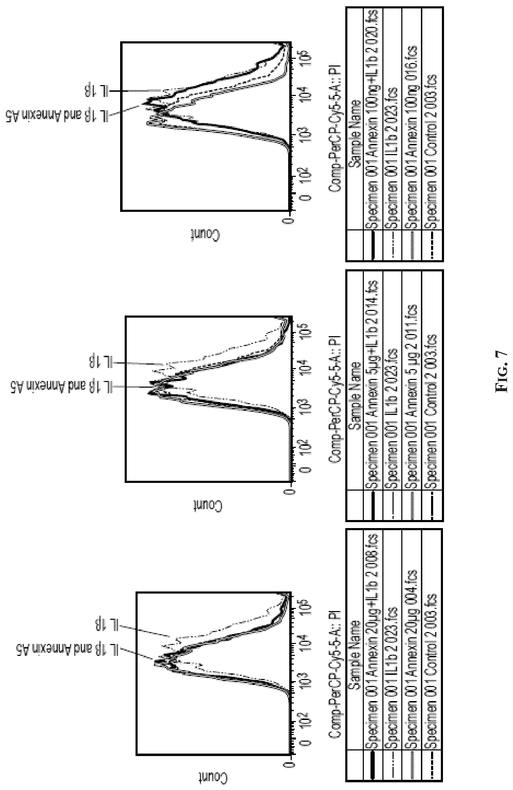

**Figure 7** shows that cell viability is increased on treatment with Annexin A5 protein, in a dose dependent manner. The application of IL1β led to a reduction in cell viability. This is shown in the figure as for each experiment the flow cytometry curve for the sample to which only IL1β (marked on the figure) has been applied is shifted to being the furthest curve from the y-axis, whilst the control cells to which IL1β has not been applied are at the centre of the graph. This particular positioning of those curves shows that cell death occurs at a higher level when IL1β has been applied. Also shown is that application of Annexin A5 protein alone at all three dosages does not cause an increase in cell death when compared to the control sample, as the control curve and the Annexin A5 protein curve for all three dosages are at the same position. Lastly, application of Annexin A5 in addition to IL1β inhibits cell death in a dose dependent manner, with dosages of 20µg and 5µg exhibiting a cell viability at the same levels as observed in the control. This is shown as the flow cytometry curves for 20µg and 5µg dosages of Annexin A5 are at the centre of the graph (marked on the figure), in line with the control curve and the Annexin A5 protein alone curve. The 100ng dosage also inhibits cell death but to a lesser extent, which can be seen as the 100ng flow cytometry curve (marked on the figure) is between the middle of the graph and the IL1-β alone curve.

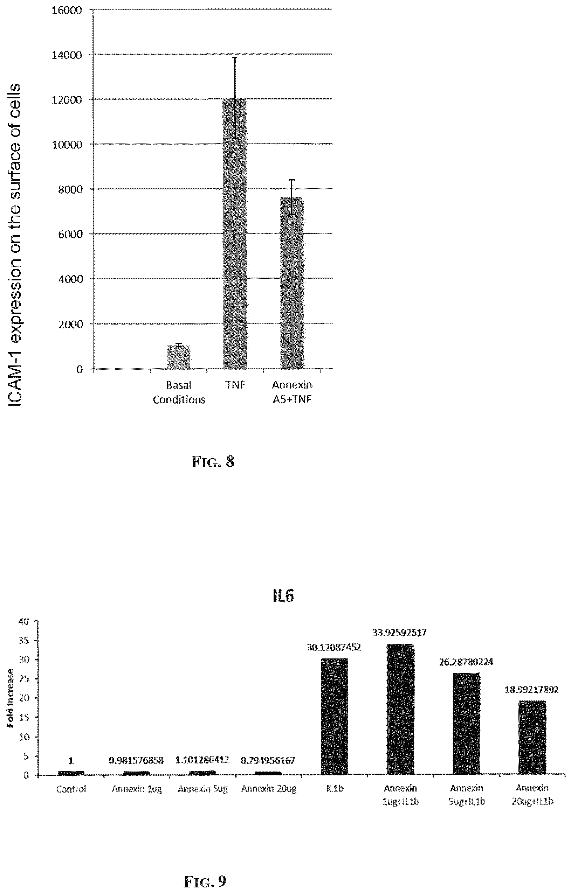

**Figure 8** shows that the expression of ICAM1, is decreased on treatment with Annexin A5. The experiment has been undertaken on a cell culture using flow cytometry. TNFα application increased surface expression of ICAM1 (column two) when compared to control cells to which TNFα had not been applied (column one). Application of Annexin A5 in addition to TNFα decreased the surface expression of ICAM1 (column three).
**Figure 9** shows that the expression of IL-6 is decreased on treatment with Annexin A5, in a dose dependent manner. The application of IL-1β led to an increase in IL-6 expression (column five) when compared to the control samples to which IL-1β has not been applied (column one). Application of Annexin A5 protein alone at all dosages did not increase the expression of IL-6 (columns one to four). Application of Annexin A5 in addition to IL-1β decreases IL-6 gene expression in a dose dependent manner (columns five to eight).

### Detailed Description of the Invention

The established standard of care for macular oedema and/or RVO, and indeed in multiple other retinal diseases, by which therapeutic agents (such as anti-VEGF therapeutics) are delivered directly into the eye, is by intravitreal injection (Haydinger *et al,* 2023, *supra;* Ip & Hendrick, 2018, *supra;* Cox et al., 2021, J. Clin. Med., 10(5): 981). Such medications are injected directly into the eye because the retina is located at the back of the eye and is surrounded by several protective layers, including the cornea, the iris, and the lens. These layers can make it difficult for medications to reach the retina when they are administered by other routes. When medications are injected into the eye, they are delivered directly to the retina and can act locally to target the source of the problem. In part, the present invention is based on the surprising findings that Annexin A5 protein, when administered to a subject systemically (such as by intravenous administration), is able to preferentially localise to the site of an occlusion in a subject with retinal vein occlusion (RVO), is able to provide a therapeutic effect in the treatment of RVO.

It has also been determined that Annexin A5 provides biological activity that modulates various factors that have been identified as contributing to dysregulation of the blood-retinal barrier (BRB) in subjects with macular oedema and/or RVO, as well as contributing other therapeutic benefits in subjects with macular oedema and/or RVO. The inventors have also determined that prophylaxis and/or treatment of macular oedema, retinal vein occlusion, and/or other diseases, disorders or conditions associated with, and/or caused by, the macular oedema and/or retinal vein occlusion, can be achieved with a range of doses of Annexin A5, including (but not limited to) monomeric Annexin A5 protein.

A further particularly surprising finding is that Annexin A5 therapy is able to substantially reduce the burden for downstream anti-VEGF therapy. Anti-VEGF therapy is established in the treatment of various ocular conditions, including macular oedema, RVO, and conditions associated with each.

Accordingly, the present invention provides for the use of Annexin A5 protein and anti-VEGF in combination therapies, for example in the context of the prophylaxis or treatment of conditions such as macular oedema, RVO, conditions associated with each, as defined in the claims.

### A. Macular Oedema

Macular oedema (also referred to as macular edema) is the pathological accumulation of fluid in the macula, the central region of the retina essential for high-acuity vision. It is a complication common to many ocular diseases, such as diabetic retinopathy, retinal vascular occlusions, uveitis, post-cataract surgery inflammation (pseudophakic macular oedema or Irvine-Gass syndrome), retinal dystrophies, drug reactions, intraocular tumors, serous central chorioretinopathy, radiation retinopathy and other retinal vascular abnormalities including retinal arterial macroaneurysms and retinal telangiectasia (Tranos et al., 2004, Surv Ophthalmol, 49:470-90). Subretinal neovascular membranes observed in conditions such as age-related macular degeneration (AMD) are also associated with intraretinal and subretinal fluid accumulation (Trichonas et al., 2014, Br J Ophthalmol. 98:ii24).

Symptoms of macular oedema include metamorphopsia, micropsia, blurred vision, a central scotoma, and reduction of contrast or colour sensitivity (Haydinger *et al.,* 2023).

Optionally, the treatment of macular oedema, or a condition associated therewith, in accordance with the various aspects of the present invention, is started within 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the onset of one or more symptoms of the macular oedema. For example, the combination therapy treatment may be started by a first administration of the Annexin A5 component of the co-therapy, or by a first administration of the anti-VEGF agent component of the co-therapy, or by a simultaneous first administration of both components either as separate compositions or combined in the same formulation.

The clinical diagnosis of macular oedema can be challenging in mild cases or when visualization of the fundus is impaired by poor pupillary dilation, cataract and other ocular media opacities. Therefore, fluorescein angiography and optical coherence tomography (OCT) are commonly employed to make the diagnosis. The latter is preferred due to its non-invasive nature, accurate measurements of retinal thickness and capacity for identifying other structural abnormalities, such as epiretinal membrane and vitreomacular traction, and it is the standard method for evaluating macular oedema in clinical trials.

Accordingly, in a preferred embodiment, a subject that has been diagnosed as having macular oedema is a subject in which the macular oedema is diagnosed by fluorescein angiography and/or optical coherence tomography.

Optionally, the treatment of macular oedema, or a condition associated therewith, in a subject, in accordance with the present invention, is started within 1, 2, 3, 4, 5, 6, 7, 8, 9, 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the diagnosis of the macular oedema. For example, the combination therapy treatment may be started by a first administration of the Annexin A5 component of the co-therapy, or by a first administration of the anti-VEGF agent component of the co-therapy, or by a simultaneous first administration of both components either as separate compositions or combined in the same formulation.

The thickness of the retina is generally measured between the inner limiting membrane and Bruch's membrane. By current spectral-domain OCT technology, the average and central (foveal) macular thicknesses in normal adult eyes are 334 and 226 µm, respectively, being thicker in males than in females. Three patterns of macular oedema have been described using OCT: cystoid macular oedema, diffuse macular oedema, and subretinal detachment. Diffuse retinal thickening is the main pattern in diabetic macular oedema, while in retinal vascular occlusions, accumulation of subretinal fluid is common. In uveitis, cystoid and diffuse macular oedema are the typical forms of macular oedema, whereas subretinal detachment is seen in less than one-third of cases, usually in combination with cystoid or diffuse oedema In some embodiments of the present invention, the macular oedema is selected from the group consisting of one or more of cystoid macular oedema, diffuse macular oedema, and subretinal detachment.

The distortion of the retinal architecture caused by fluid accumulation in the macula results in vision loss, and potentially irreversible visual impairment due to scarring when the condition becomes chronic. Macular oedema is the main cause of vision loss in patients with diabetes mellitus, retinal vein occlusions, and uveitis. Populational studies have shown a mean prevalence of diabetic macular oedema of around 6% in type 1 diabetes, and slightly higher in type 2 diabetes. Macular oedema is extremely common in central retinal vein occlusions, while in branch retinal vein occlusion, macular oedema occurs in 30% of cases. Around one-third of patients with uveitis develop macular oedema, being more frequent in intermediate uveitis and panuveitis, and less common in anterior uveitis. The burden of macular oedema not only relates to the decrease in quality of life experienced by patients, but also to the chronicity of the condition and the economic costs associated with treatment and frequent visits to medical centers. In uveitic macular oedema in particular, the most affected population group is of working age, adding to the socioeconomic impact of the condition.

In some embodiment of the present invention, a subject having, diagnosed as having, or suspected as having macular oedema may be a subject that has, has been diagnosed as having, or is suspected of having one or more conditions selected from diabetes mellitus, diabetic retinopathy, retinal vein occlusion (for example, CRVO, BRVO and/or HRVO), and/or uveitis (for example, intermediate uveitis and/or panuveitis).

In some embodiment of the present invention, a subject having, diagnosed as having, or suspected as having macular oedema may be a subject that has had, is receiving, or will receive, an associated therapy for macular oedema, for example a treatment selected from the group consisting of anti-VEGF therapy (e.g. anti-VEGF therapy delivered into the eye by intravitreal injection), corticosteroids, NSAIDs, pars plana vitrectomy, retinal laser photocoagulation, or anti-VEGF therapy that is augmented with intravitreal corticosteroid and/or retinal laser photocoagulation.

Exemplary anti-VEGF therapies can include any one or more of:
- Ranibizumab, an FDA-approved anti-VEGF drug, which is a recombinant humanized antibody fragment against VEGF,
- Aflibercept, another FDA-approved anti-VEGF drug, which is a recombinant protein comprising the ligand-binding domains of VEGF receptors 1 and 2 fused to the Fc portion of immunoglobulin G1,
- Bevacizumab, a humanized monoclonal antibody against VEGF, which is widely used off-label (Haydinger *et al,* 2023, *supra;* Ip & Hendrick, 2018, *supra),* and/or
- Brolucizumab and/or faricimab, which are newer anti-VEGF drugs.

Corticosteroids are commonly used in managing macular oedema especially in subjects with non-infectious uveitis. Exemplary corticosteroids include difluprednate, triamcinolone acetonide, triamcinolone acetonide, and dexamethasone which may be used as monotherapy, or in combination with systemic immunomodulatory therapy to achieve a more rapid resolution of the oedema. The most common complications of local corticosteroid treatments are elevated intraocular pressure and cataract. Systemic use of corticosteroids is indicated mainly for bilateral macular oedema and should be used for a limited time due to the extensive list of side effects (Foster et al., 2016, Surv Ophthalmol, 61:1-17**).**

Non-steroidal anti-inflammatory (NSAID) drugs have an adjunct role in the treatment approach, and mostly are used for pseudophakic macular oedema. Acetazolamide, an inhibitor of carbonic anhydrase (CA), is occasionally employed in cases of failed corticosteroid therapy, but sometimes is used as primary therapy for pseudophakic macular oedema or macular oedema associated with retinal dystrophies.

Pars plana vitrectomy is mostly reserved for treating mechanical factors contributing to macular oedema, such as epiretinal membranes and vitreoretinal traction. It is the last resort for the treatment of macular oedema without mechanical factors, owing to its inherent risks, which include retinal breaks and detachment, vitreous hemorrhage, cataract, glaucoma and hypotony, and limited data on outcomes.

Fluid can accumulate diffusely in the central retina or within cysts usually localized in the inner nuclear layer or Henle's fiber layer, or in the subretinal space, disrupting and distorting the retinal architecture and decreasing visual acuity. In combination with the underlying pathology, the oedema can progress to cause irreversible tissue damage with death of retinal cells, and permanent visual impairment. The common feature of most diseases that cause macular oedema is dysfunction of the blood-retinal barrier (BRB), a set of structures that, in health, strictly regulates the passage of proteins, salts, metabolites and other solutes between the blood and the retinal tissue. Vasoactive growth factors and inflammatory cytokines, including vascular endothelial growth factor-A (VEGF) and tumor necrosis factor (TNF)-α, are upregulated in the ocular fluids in macular oedema and have been implicated in disruption of the BRB. Dysregulated entry and accumulation of solutes in the retina disturb the balance of osmotic and hydrostatic forces, and lead to fluid entry when mechanisms maintaining fluid homeostasis are overcome. Current treatments target proteins and processes that are involved in angiogenesis, inflammation and blood-retinal barrier (BRB) dysfunction. This review provides an overview of clinical aspects of macular oedema and discusses cellular and molecular mechanisms that are targeted by current treatments.

Conditions associated with macular oedema include, without limitation, any one or more of dysregulation of the blood-retinal barrier (BRB), such as dysregulation of the inner BRB and/or the outer BRB, dysfunction of barriers between the vitreous and blood vessels at the ciliary body, macularedema-associated ocular diseases, such as diabetic retinopathy, retinal vascular occlusions, uveitis, post-cataract surgery inflammation (pseudophakic macular oedema or Irvine-Gass syndrome), retinal dystrophy, drug reactions, intraocular tumors, serous central chorioretinopathy, radiation retinopathy and other retinal vascular abnormalities including retinal arterial macroaneurysms and retinal telangiectasia, changes to subretinal neovascular membranes observed in conditions such as age-related macular degeneration (AMD), metamorphopsia, micropsia, blurred vision, central scotoma, and reduction of contrast or colour sensitivity.

### Mechanisms of macular oedema

Macular oedema is the outcome of the failure of mechanisms maintaining fluid homeostasis in the macula (Haydinger *et al,* 2023, *supra).* The movement of fluid across capillaries throughout the body is classically described by Starling forces. A hydrostatic pressure gradient between the intravascular space and the tissue extracellular space tends to drive fluid into the tissue, and this is opposed by a colloid osmotic (oncotic) pressure gradient which tends to draw fluid out of the extracellular space into the blood vessels. A recent review by Cunha-Vaz discusses macular oedema formation in the context of classic Starling forces *(*Ophthalmologica, 2017, 237:1-10). A relative increase in hydrostatic pressure in vessels versus the retinal tissue and a relative increase in oncotic pressure in the retinal tissue versus the vessels are the two forces that drive oedema. Of these, macular oedema in most diseases is thought to result from increased tissue oncotic pressure due to aberrant accumulation of solutes in the retinal extracellular space (Haydinger *et al,* 2023, *supra).* The compliant nature of the retinal tissue, which can expand unimpeded into the vitreous, renders it particularly susceptible to oedema as rises in tissue oncotic pressure readily incur increases in tissue volume.

The dysregulated entry of solutes to the retina occurs due to disruption of the blood-retinal barrier (BRB), and this has been the main focus of most efforts to understand the mechanisms of this condition (Haydinger *et al,* 2023, *supra).*

The BRB is a group of structures in the eye that separates the blood from the neural retina and retinal extracellular space. There are two components: the inner BRB, and the outer BRB. The inner BRB is based around the retinal endothelial cells (REC) which line the retinal blood vessels, and the outer BRB is formed at the level of the retinal pigment epithelial (RPE) cells. As there can be non-selective bidirectional diffusion of solutes between the retinal extracellular space and the vitreous, barrier processes between the vitreous and blood vessels at the ciliary body might also be considered to form part of the BRB, although this region is not grouped in either the inner or outer BRB.

The walls of the retinal vessels form the inner BRB. The luminal surface of retinal blood vessels is uninterruptedly lined by a squamous layer of RECs, and the membranes of adjacent RECs are held together by a continuous network of tight junctions. The RECs are not fenestrated, and they contain few pinocytic vesicles. The RECs and their junctions form a barrier that facilitates the regulated passage of solutes between the blood and the retina (Cunha-Vaz et al., 1966, Br J Ophthalmol., 50:441-53). Basement membrane completely covers the abluminal surface of the RECs. Evidence collected *in vivo* indicates the basement membrane does not directly constitute a barrier, although some evidence indicates it can impede diffusion of molecular tracers in vitro. Larger vessels have additional layers of smooth muscle, basement membrane, and adventitia, differing in structure depending on the size and type of vessel.

The outer BRB is not situated at a vascular wall, but rather is formed by the retinal pigment epithelium, a monolayer of RPE cells situated behind the photoreceptors and immediately opposed to Bruch's membrane which separates it from the vascular bed of the choriocapillaris (Haydinger *et al,* 2023, *supra).* In contrast to the inner BRB, the vessels of the choroid have thin and highly fenestrated endothelial cells, and do not form a selective barrier. As such, the epithelium is responsible for maintenance of the regulated environment required for retinal function.

Dysfunction of the BRB underlies development of macular oedema (Haydinger *et al,* 2023, *supra).* Loss of selectivity of the exchange of molecules between the blood and retinal tissue at the level of either the inner or outer BRB permits entry of proteins and other solutes to the retinal tissue. Leakage is usually evident well prior to macular oedema formation. It is likely that oedema eventuates when the severity of leakage overcomes solute and fluid clearance mechanisms. The rate of clearance of solutes from the subretinal space is inversely proportional to their size. Injection of variously sized, tagged dextran tracers into patients with uveitic macular oedema showed rates of leakage roughly proportional to the size of the tracer. Sizes up to 20 kDa leaked into the macula, but 150 kDa did not (Atkinson et al., 1991, Eye, 5:440-6). These data indicate that, while infiltration of larger solutes likely poses a greater challenge for clearance, complete BRB breakdown permitting non-selective entry of the largest solutes is not necessary for oedema to form.

The evidence suggests that several inflammatory cytokines act to disrupt BRB integrity (Haydinger *et al,* 2023, *supra;* Park et al. 2019, J Diabetes Res, 2019: 813741; Noma et al., 2020, J. Clin.Med, 9(1): 3457).

VEGF plays a major role in driving progression of retinal diseases and macular oedema, which is irrefutably evidenced by the broad utility of anti-VEGF therapies (Haydinger *et al, 2023, supra; ;* Park *et al.* 2019, *supra;* Noma *et al.,* 2020, *supra).*

Intravitreal levels of VEGF and IL-6 correlate with the severity of macular oedema and the size of the non-perfusion area (Noma et al., 2009, Ophthalmology, 116:87-93; Noma et al, 2006, Graefes Arch Clin Exp Ophthalmol., 244:309-15). Evidence implicates IL-6 in diseases leading to macular oedema (Haydinger *et al,* 2023, *supra).* It is elevated in patients with uveitis (van Kooij et al., 2006, Am J Ophthalmol. 142:192-4). It is elevated in diabetic macular oedema (Funatsu et al., 2002, Am J Ophthalmol. 133:70-7) and correlates with macular thickness (Oh et al.,2010, Curr Eye Res. 35:1116-27). IL-6 is also upregulated in patients with central retinal vein occlusion, especially those with ischemia (Noma et al., 2009, Ophthalmology. 116:87-93). Its expression often correlates with that of VEGF (Haydinger *et al,* 2023, *supra).* Furthermore, it is proposed that VEGF increases vascular permeability and further expression of inflammatory cytokines, including MCP-1 and ICAM-1 which disrupt the BRB, thus causing the onset and progression of macular oedema (Noma *et al.,* 2020, *supra).*

Tumor necrosis factor-a (TNF-α) and interleukin-1β (IL-1β) are also implicated in the mechanism of BRB dysfunction in macular oedema (Haydinger *et al,* 2023, *supra).*

TNF-α is a master inflammatory cytokine which has been shown to increase barrier permeability of REC monolayers in vitro (Haydinger *et al,* 2023, *supra;* Aveleira et al., 2010, Diabetes. 59:2872-82). When injected into the vitreous in rabbits, TNF-α increased the proportion of REC tight junctions that appeared open, and albumin was detected within these junctions, indicating leakiness (Luna et al., 1997, J Neurosci Res. 49:268-80). Larger vesicles also stained positively for albumin in RECs in treated eyes, but not controls. These results indicate breakdown of both transcellular and paracellular pathways. Interestingly, 24 h after injection, TNF-α-induced tight junction changes had reversed, a promising sign for a potential treatment target. When injected into the vitreous in rats, it also induced barrier leakage, and the effect of injection recovered after 7 days (Bamforth et al., 1996, Acta Neuropathol. 91:624-32). Huang et al. used two mouse models of diabetes to assess the pathological role of TNF-α *(*Ophthalmol Vis Sci. (2011) 52:1336-44); vascular leakage late in disease was completely prevented by TNF-α knockout.

Along with TNF-α, another master inflammatory cytokine which is of increasing interest is IL-1β. Similar to TNF-α, when injected into the rabbit vitreous, IL-1β increases the number of open, leaky tight junctions (140). It also increases the permeability of REC monolayers in vitro (Aveleira *et al.,* 2010, *supra).* In streptozotocin-diabetic rats, retinal IL-1β expression increased between 1.5 and 4.5 months of diabetes, and IL-1β mRNA is induced by high glucose in bovine RECs in vitro (Liu et al., 2012, PLoS One. 7:e36949); it induces its own expression in RECs, Müller cells and brain astrocytes in vitro, which is prevented by inhibition of protein kinase C.

### B. Retinal Vein Occlusion

### Types of retinal vein occlusion

Aspects of the present invention are directed to the prophylaxis and treatment of retinal vein occlusion, and/or one or more diseases, disorders or conditions associated with, and/or caused by, the retinal vein occlusion.

Retinal vein occlusion (sometimes referred to as an eye stroke) is a common vascular disorder of the retina, and is one of the most common causes of a loss of vision in the world. It is generally considered that retinal vein occlusion is caused by obstruction *(i.e.* blockage) of the retinal venous system, such as by the formation of a thrombus. Other possible causes of retinal vein occlusion are thought to be obstructions of the veins caused by external pressure on the veins of the retina or disease of the retinal vein walls, such as by vasculitis ("Retinal Vein Occlusion (RVO) Guidelines" *supra).*

It is known that red blood cells can clot to form thrombi, and that this is sometimes mediated by exposed phosphatidylserine on the red blood cells' surface. Without being bound by theory, the applicant has characterised that a role of Annexin A5 in masking (put in another way, covering or shielding) the phosphatidylserine on the surface of those red blood cells can avoid and treat the thrombi formation in retinal vein occlusion. By characterising the role of exposed phosphatidylserine in thrombi formation in retinal vein occlusion, the applicant has identified that it is possible to treat patients with substantially less monomeric Annexin A5 than had previously been considered to be effective in the art.

In one embodiment, the retinal vein occlusion is caused by an obstruction of a vein. For example, the obstruction of the retinal vein may be associated with one or more of the following: the formation of a thrombus (or thrombi); an observably enhanced erythrocyte aggregation and/or blood hyperviscosity; external pressure on a vein; and disease of the wall of a vein (such as vasculitis). Most preferably, the obstruction of the retinal vein is associated with the formation of a thrombus.

By "associated" we include that the retinal vein occlusion is caused by a particular mechanism (such as the formation of a thrombus); and/or it is suspected that the retinal vein occlusion is caused by a particular mechanism; and/or it is diagnosed that the retinal vein occlusion is caused by a particular mechanism. It would be known to one skilled in medicine how to identify whether the retinal vein occlusion is associated with a particular mechanism, as discussed herein.

As would be known to one skilled in medicine, a thrombosis is generally considered to be a blood clot formed within a blood vessel (such as a vein) by an aggregation of platelets and/or red blood cells, which impedes the flow of blood. Accordingly, by "thrombus" we include a blood clot formed within a blood vessel (such as the vein) which impedes blood flow.

A subject may be categorised as having observably enhanced erythrocyte aggregation and/or blood hyperviscosity by any appropriate method. The enhancement is compared to a heathy subject, or to the mean average for group of healthy subjects (such a group of 10, 50 or 100 healthy subjects). For example, the level of erythrocyte aggregation and/or blood hyperviscosity may be measured by determining the level of erythrocytes adhesion to endothelial cells. A suitable methodology is, for example, described in Wauter et al, 2011, J. Thrombosis and Hemostasis, 9: 1049-1055.

More particularly, in one option, the level of erythrocytes adhesion to endothelial cells may be determined under static conditions. This may, for example, involve using a radiometric technique as described Kuypers et al, 1996, Blood, 87: 1179-87. Human microvascular endothelial cells (HMEC-1) may be cultured; washed erythrocytes were resuspended (for example, to hematocrit 25% in Hanks Balanced Salt Solution (HBSS) plus 0.5% human serum albumin suitably labeled (e.g. with ⁵¹chromium), and incubated with HMEC (for example, for 30 min at 37°C). Non-adhering erythrocytes may be removed by washing, and the remaining adherent erythrocytes detected. Such detection may, for example, including lysing the remaining adherent erythrocytes (e.g. with distilled water) and measuring the radioactivity in the lysate. Using such a technique, adhesion may, for example, be calculated as number of erythrocytes adhered per mm².

Under such static conditions, for example, heathy individuals may typically display adhesion of 3,700 ± 300 erythrocytes per mm², whereas more commonly a patient with CRVO may display 13,500 ± 700 erythrocytes per mm². A subject for treatment in accordance with the present invention may optionally be categorised as having erythrocytes that display greater than 4,000, such as about 4,500, about 5,000, about 5,500, about 6,000, about 6,500, about 7,000, about 7,500, about 8,000, about 8,500, about 9,000, about 9,500, about 10,000, about 10,500, about 11,000, about 11,500, about 12,000, about 12,500, about 13,000, about 13,500, about 14,000, about 14,500, about 15,000, about 15,500, about 16,000, about 16,500, about 17,000, or more erythrocytes per mm² when assessed for adhesion to endothelial cells under static conditions, for example when determined by a technique as described above. The term "about" in this context refers to a value that is ± 500, 400, 300, 200, 100, 50 or less erythrocytes per mm² of the stated value.

In another option, the level of erythrocytes adhesion to endothelial cells may be determined under flow conditions. This may, for example, involve the adhesion of erythrocytes to HMEC under flow conditions as determined according to a Wautier et al, 2007, Blood, 110: 894-901. For example, HMEC may be cultured (such as in gelatin (2%) coated glass capillaries, for for 24 h), microslides containing confluent HMEC then mounted on the stage of a video-microscope, and an erythrocyte suspension (e.g. hematocrit 0.5%) perfused through the microslide at a suitable flow rate (such as a rate equivalent to a wall shear stress of 0.03 Pa) for a suitable time (e.g. for 10 mins), followed by a washout of non-adherent cells (e.g. for 10 min). The wall shear stress may then be increased stepwise (e.g. from 0.03 to 0.3 Pa, every 5 min). At each step, adherent erythrocytes may be recorded (e.g. by video-imaging in 10 consecutive fields, wherein one field measures 0.1 mm²), and counted (e.g. using a computerized image analysis system), and the results may be expressed as number of erythrocytes per mm².

Under such flow conditions, for example, when adhered at 0.03 Pa and analysed for wash out at 0.07 Pa, heathy individuals may typically display adhesion of 30 ± 4 erythrocytes per mm², whereas more commonly a patient with CRVO may display 190 ± 8 erythrocytes per mm². A subject for treatment in accordance with the present invention may optionally be categorised as having erythrocytes that display greater than 35, such as about 40, about 45, about 50, about 55, about 60, about 65, about 70, about 75, about 80, about 85, about 90, about 95, about 100, about 105, about 110, about 115, about 120, about 125, about 130, about 135, about 140, about 145, about 150, about 155, about 160, about 165, about 170, about 175, about 180, about 185, about 190, about 195, about 200, or more erythrocytes per mm² when assessed for adhesion to endothelial cells under flow conditions, for example when determined by a technique as described above. The term "about" in this context refers to a value that is ± 5, 4, 3, 2, or 1 erythrocytes per mm² of the stated value.

Under such flow conditions, for example, when adhered at 0.03 Pa and analysed for wash out at 0.3 Pa, heathy individuals may typically display adhesion of less than 1 erythrocyte per mm², whereas more commonly a patient with CRVO may display 20 ± 8 erythrocytes per mm². A subject for treatment in accordance with the present invention may optionally be categorised as having erythrocytes that display greater than 1, such as about 2, about 3, about 4 about 5, about 6, about 7, about 8, about 9, about 10, about 11, about 12, about 13, about 14, about 15, about 16, about 17, about 18, about 19, about 20, about 21, about 22, about 23, about 24, about 25, about 26, about 27, about 28, about 29, about 30, more erythrocytes per mm² when assessed for adhesion to endothelial cells under such flow conditions, for example when determined by a technique as described above. The term "about" in this context refers to a value that is ± 50%, 40%, 30%, 20%, 10% or less erythrocytes per mm² of the stated value.

There are three veins in the retina that are usually associated with retinal vein occlusion, which are the central retinal vein, the hemi-central retinal vein or the branch retinal vein ("Retinal Vein Occlusion (RVO) Guidelines", The Royal College of Ophthalmologists, July 2015).

The central vein, the hemi-central vein and the branch central vein provide the names of subtypes of retinal vein occlusion, which are central retinal vein occlusion (CRVO), hemi-central retinal vein occlusion (HRVO), and branch retinal vein occlusion (BRVO).

Central retinal vein occlusion is caused by an obstruction to the central retinal vein, in particular where it passes through the lamina cribosa. Hemi-central retinal vein occlusion is caused by retinal vein obstructions in the superior or interior retinal hemispheres. Branch retinal vein occlusion is caused by an obstruction at the arteriovenous crossing, where the retinal vein and retinal artery share a common vascular sheath.

Accordingly, in one embodiment the retinal vein in which the RVO presents, and/or to which the present invention is directed for prophylaxis or treatment, is one or more selected from the following: the central vein; the hemi-central vein; and the branch central vein. To put in another way, in one embodiment the retinal vein occlusion is one or more of the following: central retinal vein occlusion, hemi-central retinal vein occlusion, and branch retinal vein occlusion.

Preferably, the retinal vein occlusion is central retinal vein occlusion.

Optionally, the treatment of the retinal vein occlusion (RVO) or macular oedema in a subject with RVO, in accordance with the various aspects of the present invention, is started within 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the onset of symptoms of the retinal vein occlusion. More preferably, the treatment of the retinal vein occlusion or macular oedema in a subject with RVO, in accordance with the present invention, is started within 1, 2, 3, 4, 5, 6, 7, 8, 9, 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6,or 7 days of the diagnosis of the retinal vein occlusion. For example, the combination therapy treatment may be started by a first administration of the Annexin A5 component of the co-therapy, or by a first administration of the anti-VEGF agent component of the co-therapy, or by a simultaneous first administration of both components either as separate compositions or combined in the same formulation.

The retinal vein occlusion can also be classified as either ischemic or non-ischemic retinal vein occlusion. This is classification generally relates to the area of capillary non-perfusion, as discussed herein. Accordingly, in one embodiment the retinal vein occlusion is ischemic retinal vein occlusion (such as ischemic central retinal vein occlusion) or non-ischemic retinal vein occlusion (such as non-ischemic central retinal vein occlusion).

The retinal vein occlusion can further be classified as acute retinal vein occlusion. In acute retinal vein occlusion, the RVO can develop due to a severe grade of ischemia within the retinal vein, and the onset of symptoms is quick. It is therefore necessary to provide treatment as soon as possible after the onset, and/or diagnosis, of acute retinal vein occlusion. Accordingly, in one embodiment the retinal vein occlusion is acute retinal vein occlusion, such as acute central retinal vein occlusion. Preferably, the treatment of the acute retinal vein occlusion, in accordance with the present invention, is started within 10, 20, 30 40 50 or 60 minutes, within 1, 2, 3, 4, 5, 6, 7, 8, 9, 10, 11, 12, 13, 14, 15, 16, 17, 18, 19, 20, 21, 22, 23 or 24 hours, or within 1, 2, 3, 4, 5, 6 or 7 days of the onset of symptoms of acute retinal vein occlusion. More preferably, the treatment of the acute retinal vein occlusion, in accordance with the present invention, is started within 1, 2, 3, 4, 5, 6, 7, 8, 9, 10, 20, 30 40 50 or 60 minutes, or within 1, 2, 3, 4, 5 or 6 hours, of the diagnosis of acute retinal vein occlusion. For example, the combination therapy treatment may be started by a first administration of the Annexin A5 component of the co-therapy, or by a first administration of the anti-VEGF agent component of the co-therapy, or by a simultaneous first administration of both components either as separate compositions or combined in the same formulation.

Retinal vein occlusion can affect either one eye (or one retina), referred to as unilateral retinal vein occlusion, or both eyes (or both retinas), referred to as bilateral retinal vein occlusion. Accordingly, in one embodiment the retinal vein occlusion is unilateral retinal vein occlusion (such as unilateral central retinal vein occlusion). In another embodiment the retinal vein occlusion is bilateral retinal vein occlusion (such as bilateral central retinal vein occlusion).

### Diagnosis and characterisation of retinal vein occlusion

It is known to one skilled in medicine how to diagnose retinal vein occlusion, as well as the subtypes of central retinal vein occlusion, hemi-central retinal vein occlusion, and branch retinal vein occlusion. Examples of such diagnoses are discussed in "Retinal Vein Occlusion (RVO) Guidelines" (*supra*)*.* Generally, a patient with retinal vein occlusion will present to the physician with changes in vision, such as blurred vision, loss of a portion of the field of vision, floating spots in the field of vision, flashing lights in the field of vision, reduced or decreased vision *(i.e.* reduced or decreased visual acuity), and a loss of vision *(i.e.* blindness). In some situations, the loss of vision is sudden. Additionally, in some situations the patient does not exhibit any pain.

Some of the characteristics of retinal vein occlusions, which can be used for diagnosis, are as follows: a disc oedema *(i.e.* swelling of the optic disc); increased dilatation of the retinal vein(s); increased tortuosity of the retinal vein(s); bleeding on the eye; a macula oedema; haemorrhages; "cotton wool spots" (these are white patches on the retina, which can be observed in a fundoscopic examination of the retina); a retinal oedema; electrodiagnostic test (ERG) results; afferent pupillary defect; capillary non-perfusion; arteriolar narrowing; vein obstructions; and, retinal pigment epithelial changes. These characteristics can generally be observed by a physician by an examination of the fundus (the interior surface of the eye, opposite the lens) or the retina, using ophthalmoscopy and/or fundus photography. Another diagnostic method used, in particular to identify vein obstructions, is an angiography (such as an angiography with a fluorescent dye (for example, fluorescein)). In the case of the fluorescent dye angiography the patient is injected with the dye, and the behaviour of the dye in the veins of the eyes can be used to identify if there is an obstruction in the veins.

In one embodiment, the subject has been diagnosed as having retinal vein occlusion; such as central retinal vein occlusion, hemi-central retinal vein occlusion, and branch retinal vein occlusion; preferably, central retinal vein occlusion. In an alternative embodiment, the subject is suspected as having retinal vein occlusion; such as central retinal vein occlusion, hemi-central retinal vein occlusion, and branch retinal vein occlusion; preferably, central retinal vein occlusion.

In one embodiment, the subject has one or more symptom is retinal vein occlusion; such as central retinal vein occlusion, hemi-central retinal vein occlusion, and branch retinal vein occlusion; preferably, central retinal vein occlusion.

In one embodiment, the retinal vein occlusion is characterised by a change in the subject's vision, such as one or more of the following: blurred vision, loss of a portion of the field of vision, floating spots in the field of vision, flashing lights in the field of vision, reduced or decreased vision, and a loss of vision. Typically, the retinal vein occlusion is characterised by a reduction or loss of vision, most typically a sudden reduction or loss of vision.

In another embodiment, the retinal vein occlusion is characterised by a notable change in the subject's vision that occurs in seven days or less, such as: six days or less; five days or less; four days or less; three days or less; two days or less; or a day or less. This may optionally be characteristic of acute RVO.

Otherwise, typically, a loss of vision, or a reduction or decrease in vision, occurs for a period of about seven days or more, such as: about eight days or more; about nine days or more; about ten days or more; about 11 days or more; about 12 days or more; about 13 days or more; about 14 days or more; about 15 days or more; about 16 days or more; about 17 days or more; about 18 days or more; about 19 days or more; about 20 days or more; about 21 days or more; about 22 days or more; about 23 days or more; about 24 days or more; about 25 days or more; about 26 days or more; about 27 days or more; about 28 days or more; about 29 days or more; about 30 days or more; about 31 days or more; about one month or more; about two months or more; about three months or more; about four months or more; about five months or more; about six months or more; about seven months or more; about eight months or more; about nine months or more; about ten months or more; about 11 months or more; about 12 months or more; about 13 months or more; about 14 months or more; about 15 months or more; about 16 months or more; about 17 months or more; about 18 months or more; about 19 months or more; about 20 months or more; about 21 months or more; about 22 months or more; about 23 months or more; about 24 months or more; about 36 months or more; about three years or more; about four years or more; about five years or more; about six years or more; about seven years or more; about eight years or more; about nine years or more; or about ten years or more.

During the immediately aforementioned periods, vision can reduce or decrease until there is a loss of vision *(i.e.* blindness) or until the vision is at a percentage of normal vision, at which time that percentage of vision is maintained. Typically, the percentage reduction or decrease in vision is about 95% or less than normal vision, such as: about 90% or less; about 85% or less; about 80% or less; about 75% or less; about 70% or less; about 69% or less; about 68% or less; about 67% or less; about 66% or less; about 65% or less; about 64% or less; about 63% or less; about 62% or less; about 61% or less; about 60% or less; about 59% or less; about 58% or less; about 57% or less; about 56% or less; about 55% or less; about 54% or less; about 53% or less; about 52% or less; about 51% or less; about 50% or less, about 49% or less; about 48% or less; about 47% or less; about 46% or less; about 45% or less; about 44% or less; about 43% or less; about 42% or less; about 41% or less; about 40% or less; about 39% or less; about 38% or less; about 37% or less; about 36% or less; about 35% or less; about 34% or less; about 33% or less; about 32% or less; about 31% or less; about 30% or less; about 25% or less; about 20% or less; about 15% or less; about 10% or less; or about 5% or less than normal vision. We include that normal vision is the vision of the subject prior to the onset of retinal vein occlusion or the normal vision or the general population. If the retinal vein occlusion is unilateral retinal vein occlusion, the normal vision can be in the eye not affected by the retinal vein occlusion.

In one embodiment, the subject does not exhibit any pain in the eye, and/or does not exhibit any pain in the retina.

In one embodiment, the retinal vein occlusion is characterised by one or more of the following: a disc oedema; increased dilatation of the retinal vein(s); increased tortuosity of the retinal vein(s); haemorrhages; "cotton wool spots"; a retinal oedema; electrodiagnostic tests (ERG); afferent pupillary defect; capillary non-perfusion; arteriolar narrowing; and, retinal pigment epithelial changes.

In one embodiment, the retinal vein occlusion (such as central retinal vein occlusion, preferably ischemic central retinal vein occlusion) is characterised by one or more of the following:
- poor visual acuity (for example, about 95% or less than normal vision, such as: about 90% or less; about 85% or less; about 80% or less; about 75% or less; about 70% or less; about 69% or less; about 68% or less; about 67% or less; about 66% or less; about 65% or less; about 64% or less; about 63% or less; about 62% or less; about 61% or less; about 60% or less; about 59% or less; about 58% or less; about 57% or less; about 56% or less; about 55% or less; about 54% or less; about 53% or less; about 52% or less; about 51% or less; about 50% or less, about 49% or less; about 48% or less; about 47% or less; about 46% or less; about 45% or less; about 44% or less; about 43% or less; about 42% or less; about 41% or less; about 40% or less; about 39% or less; about 38% or less; about 37% or less; about 36% or less; about 35% or less; about 34% or less; about 33% or less; about 32% or less; about 31% or less; about 30% or less; about 25% or less; about 20% or less; about 15% or less; about 10% or less; or about 5% or less than normal vision - preferably, about 44% or less than normal vision);
- afferent pupillary defect;
- presence of one or more retinal haemorrhages, such as one or more intra-retinal haemorrhages (for example: two or more; three or more; four or more; five or more; six or more; seven or more; eight or more; nine or more; ten or more; 11 or more; 12 or more; 13 or more; 14 or more; 15 or more; 16 or more; 17 or more; 18 or more; 19; or more; 20 or more retinal haemorrhages);
- presence or one or more "cotton wool spots" (for example: two or more; three or more; four or more; five or more; six or more; seven or more; eight or more; nine or more; ten or more; 11 or more; 12 or more; 13 or more; 14 or more; 15 or more; 16 or more; 17 or more; 18 or more; 19; or more; 20 or more "cotton wool spots");
- retinal vein dilatation;
- retinal vein tortuosity;
- an angiography showing one or more areas of retinal capillary non-perfusion (for example: two or more; three or more; four or more; five or more; six or more; seven or more; eight or more; nine or more; ten or more; 11 or more; 12 or more; 13 or more; 14 or more; 15 or more; 16 or more; 17 or more; 18 or more; 19; or more; 20 or more areas of retinal capillary non-perfusion - preferably 10 or more areas of retinal capillary non-perfusion)
- a electrodiagnostic test (ERG), which presents one or more of: a reduced b-wave amplitude, a reduced b-wave:a-wave ratio; and a prolonged b-wave implicit time; or a reduced b-wave:a-wave ratio and a prolonged b-wave implicit time.

### Retinal vein occlusion patient subgroups

In addition to the aforementioned patients having different subtypes of retinal vein occlusion (for example, central retinal vein occlusion), retinal vein occlusion patients can also be identified by different characteristics.

It is known that about 1% to about 2% of erythrocytes in patients with retinal vein occlusion are Annexin A5 positive *(i.e.* have monomeric Annexin A5 binding sites, such as phosphatidylserine on the outside of the erythrocyte membrane), whilst typically in a "normal" patient (such as one without retinal vein occlusion) about 0.7% of erythrocytes are Annexin A5 positive. By point of comparison, in a Sickle Cell Disease patient, typically about 2.84% of erythrocytes are Annexin A5 positive. Similarly, it is known that there is typically a mean number of monomeric Annexin A5 binding sites of about 400 to about 1000 on each Annexin A5 positive erythrocyte in patients with retinal vein occlusion, whilst in a "normal" patient there is typically a mean number of monomeric Annexin A5 binding sites of about 280 monomeric on each Annexin A5 positive erythrocyte. By point of comparison, in a Sickle Cell Disease patient typically there is a mean number of monomeric Annexin A5 binding sites of about 12,340 monomeric on each Annexin A5 positive erythrocyte.

There can also be distinct differences between subjects in terms of the mean number of monomeric Annexin A5 binding sites exposed on the surface of vascular endothelial cells.

Microparticles are submicron vesicles shed from various cell types (including, for example erythrocytes and/or vascular endothelial cells) and released into the extracellular environment (Hargett and Bauer, 2013, Pulm. Circ., 3(2): 329-340). They can typically be detected in the circulating plasma of a subject. The mean number of monomeric Annexin A5 binding sites exposed on the surface of microparticles can also vary between subjects, for example, based on the binding site levels in the subject's erythrocytes and/or vascular endothelial cells that are responsible for microparticle release.

Accordingly, in one embodiment, the subject is characterised as having about 1% (or 1.0%) to about 2% of cells (for example, erythrocytes *(i.e.* red blood cells) and/or vascular endothelial cells) and/or microparticles that are Annexin A5 positive (for example, about 1.1% to about 2%, about 1.2% to about 2%, about 1.3% to about 2%, about 1.4% to about 2%, about 1.5% to about 2%, about 1.6% to about 2%, about 1.7% to about 2%, about 1.8% to about 2%, or about 1.9% to about 2% of cells (for example, erythrocytes and/or vascular endothelial cells) and/or microparticles that are Annexin A5 positive. It may be preferred that the subject is characterised as having about 1.7% to about 1.9%; of cells (for example, erythrocytes and/or vascular endothelial cells) and/or microparticles that are Annexin A5 positive.

Accordingly, the subject may be characterised in that about 1%, about 1.1%, about 1.2%, about 1.3%, about 1.4%, about 1.5%, about 1.6%, about 1.7%, about 1.8%, or about 1.9%, or about 2% of cells (for example, erythrocytes and/or vascular endothelial cells) and/or microparticles that are Annexin A5 positive. It may be preferred that about 1.8% of cells (erythrocytes and/or vascular endothelial cells) and/or microparticles are Annexin A5 positive.

Additionally or alternatively, the subject may be characterised as having a mean number of monomeric Annexin A5 binding sites of about 400 to about 1000 *per* cell (for example, erythrocyte and/or vascular endothelial cell) and/or microparticle.

For example, the subject may be characterised as having a mean number of monomeric Annexin A5 binding sites of about 410 to about 1000, about 420 to about 1000, about 430 to about 1000, about 440 to about 1000, about 450 to about 1000, about 460 to about 1000, about 470 to about 1000, about 480 to about 1000, about 490 to about 1000, about 500 to about 1000, about 510 to about 1000, about 520 to about 1000, about 530 to about 1000, about 540 to about 1000, about 550 to about 1000, about 560 to about 1000, about 570 to about 1000, about 580 to about 1000, about 590 to about 1000, about 600 to about 1000, about 610 to about 1000, about 620 to about 1000, about 630 to about 1000, about 640 to about 1000, about 650 to about 1000, about 660 to about 1000, about 670 to about 1000, about 680 to about 1000, about 690 to about 1000, about 700 to about 1000, about 710 to about 1000, about 720 to about 1000, about 730 to about 1000, about 740 to about 1000, about 750 to about 1000, about 760 to about 1000, about 770 to about 1000, about 780 to about 1000, about 790 to about 1000, about 800 to about 1000, about 810 to about 1000, about 820 to about 1000, about 830 to about 1000, about 840 to about 1000, about 850 to about 1000, about 860 to about 1000, about 870 to about 1000, about 890 to about 1000, about 900 to about 1000, about 910 to about 1000, about 920 to about 1000, about 930 to about 1000, about 940 to about 1000, about 950 to about 1000, about 960 to about 1000, about 970 to about 1000, about 980 to about 1000, about 990 to about 1000 *per* cell (for example, erythrocyte and/or vascular endothelial cell) and/or microparticle.

It may be preferred that the subject is characterised as having a mean number of monomeric Annexin A5 binding sites of about 700 to about 800 or about 700 to about 740, most preferably about 710 to about 730 *per* cell (for example, erythrocyte and/or vascular endothelial cell) and/or microparticle.

To put in another way, the subject may be characterised as having a mean number of monomeric Annexin A5 binding sites of: about 410, about 420, about 430, about 440, about 450, about 460, about 470, about 480, about 490, about 500, about 510, about 520, about 530, about 540, about 550, about 560, about 570, about 580, about 590, about 600, about 610, about 620, about 630, about 640, about 650, about 660, about 670, about 680, about 690, about 700, about 710, about 720, about 730, about 740, about 750, about 760, about 770, about 780, about 790, about 800, about 810, about 820, about 830, about 840, about 850, about 860, about 870, about 890, about 900, about 910, about 920, about 930, about 940, about 950, about 960, about 970, about 980, about 990 *per* cell (for example, erythrocyte and/or vascular endothelial cell) and/or microparticle - preferably about 720 monomeric Annexin A5 binding sites *per* cell (for example, erythrocyte and/or vascular endothelial cell) and/or microparticle.

It is included that the binding sites on the cell (for example, erythrocyte and/or vascular endothelial cell) and/or microparticle are on the outside of the cell's or microparticle's surface membrane *(i.e.* the external membrane). Preferably, in the embodiment outlined immediately above, the monomeric Annexin A5 binding sites *per* cell (for example, erythrocyte and/or vascular endothelial cell) and/or microparticle are on cells and/or microparticles that are Annexin A5 positive.

It is known to one skilled in the art of molecular biology how to identify the number of Annexin A5 positive cells and/or microparticles in a patient, for example by determining the number of Annexin A5 positive erythrocytes and/or microparticles in a patient's blood. Various methods are available that allow for cells and/or microparticles to be counted, based on a marker on surface of the cell and/or microparticle (such as flow cytometry). See, for example, Hargett and Bauer, 2013, Pulm. Circ., 3(2): 329-340. There are also numerous variants of Annexin A5 that are conjugated to fluorescent markers which allow for such an analysis to be undertaken. A simple example of a possible method, as would be known to the skilled person, is to obtain a sample of the subject's blood, label it with a fluorescent Annexin A5, and calculate the number of Annexin A5 positive cells and/or microparticles using a flow cytometer. Accordingly, by "Annexin A5 positive" we include a cell (such as an erythrocyte) which can be bound by Annexin A5 (in particular, monomeric Annexin A5).

In one embodiment, a cell (for example, erythrocyte and/or vascular endothelial cell) and/or a microparticle is considered to be Annexin A5 positive if it has 290 or more monomeric Annexin A5 binding sites, such as: 300 or more; 310 or more; 320 or more; 330 or more; 340 or more; 350 or more; 360 or more; 370 or more; 380 or more; 390 or more; or 400 or more Annexin A5 binding sites.

However, the mean number of monomeric Annexin A5 binding sites determine per cell and/or a microparticle, for the same sample, may vary dependent on the specific method of determination used. Therefore, in another embodiment, a cell (for example, erythrocyte and/or vascular endothelial cell) and/or a microparticle is considered to be Annexin A5 positive if it has more than 100% of the mean number of Annexin A5 binding sites of a corresponding cell (such as an erythrocyte and/or vascular endothelial cell) and/or a microparticle in a healthy subject. For example, the mean number of monomeric Annexin A5 binding sites *per* erythrocyte characteristic of RVO may be in the region of about 1.4-fold to about 4-fold greater than the mean number of monomeric Annexin A5 binding sites *per* erythrocyte in a healthy human (such as about 2-fold to about 3-fold greater).

One of the molecules that can be bound by Annexin A5 is phosphatidylserine. A monomeric Annexin A5 binding site is about 30 to about 60 phosphatidylserine molecules, and a monomeric Annexin A5 binding site is an about 3,100 Ångström (Å) to about 3,300 Å area on the surface membrane of a cell and/or a microparticle. In addition, a monomeric Annexin A5 binding site is an area of about 40 to about 60 phospholipid heads on the surface membrane of a cell and/or a microparticle.

In one embodiment, the term "Annexin A5 binding site" refers to an area of about 30 to about 60 phosphatidylserine molecules on the surface membrane of a cell (for example, an erythrocyte and/or a vascular endothelial cell) and/or a microparticle, such as: about 31 to about 59; about 32 to about 58; about 33 to about 57; about 34 to about 56; about 35 to about 55; about 36 to about 54; about 37 to about 53; about 38 to about 52; about 39 to about 51; about 40 to about 50; about 41 to about 49; about 42 to about 48; about 43 to about 47; or about 44 to about 46 phosphatidylserine molecules on the surface membrane of a cell (for example, an erythrocyte and/or a vascular endothelial cell) and/or a microparticle, preferably about 35 to about 45 phosphatidylserine molecules on the surface membrane of a cell (for example, an erythrocyte and/or a vascular endothelial cell) and/or a microparticle, more preferably about 42 to about 57 phosphatidylserine molecules on the surface membrane of a cell (for example, an erythrocyte and/or a vascular endothelial cell) and/or a microparticle, most preferably about 40 phosphatidylserine molecules on the surface membrane of a cell (for example, an erythrocyte and/or a vascular endothelial cell) and/or a microparticle.

In an and additional or alternative embodiment, the term "Annexin A5 binding site" refers to a site that is an about 3,100 Å to about 3,300 Å area on the surface membrane of a cell (for example, an erythrocyte and/or a vascular endothelial cell) and/or a microparticle, such as: about 3,110 Å to about 3,290 Å; about 3,120 Å to about 3,280 Å, about 3,130 Å to about 3,270 Å, about 3,140 Å to about 3,260 Å; about 3,150 Å to about 3,250 Å; about 3,160 Å to about 3,240 Å; about 3,170 Å to about 3,230 Å; about 3,180 Å to about 3,220 Å; about 3,190 Å to about 3,210 Å area on the surface membrane of a cell (for example, an erythrocyte and/or a vascular endothelial cell) and/or a microparticle, preferably about 3,150 Å to about 3,250 Å area on the surface membrane of a cell (for example, an erythrocyte and/or a vascular endothelial cell) and/or a microparticle, more preferably about 3,000 Å area on the surface membrane of a cell (for example, an erythrocyte and/or a vascular endothelial cell) and/or a microparticle.

In a further embodiment, the term "Annexin A5 binding site" refers to an area of about 40 to about 60 phospholipid heads on the area on the surface membrane of a cell (for example, an erythrocyte and/or a vascular endothelial cell) and/or a microparticle, such as: about 41 to about 59; about 42 to about 58; about 43 to about 57; about 44 to about 56; about 45 to about 55; about 46 to about 54; about 47 to about 53; about 48 to about 52; about 49 to about 51 phospholipid heads on the area on the surface membrane of a cell (for example, an erythrocyte and/or a vascular endothelial cell) and/or a microparticle, preferably about 45 to about 55 phospholipid heads on the area on the surface membrane of a cell (for example, an erythrocyte and/or a vascular endothelial cell) and/or a microparticle, more preferably about 50 phospholipid heads on the area on the surface membrane of a cell (for example, an erythrocyte and/or a vascular endothelial cell) and/or a microparticle.

In one embodiment of any of the first, second, third or fourth aspects of the present invention, the subject is characterised by one or more of the following:
a. the subject is aged about 50 years or more, such as at least 55 years, 60 years, 65 years, 70 years, 75 years, or 80 years (optionally 90 years or less, such as, about 89 years or less; about 88 years or less; about 87 years or less; about 86 years or less; about 85 years or less; about 84 years or less; about 83 years or less; about 82 years or less; about 81 years or less; about 80 years or less; about 79 years or less; about 78 years or less; about 77 years or less; about 76 years or less; about 75 years or less; about 74 years or less; about 73 years or less; about 72 years or less; about 71 years or less; about 70 years or less; about 69 years or less; about 68 years or less; about 67 years or less; about 66 years or less; about 65 years or less; about 64 years or less; about 63 years or less; about 62 years or less; about 61 years or less; about 60 years or less; about 59 years or less; about 58 years or less; about 57 years or less; about 56 years or less; about 55 years or less; about 54 years or less; about 53 years or less; about 52 years or less; about 51 years or less);
b. the subject does not have, or is not suspected as having, a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus);
c. the subject does not have a medical history of having a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sarcoidoisis, Wegener's Granulomatosis and/or Goodpasture's Syndrome), although preferably excluding diabetes and diabetes mellitus);
d. the subject does not have a familial history of having a cardiovascular disease (such as a myocardial infarction, a stroke, hypertension, hyperlipidaemia, hyperhomocysteinemia, a blood coagulation disease, and/or peripheral artery disease, and optionally excluding atherosclerosis) or a disease associated with a cardiovascular disease (such as sickle cell disease, glaucoma, diabetes, diabetes mellitus, polycythemia vera, and/or systematic inflammatory disorders (for example, Behçets disease, polyarteritis nodosa, sacoidoisis, Wegener's Granulomatosis and/or Goodpasture's Syndrome), although preferably excluding diabetes and diabetes mellitus); and/or
e. the subject does not have, or is not suspected as having, an oncological condition, such as a solid tumour, a lymphoma and/or a blood cancer, for example one or more condition selected from breast cancer, endometrial cancer, cervix cancer, ovary cancer, lung cancer, head and/or neck cancer, brain cancer, thyroid cancer, oesophagus cancer, stomach cancer, colon and/or rectal cancer, liver cancer, pancreatic cancer, skin cancer, kidney cancer, bladder cancer, prostate cancer, testis cancer, bone cancer, lymphoma (such as Hodgkin lymphoma and/or non-Hodgkin lymphoma) and/or blood cancer (such as acute lymphoblastic leukemia, acute myeloid leukemia and/or chronic myeloid leukemia).

The "familial history" of a subject can be readily determined by one skilled in medicine. In one embodiment, the familial history includes the history of one or more of the following relatives: a parent (such as a mother or father); a grandparent (such as a grandmother or grandfather); a sibling (such as a brother or sister); a child (such as a son or daughter); and/or a grandchild (such as a grandson or granddaughter).

In one embodiment, the subject does not have or is not suspected as having a cardiovascular disease or a disease associated with a cardiovascular disease if the subject does not display one or more symptoms of those diseases. The symptoms of those diseases are known to one skilled in medicine.

In one embodiment, the blood coagulation disorder is not retinal vein occlusion, such as central retinal vein occlusion, branch retinal vein occlusion, or hemi-retinal vein occlusion.

Preferably, the subject is characterised as:
a. being about 50 years or more;
b. the subject does not have, or is not suspected as having one or more conditions selected from hypertension, diabetes, and/or sickle cell disease;
c. the subject does not have a medical history of having one or more conditions selected from hypertension, diabetes, and/or sickle cell disease; and
d. the subject does not have a familial history of having one or more conditions selected from hypertension, diabetes, and/or sickle cell disease.

In an alternative embodiment, the subject may be (i) aged 50 years or more and/or (ii) be suspected as having, have a medical history of, and/or have a familiar history of one or more conditions selected from hypertension, diabetes, and/or sickle cell disease.

In one embodiment, the subject does not have or is not suspected of having a condition that is characterised as having increased extracellular Annexin A5. Preferably, the increased extracellular Annexin A5 is increased when compared to a patient without the condition that is characterised as having increased extracellular Annexin A5. In one embodiment, the condition that is characterised as having increased extracellular Annexin A5 is not retinal vein occlusion and/or a retinal vein occlusion associated disease.

It has been demonstrated that a subset of retinal vein occlusion patients might be genetically disposed to (that is, carry a genetic risk factor for) forming the retinal vein occlusions. For example, genetically disposed patients may generate red blood cells that preferentially form thrombi in the veins of the eye - in particular, the retina. The formation of retinal vein occlusions in these genetically disposed patients is less likely to associated with other cardiovascular diseases.

One skilled in the art of medicine would know how to identify a patient as being genetically disposed to forming a particular disease, such as retinal vein occlusion.

Accordingly, in one embodiment the subject is genetically disposed to retinal vein occlusion, or suspected of being genetically disposed to retinal vein occlusion. Preferably, the subject is genetically disposed to the formation of a thrombus (or thrombi) in the veins of the eye (in particular, the retina). Most preferably, the subject is genetically disposed to produce red blood cells that form a thrombus (or thrombi) preferentially in the veins of the eye (in particular, the retina). For example it is known from a study of *in vitro* bone marrow culture in CRVO patients, that about 27% of the CRVO patients exhibited spontaneous growth of erythroid precursors in the absence of any detectable myeloproliferative disorder (Heron et al, 2007, Ophthalmology, 114: 2155-61). Accordingly, in one embodiment the subject may be a subject that displays spontaneous *in vitro* growth of erythroid precursors from bone marrow, in the absence of any detectable myeloproliferative disorder. Typically, the subject is a human subject.

In one embodiment, the subject (in particular, a human subject) has a body weight of about 10 kg to about 110 kg, such as: about 20 kg to about 100 kg; about 30 kg to about 90 kg; about 40 kg to about 80 kg; or about 50 kg to about 70 kg. In a preferred embodiment, the subject has a body weight of about 40 kg to about 80 kg. In another preferred embodiment, the subject has a body weight of about 50 kg to about 70 kg. In a more preferred embodiment, the subject has a body weight of about 60 kg. As used in this context, the term "about" may be used herein to mean a range of ±50%, ±40%, ±30%, ±20%, ±10%, ±5%, ±4%, ±3%, ±2% or ±1% of the value mentioned.

Diseases, disorders or conditions associated with, and/or caused by, retinal vein occlusion

Various diseases, disorders or conditions are associated with, and/or caused by, RVO, and can also be treated by the current invention, as further defined by the present claims.

Accordingly, in one embodiment, the invention provides for the prophylaxis or treatment of one or more diseases, disorders or conditions are associated with, and/or caused by, RVO, as further defined by the present claims.

For example, a condition associated with RVO may be macular oedema. When retinal veins are blocked during retinal vein occlusion, blood does not drain properly and it can leak into the retina. If it leaks into the macula, this can produce macular oedema (MO). Leakage is worsened by the severity of the blockage, how many veins are involved, and the pressure inside them.

Macular oedema (MO) is the build-up of fluid in the macula, an area in the centre of the retina. The retina is the light-sensitive tissue at the back of the eye and the macula is the part of the retina responsible for sharp, straight-ahead vision. Fluid build up causes the macula to swell and thicken, which distorts vision. It typically occurs when there is leakage of fluid and blood into the retina, due to increased vein and/or capillary pressure causing vessel permeability. The reduction, decrease or loss of vision in retinal vein occlusion is often associated with macular oedema.

Accordingly, in one embodiment, the invention provides a molecule comprising, or consisting of, monomeric Annexin A5 protein for use in the prophylaxis or the treatment of macular oedema in a subject, wherein the use is as a combination therapy with an anti-vascular endothelial growth factor (anti-VEGF) agent, and preferably in a subject characterised as having, or being suspected of having, or being at risk of having, RVO. Other diseases, disorders or conditions are associated with, and/or caused by, RVO include retinal ischemia, iris neovascularization, retinal neovascularization, neovascular glaucoma, vitreous haemorrhage, rubeosis, and retinal detachment.

That is to say, in separate embodiments:
the present invention provides a molecule comprising, or consisting of, Annexin A5 protein (such as monomeric Annexin A5 protein) for use in the prophylaxis or the treatment of retinal ischemia in a subject, wherein the use is as a combination therapy with an anti-vascular endothelial growth factor (anti-VEGF) agent, and preferably in a subject characterised as having, or being suspected of having, or being at risk of having, RVO,
the present invention provides a molecule comprising, or consisting of, Annexin A5 protein (such as monomeric Annexin A5 protein) for use in the prophylaxis or the treatment of diabetic iris neovascularization in a subject, wherein the use is as a combination therapy with an anti-vascular endothelial growth factor (anti-VEGF) agent, and preferably in a subject characterised as having, or being suspected of having, or being at risk of having, RVO,
the present invention provides a molecule comprising, or consisting of, Annexin A5 protein (such as monomeric Annexin A5 protein) for use in the prophylaxis or the treatment of retinal neovascularization in a subject, wherein the use is as a combination therapy with an anti-vascular endothelial growth factor (anti-VEGF) agent, and preferably in a subject characterised as having, or being suspected of having, or being at risk of having, RVO,
the present invention provides a molecule comprising, or consisting of, Annexin A5 protein (such as monomeric Annexin A5 protein) for use in the prophylaxis or the treatment of neovascular glaucoma in a subject, wherein the use is as a combination therapy with an anti-vascular endothelial growth factor (anti-VEGF) agent, and preferably in a subject characterised as having, or being suspected of having, or being at risk of having, RVO,
the present invention provides a molecule comprising, or consisting of, Annexin A5 protein (such as monomeric Annexin A5 protein) for use in the prophylaxis or the treatment of vitreous haemorrhage in a subject, wherein the use is as a combination therapy with an anti-vascular endothelial growth factor (anti-VEGF) agent, and preferably in a subject characterised as having, or being suspected of having, or being at risk of having, RVO,
the present invention provides a molecule comprising, or consisting of, Annexin A5 protein (such as monomeric Annexin A5 protein) for use in the prophylaxis or the treatment of rubeosis in a subject, wherein the use is as a combination therapy with an anti-vascular endothelial growth factor (anti-VEGF) agent, and preferably in a subject characterised as having, or being suspected of having, or being at risk of having, RVO, and
the present invention provides a molecule comprising, or consisting of, Annexin A5 protein (such as monomeric Annexin A5 protein) for use in the prophylaxis or the treatment of retinal detachment in a subject, wherein the use is as a combination therapy with an anti-vascular endothelial growth factor (anti-VEGF) agent, and preferably in a subject characterised as having, or being suspected of having, or being at risk of having, RVO.

Retinal ischemia is ischemia due to the non-perfusion of retinal veins and capillary caused by retinal vein occlusion.

Iris neovascularization and retinal neovascularization is the growth of new blood vessels in the eye. These neovascularizations cause an increase of cytokines (such as VEGF), which acts in a positive feedback loop to further promote new growth of blood vessels.

One or more of retinal ischemia, iris neovascularization and retinal neovascularization can lead to neovascular glaucoma *(i.e.* damage to the optical nerve), vitreous haemorrhage *(i.e.* leaking of blood into the vitreous humor of the eye), rubeosis *(i.e.* new blood vessels forming on the iris), and/or retinal detachment *(i.e.* the retina detaching from its blood supply) ("Retinal Vein Occlusion (RVO) Guidelines" *supra).*

Accordingly, the present invention also provides for the prophylaxis or treatment of one or more diseases, disorders or conditions selected from: retinal ischemia, iris neovascularization, retinal neovascularization, neovascular glaucoma, vitreous haemorrhage, rubeosis, and retinal detachment, preferably in a subject characterised as having, or being suspected of having, or being at risk of having, RVO.

In one embodiment, the iris neovascularization and/or retinal neovascularization is characterised by an increase in cytokines, such as increase in VEGF. In one embodiment, retinal vein occlusion is central retinal vein occlusion and the retinal vein occlusion associated disease is iris neovascularization. In some embodiments, retinal vein occlusion is branch retinal vein occlusion and the retinal vein occlusion associated disease is retinal neovascularization. In one embodiment, the retinal vein occlusion is hemi-central vein occlusion and the retinal vein occlusion associated disease is rubeosis. Preferably, the retinal vein occlusion associated disease is macular oedema.

Accordingly, a molecule comprising, or consisting of, Annexin A5 protein (such as monomeric Annexin A5 protein) is provided for use in the prophylaxis or the treatment of a disease, disorder or condition that is associated with, and/or caused by, RVO, as further defined by the present claims, in a subject, wherein the use is as a combination therapy with an anti-vascular endothelial growth factor (anti-VEGF) agent,
optionally wherein the composition comprises the molecule consisting of monomeric Annexin A5 protein in an amount of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of a subject, and/or
optionally wherein the composition comprises the molecule consisting of monomeric Annexin A5 protein in a total amount of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg.

The embodiments of the invention discussed herein with respect to the first, second, third and fourth aspects of the present invention are applicable to the foregoing, unless otherwise stated.

### C. Annexin A5

The molecule comprising an Annexin A5 protein, such as a monomeric Annexin A5 protein, for use in all aspects of the present invention may comprise a protein consisting of the sequence of Annexin A5, such as the sequence of human Annexin A5.

The molecule consisting of monomeric Annexin A5 protein for use in all aspects of the present invention may consist essentially of, or consist of, a protein consisting of the sequence of Annexin A5, such as the sequence of human Annexin A5.

For the avoidance of doubt, the molecule consisting of monomeric Annexin A5 protein is not a dimeric Annexin A5 (commonly referred to in the art as "Diannexin"), and is not a PEGylated Annexin A5.

Preferably the molecule comprising, or consisting of, human Annexin A5 protein (such as human monomeric Annexin A5 protein) is a protein comprising, consisting, or consisting essentially of, a polypeptide molecule consisting of the amino acid sequence of SEQ ID NO: 1, as shown below, either with or without the N-terminal methionine. Preferably the molecule comprising, consisting, or consisting essentially of, Annexin A5 protein (such as monomeric Annexin A5 protein) is a recombinant human Annexin A5 protein (such as a recombinant human monomeric Annexin A5 protein), for example, a protein created by recombinantly expressing a gene encoding a protein having the sequence of SEQ ID NO: 1 in a recombinant host cell organism, such as a recombinant *E. coli* host, preferably followed by the recovery and purification of the Annexin A5 protein therefrom.

The sequence of the protein encoded by the human Annexin A5 gene is defined by SEQ ID NO: 1 as follows -

Met Ala Gln Val Leu Arg Gly Thr Val Thr Asp Phe Pro Gly Phe Asp
1               5                   10                  15

Glu Arg Ala Asp Ala Glu Thr Leu Arg Lys Ala Met Lys Gly Leu Gly
            20                  25                  30

Thr Asp Glu Glu Ser Ile Leu Thr Leu Leu Thr Ser Arg Ser Asn Ala
        35                  40                  45

Gln Arg Gln Glu Ile Ser Ala Ala Phe Lys Thr Leu Phe Gly Arg Asp
    50                  55                  60

Leu Leu Asp Asp Leu Lys Ser Glu Leu Thr Gly Lys Phe Glu Lys Leu
65                  70                  75                  80

Ile Val Ala Leu Met Lys Pro Ser Arg Leu Tyr Asp Ala Tyr Glu Leu
                85                  90                  95

Lys His Ala Leu Lys Gly Ala Gly Thr Asn Glu Lys Val Leu Thr Glu
            100                 105                 110

Ile Ile Ala Ser Arg Thr Pro Glu Glu Leu Arg Ala Ile Lys Gln Val
        115                 120                 125

Tyr Glu Glu Glu Tyr Gly Ser Ser Leu Glu Asp Asp Val Val Gly Asp
    130                 135                 140

Thr Ser Gly Tyr Tyr Gln Arg Met Leu Val Val Leu Leu Gln Ala Asn
145                 150                 155                 160

Arg Asp Pro Asp Ala Gly Ile Asp Glu Ala Gln Val Glu Gln Asp Ala
                165                 170                 175

Gln Ala Leu Phe Gln Ala Gly Glu Leu Lys Trp Gly Thr Asp Glu Glu
            180                 185                 190

Lys Phe Ile Thr Ile Phe Gly Thr Arg Ser Val Ser His Leu Arg Lys
        195                 200                 205

Val Phe Asp Lys Tyr Met Thr Ile Ser Gly Phe Gln Ile Glu Glu Thr
    210                 215                 220

Ile Asp Arg Glu Thr Ser Gly Asn Leu Glu Gln Leu Leu Leu Ala Val
225                 230                 235                 240

Val Lys Ser Ile Arg Ser Ile Pro Ala Tyr Leu Ala Glu Thr Leu Tyr
                245                 250                 255

Tyr Ala Met Lys Gly Ala Gly Thr Asp Asp His Thr Leu Ile Arg Val
            260                 265                 270

Met Val Ser Arg Ser Glu Ile Asp Leu Phe Asn Ile Arg Lys Glu Phe
        275                 280                 285

Arg Lys Asn Phe Ala Thr Ser Leu Tyr Ser Met Ile Lys Gly Asp Thr
    290                 295                 300

Ser Gly Asp Tyr Lys Lys Ala Leu Leu Leu Leu Cys Gly Glu Asp Asp
305                 310                 315                 320

In another embodiment, the Annexin A5 (such as monomeric Annexin A5) protein may comprise, consist essentially of, or consist of, a variant or mutant of the sequence of Annexin A5, such as the sequence of human Annexin A5 (optionally, a protein consisting of the sequence of SEQ ID NO:1, either with or without the N-terminal methionine). For example, the sequence of the variant or mutant may differ from the sequence of SEQ ID NO: 1 (either with, or without, the N-terminal methionine) at any one or more positions, such as at, or up to, 1, 2, 3, 4, 5, 6, 7, 8, 9, 10, 11, 12, 13, 14, 15, 16, 17, 18, 19, 20, 25, 30, 35, 40, 45, 50, 55, 60, 65, 70, 75, 80, 85, 90, 95, 100, 110, 120, 130, 140, 150, 160 or more positions.

In another embodiment, the Annexin A5 (such as monomeric Annexin A5) protein may comprise, consist essentially of, or consist of, a biologically active fragment of mature Annexin A5, such as human Annexin A5 (optionally, a protein consisting of the sequence of SEQ ID NO:1, either with or without the N-terminal methionine) or of a variant or mutant thereof.

Biologically active fragments of Annexin A5 share a functional or binding property with full length Annexin A5. Epitopic fragments of Annexin A5 bind to a monoclonal antibody that binds to full length Annexin A5. "Activity" of Annexin A5 shall mean any binding function performed by that protein, further examples of which are defined further below.

A biologically active fragment of the monomeric Annexin A5 protein, which can be used in the present invention, may comprise at least about 50 contiguous amino acids, usually at least about 100 contiguous amino acids, at least about 150 contiguous amino acids, at least about 200 contiguous amino acids, at least about 250 contiguous amino acids, at least about 260 contiguous amino acids, at least about 270 contiguous amino acids, at least about 280 contiguous amino acids, at least about 290 contiguous amino acids, at least about 300 contiguous amino acids, at least about 310 contiguous amino acids, and which may include up to about 320 contiguous amino acids of an Annexin A5 protein, including without limitation human Annexin A5 protein (optionally, a protein consisting of the sequence of SEQ ID NO:1, either with or without the N-terminal methionine), or modifications thereof, and may further include fusion polypeptides as known in the art in addition to the provided sequences. The Annexin A5 sequence may be from any mammalian or avian species, e.g. primate sp., particularly humans; rodents, including mice, rats and hamsters; rabbits; equines, bovines, canines, felines; etc. Of particular interest is the human Annexin A5 protein (optionally, a protein consisting of the sequence of SEQ ID NO:1, either with or without the N-terminal methionine).

In one embodiment, the biologically active fragment of the Annexin A5 (such as monomeric Annexin A5) protein is effective for the treatment of retinal vein occlusion or a retinal vein occlusion associated disease; preferably, 10% or more effective, such as: 20% or more; 30% or more; 40% or more; 50% or more; 60% or more; 70% or more; 80% or more; 90% or more effective, in comparison to human monomeric Annexin A5 protein that is a polypeptide molecule consisting of the amino acid sequence of SEQ ID NO:1, either with or without the N-terminal methionine.

In one embodiment, the monomeric Annexin A5 protein consists of about 320 or less amino acids, such as: about 319 amino acids or less; about 318 amino acids or less; about 317 amino acids or less; about 316 amino acids or less; about 315 amino acids or less; about 314 amino acids or less; about 313 amino acids or less; about 312 amino acids or less; about 311 amino acids or less; about 310 amino acids or less; about 305 amino acids or less; about 300 amino acids or less; about 295 amino acids or less; about 290 amino acids or less; about 285 amino acids or less; about 280 amino acids or less; about 270 amino acids or less; about 260 amino acids or less; or about 250 amino acids or less.

In one embodiment, the recombinant monomeric Annexin A5 protein consists of 319 amino acids, such as: about 318 amino acids or less; about 317 amino acids or less; about 316 amino acids or less; about 315 amino acids or less; about 314 amino acids or less; about 313 amino acids or less; about 312 amino acids or less; about 311 amino acids or less; about 310 amino acids or less; about 305 amino acids or less; about 300 amino acids or less; about 295 amino acids or less; about 290 amino acids or less; about 285 amino acids or less; about 280 amino acids or less; about 270 amino acids or less; about 260 amino acids or less; or about 250 amino acids or less.

The Annexin A5 (such as monomeric Annexin A5) protein may include one or more of the following:
a. a protein comprising, consisting essentially of, or consisting of the sequence of human monomeric Annexin A5 protein (optionally, a protein consisting of the sequence of SEQ ID NO:1, either with or without the N-terminal methionine);
b. a recombinant human Annexin A5 protein, such as a human monomeric Annexin A5 protein;
c. a mammalian orthologue of human Annexin A5 protein, such as a a mammalian orthologue of human monomeric Annexin A5 protein;
d. an allelic or genetic variant of a), b) or c);
e. a functional analogue or variant of an Annexin A5 protein, such as a monomeric Annexin A5 protein, which is a protein which consists of an amino acid sequence that is more than 50%, 60%, 70%, 75%, such as more than 80%, 85%, more than 90%, or even more preferably more than 95% or 99% identical to human monomeric Annexin A5 protein having the sequence defined by SEQ ID NO:1 either with, or without, the N-terminal methionine; and
f. a biologically active fragment of any of a) to e).

The molecule comprising of Annexin A5 protein may also be referred to herein as an "agent comprising Annexin A5 protein" or "therapeutic agent comprising Annexin A5 protein", and might be referred to as "Annexin A5". Optionally, the term "the molecule comprising Annexin A5 protein" can exclude molecules include forms of Annexin A5 other than monomeric Annexin A5, such as dimeric monomeric Annexin A5 protein (also known as Diannexin).

In some embodiments, the molecule comprising the Annexin A5 protein for use in the present invention may be a conjugate in which the Annexin A5 protein (such as a monomeric Annexin A5 protein) is conjugated to moiety, such as a non-proteinaceous moiety. Without limitation, said moiety may for example be a therapeutic, prophylactic, diagnostic, prognostic, or theragnostic moiety. In some embodiments, the moiety is a drug (such as a non-proteinaceous drug) and/or a radioactive moiety (for example, wherein the conjugate is suitable for use in radioimmunotherapy ("RIT")).

The molecule consisting of monomeric Annexin A5 protein may also be referred to herein as an "agent consisting of monomeric Annexin A5 protein" or "therapeutic agent consisting of monomeric Annexin A5 protein", and might be referred to as "monomeric Annexin A5". The purpose of the term "the molecule consisting of monomeric Annexin A5 protein" is to exclude molecule that are not monomeric Annexin A5, such as PEGylated Annexin A5, dimeric monomeric Annexin A5 protein (also known as Diannexin), and an Annexin A5 fusion protein, in particular Annexin A5 fusion proteins having a higher weight average molecular weight and/or plasma half-life compared to human monomeric Annexin A5 protein that is a polypeptide molecule consisting of the amino acid sequence of SEQ ID NO:1, either with or without the N-terminal methionine.

Accordingly, the molecule consisting of monomeric Annexin A5 protein is not PEGylated Annexin A5, dimeric monomeric Annexin A5 protein, or an Annexin A5 fusion protein. For the avoidance of doubt, in one embodiment the molecule consisting of monomeric Annexin A5 protein is not a "modified annexin" as defined in US 9,463,217.

In particular embodiments, the functional analogue, mutant or variant of Annexin A5 according to the invention is more than 50%, 60%, 70%, 75%, such as more than 80%, 85%, more than 90%, or even more preferably more than 95% or 99% identical to human Annexin A5, SEQ ID NO:1 either with, or without, the N-terminal methionine.

The percent identity between two amino acid sequences is determined as follows. First, an amino acid sequence is compared to, for example, SEQ ID NO:1 using the BLAST 2 Sequences (BI2seq) program from the stand-alone version of BLASTZ containing BLASTN version 2.0.14 and BLASTP version 2.0.14. This stand-alone version of BLASTZ can be obtained from the U.S. government's National Center for Biotechnology Information web site at ncbi.nlm.nih.gov. Instructions explaining how to use the BI2seq program can be found in the readme file accompanying BLASTZ. BI2seq performs a comparison between two amino acid sequences using the BLASTP algorithm. To compare two amino acid sequences, the options of BI2seq are set as follows: -i is set to a file containing the first amino acid sequence to be compared (e.g., C:\seq1.txt); -j is set to a file containing the second amino acid sequence to be compared (e.g., C:\seq2.txt); -p is set to blastp; -o is set to any desired file name (e.g., C:\output.txt); and all other options are left at their default setting. For example, the following command can be used to generate an output file containing a comparison between two amino acid sequences: C:\BI2seq -i c:\seq1.txt -j c:\seq2.txt -p blastp -o c:\output.txt. If the two compared sequences share homology, then the designated output file will present those regions of homology as aligned sequences. If the two compared sequences do not share homology, then the designated output file will not present aligned sequences. Once aligned, the number of matches is determined by counting the number of positions where an identical nucleotide or amino acid residue is presented in both sequences.

The percent identity is determined by dividing the number of matches by the length of the sequence set forth in an identified sequence followed by multiplying the resulting value by 100. For example, if a sequence is compared to the sequence set forth in SEQ ID NO:1 (the length of the sequence set forth in SEQ ID NO:1 is 320) and the number of matches is 288, then the sequence has a percent identity of 90 (*i.e.*, 288 ÷ 320 * 100 = 90) to the sequence set forth in SEQ ID NO:1.

Thus, a functional analogue, mutant or variant of Annexin A5 may be a protein wherein at one or more positions there have been amino acid insertions, deletions, or substitutions, either conservative or non-conservative, provided that such changes result in a protein whose basic properties to function in an equivalent manner to Annexin A5 have not significantly been changed. "Significantly" in this context means that one skilled in the art would say that the properties of the variant may still be different but would not be unobvious over the ones of the original protein.

By "conservative substitutions" is intended combinations such as Gly, Ala; Val, Ile, Leu; Asp, Glu; Asn, GIn; Ser, Thr; Lys, Arg; and Phe, Tyr.

Such variants and mutants may be made using the methods of protein engineering and site-directed mutagenesis which are well known in the art.

A functional analogue, mutant, variant or biologically active fragment of Annexin A5 may, optionally, consist of the sequence of human Annexin A5 (such as SEQ ID NO:1), either with, or without, the N-terminal methionine, with no greater than 50, 40, 30, 20, 10, 5, 4, 3, 2 or 1 consecutive or non-consecutive additional amino acid; and/or no greater than 50, 40, 30, 20, 10, 5, 4, 3, 2 or 1 consecutive or non-consecutive amino acid deletions; and/or no greater than 50, 40, 30, 20, 10, 5, 4, 3, 2 or 1 consecutive or non-consecutive amino acid substitutions.

### D. Subjects for Treatment

The subject to be treated in accordance with the present invention may be a human subject or a non-human subject. Preferably the subject in accordance with any of the aspects of the present invention is characterised as having, or being suspected of having, or being at risk of having, macular oedema and/or RVO, or a condition associated with either or both of macular oedema and/or RVO as further defined by the present claims.

Optionally, although not necessarily, the subject may be treatment naïve, in the sense of not having previously received any form of therapeutic agent for the specific event that is being treated in accordance with the present invention.

In a preferred embodiment, the subject is a human subject.

In another embodiment, the subject is a non-human subject, and may be selected from the group consisting of: a mammalian subject, which is not a human; a bird; an amphibian; and a reptile.

A mammalian subject, which is not a human, may be selected from the list consisting of: an equine (such as: a horse or donkey); a bovine (such as: a cow; a buffalo; a bison; or a yak); a camel; a pig; a llama; an alpaca; a bird (such as: an ostrich; a chicken; a goose; a duck; a turkey; a quail; or a pigeon) a sheep; a goat; a canine; a feline; a reptile (such as: a snake; a lizard; a crocodile; an alligator; a tortoise; or a turtle); a rabbit; an amphibian (such as: a frog; or a salamander) or a rodent (such as: a mouse; a rat; a chinchilla; a guinea pig; or a squirrel).

### E. Administrative Regimes

### Dosages & Timings:

In accordance with all aspects of the present invention, for any given round of administration, the molecule comprising, or consisting of, Annexin A5 protein (such as monomeric Annexin A5 protein) may be administered to the subject:
optionally in a dosage that is determined in respect of the body weight of the subject, at a dose of the molecule of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of a subject, and/or
optionally, independently of the body weight of the subject, at a total dose of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg, as defined above.

### (a) Dose per body weight:

For the avoidance of doubt, it is to be understood that the term "mg/kg body weight" is a reference to mg of the molecule comprising, or consisting of, Annexin A5 protein (such as monomeric Annexin A5 protein), per kg body weight of the subject.

A preferred dose may be selected from one or more of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg, such as about 0.01 µg/kg, about 0.02 µg/kg, about 0.03 µg/kg, about 0.04 µg/kg, about 0.05 µg/kg, about 0.06 µg/kg, about 0.07 µg/kg, about 0.08 µg/kg, about 0.09 µg/kg, and/or about 0.1 µg/kg.

In some embodiments, the dose may be selected from one or more of about 0.1 µg/kg, about 0.2 µg/kg, about 0.3 µg/kg, about 0.4 µg/kg, about 0.5 µg/kg, about 0.6 µg/kg, about 0.7 µg/kg, about 0.8 µg/kg, about 0.9 µg/kg, and/or about 1 µg/kg.

In other embodiments, the dose may be selected from one or more of about 1 µg/kg, about 2 µg/kg, about 3 µg/kg, about 4 µg/kg, about 5 µg/kg, about 6 µg/kg, about 7 µg/kg, about 8 µg/kg, about 9 µg/kg, and/or about 10 µg/kg.

A pharmaceutically, or veterinarially, acceptable composition comprising the molecule comprising, or consisting of, Annexin A5 protein (such as monomeric Annexin A5 protein) can also be administered as defined above, wherein the composition is administered so that the amount of the molecule comprising, or consisting of, Annexin A5 protein (such as monomeric Annexin A5 protein) in the composition is about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg of the molecule.

In one embodiment, the subject is administered with the molecule comprising, or consisting of, Annexin A5 protein (such as monomeric Annexin A5 protein) in an amount of about 0.00001 mg/kg body weight to about "X" mg/kg body weight, wherein "X" is about 1 mg/kg, 0.5 mg/kg, 0.4 mg/kg, 0.3 mg/kg, 0.2 mg/kg, 0.1 mg/kg, 0.09 mg/kg, 0.08 mg/kg, 0.07 mg/kg, 0.06 mg/kg, 0.05 mg/kg, 0.04 mg/kg, 0.03 mg/kg, 0.02 mg/kg, 0.01 mg/kg, 0.009 mg/kg body weight or less, such as: about 0.008 mg/kg body weight or less; about 0.007 mg/kg body weight or less; about 0.006 mg/kg body weight or less; about 0.005 mg/kg body weight or less; about 0.004 mg/kg body weight or less; about 0.003 mg/kg body weight or less; about 0.002 mg/kg body weight or less; more preferably about 0.001 mg/kg body weight or less; 0.0009 mg/kg body weight or less, such as: about 0.0008 mg/kg body weight or less; about 0.0007 mg/kg body weight or less; about 0.0006 mg/kg body weight or less; about 0.0005 mg/kg body weight or less; about 0.0004 mg/kg body weight or less; about 0.0003 mg/kg body weight or less; about 0.0002 mg/kg body weight or less; about 0.0001 mg/kg body weight or less; about 0.00009 mg/kg body weight or less; about 0.00008 mg/kg body weight or less; about 0.00007 mg/kg body weight or less; about 0.00006 mg/kg body weight or less; about 0.00005 mg/kg body weight or less; about 0.00004 mg/kg body weight or less; about 0.00003 mg/kg body weight or less; or about 0.00002 mg/kg body weight or less of the molecule comprising, or consisting of, Annexin A5 protein (such as monomeric Annexin A5 protein).

In an additional or alternative embodiment, the subject is administered with the molecule comprising, or consisting of, Annexin A5 protein (such as monomeric Annexin A5 protein) in an amount of about "Y" mg/kg body weight to about 0.01 mg/kg body weight, preferably about "Y" mg/kg body weight to about 1 mg/kg, 0.5 mg/kg, 0.4 mg/kg, 0.3 mg/kg, 0.2 mg/kg, 0.1 mg/kg, 0.09 mg/kg, 0.08 mg/kg, 0.07 mg/kg, 0.06 mg/kg, 0.05 mg/kg, 0.04 mg/kg, 0.03 mg/kg, 0.02 mg/kg, or 0.001 mg/kg body weight, wherein "Y" is about 0.00002 mg/kg body weight or more, such as: about 0.00003 mg/kg body weight or more; about 0.00004 mg/kg body weight or more; about 0.00005 mg/kg body weight or more; about 0.00006 mg/kg body weight or more; about 0.00007 mg/kg body weight or more; about 0.00008 mg/kg body weight or more; about 0.00009 mg/kg body weight or more; about 0.0001 mg/kg body weight or more; about 0.0002 mg/kg body weight or more; about 0.0003 mg/kg body weight or more; about 0.0004 mg/kg body weight or more; about 0.0005 mg/kg body weight or more; about 0.0006 mg/kg body weight or more; about 0.0007 mg/kg body weight or more; about 0.0008 mg/kg body weight or more; or about 0.0009 mg/kg body weight or more; about 0.001 mg/kg body weight or more; about 0.002 mg/kg body weight or more; about 0.003 mg/kg body weight or more; about 0.004 mg/kg body weight or more; about 0.005 mg/kg body weight or more; about 0.006 mg/kg body weight or more; about 0.007 mg/kg body weight or more; about 0.008 mg/kg body weight or more; or about 0.009 mg/kg body weight or more of the molecule comprising, or consisting of, Annexin A5 protein (such as monomeric Annexin A5 protein).

In an additional or alternative embodiment, the subject is administered with the molecule comprising, or consisting of, Annexin A5 protein (such as monomeric Annexin A5 protein) in an amount of about "X" mg/kg body weight to about "Y" mg/kg body weight, wherein "X" and "Y" are each a value as defined above.

The foregoing doses per body weight may be particularly suitable for human subjects.

### (b) Total dose:

Additionally, or alternatively, the dosage may be determined independently of the body weight of the subject.

A preferred dose may be a total dose selected from one or more of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg. Optionally, the dose may be:
about 1 µg, about 2 µg, about 3 µg, about 4 µg, about 5 µg, about 6 µg, about 7 µg, about 8 µg, about 9 µg, and/or about 10 µg;
about 10 µg, about 20 µg, about 30 µg, about 40 µg, about 50 µg, about 60 µg, about 70 µg, about 80 µg, about 90 µg, and/or about 100 µg; or
about 100 µg, about 200 µg, about 300 µg, about 400 µg, about 500 µg, about 600 µg, about 700 µg, about 800 µg, about 900 µg, and/or about 1 mg.

The foregoing total doses may be particularly suitable for human subjects, in particular adult human subjects and/or a human subject with a body weight of from about 30 kg to about 200 kg, such as about 40 kg to about 150 kg, or 50 kg to about 120 kg, for example, about 60 kg to about 100 kg.

In an additional or alternative embodiment, the subject is administered with the molecule comprising, or consisting of, Annexin A5 protein (such as monomeric Annexin A5 protein) in amount of about 0.001 mg to about "A" mg, wherein "A" is about 5 mg, about 4 mg, about 3mg, about 2mg, less than about 2 mg, such as about 1 mg, about 0.9 mg or less, such as: about 0.8 mg or less; about 0.7 mg or less; about 0.6 mg or less; about 0.5 mg or less; about 0.4 mg or less; about 0.3 mg or less; about 0.2 mg or less; and more preferably about 0.1 mg or less; about 0.09 mg or less, such as: about 0.08 mg or less; about 0.07 mg or less; about 0.06 mg or less; about 0.05 mg or less; about 0.04 mg or less; about 0.03 mg or less; about 0.02 mg or less; about 0.01 mg or less; about 0.009 mg or less; about 0.008 mg or less; about 0.007 mg or less; about 0.006 mg or less; about 0.005 mg or less; about 0.004 mg or less; about 0.003 mg or less; or about 0.002 mg or less of the molecule comprising, or consisting of, Annexin A5 protein (such as monomeric Annexin A5 protein).

In an additional or alternative embodiment, the subject is administered with the molecule comprising, or consisting of, Annexin A5 protein (such as monomeric Annexin A5 protein) in an amount of about "B" mg to about 5 mg, about 4 mg, about 3mg, about 2mg, less than about 2 mg, such as about 1 mg, or about 0.1 mg, wherein "B" is about 0.002 mg or more, such as: about 0.003 mg or more; about 0.004 mg or more; about 0.005 mg or more; about 0.006 mg or more; about 0.007 mg or more; about 0.008 mg or more; about 0.009 mg or more; about 0.01 mg or more; about 0.02 mg or more; about 0.03 mg or more; about 0.04 mg or more; about 0.05 mg or more; about 0.06 mg or more; about 0.07 mg or more; about 0.08 mg or more, or about 0.09 mg or more of the molecule comprising, or consisting of, Annexin A5 protein (such as monomeric Annexin A5 protein).

In an additional or alternative embodiment, the subject is administered with the molecule comprising, or consisting of, Annexin A5 protein (such as monomeric Annexin A5 protein) in an amount of about "B" mg to about 5 mg, about 4 mg, about 3mg, about 2mg, less than about 2 mg, such as about 1 mg, wherein "B" is about 0.002 mg or more, such as: about 0.003 mg or more; about 0.004 mg or more; about 0.005 mg or more; about 0.006 mg or more; about 0.007 mg or more; about 0.008 mg or more; about 0.009 mg or more; about 0.01 mg or more; about 0.02 mg or more; about 0.03 mg or more; about 0.04 mg or more; about 0.05 mg or more; about 0.06 mg or more; about 0.07 mg or more; about 0.08 mg or more; about 0.09 mg or more; about 0.1 mg or more; about 0.2 mg or more; about 0.3 mg or more; about 0.4 mg or more; about 0.5 mg or more; about 0.6 mg or more; about 0.7 mg or more; about 0.8 mg or more; about 0.9 mg or more of the molecule comprising, or consisting of, Annexin A5 protein (such as monomeric Annexin A5 protein).

In an additional or alternative embodiment, the subject is administered with the molecule comprising, or consisting of, Annexin A5 protein (such as monomeric Annexin A5 protein) in an amount of about "A" mg to about "B" mg, wherein "A" and "B" are each a value as defined above.

### Administrative Routes:

In one preferred embodiment in the context of the prophylaxis or treatment of macular oedema, RVO, a condition associated with macular oedema or RVO, or any other ocular condition, the molecule comprising, or consisting of, Annexin A5 protein (such as monomeric Annexin A5 protein), or the composition thereof, is administered distal from the eye(s). Distal administration has the advantage of avoiding the administration of the molecule comprising, or consisting of, Annexin A5 protein (such as monomeric Annexin A5 protein) into the eye of the subject, which can be unpleasant for the subject. In particular, there are numerous potential complications associated with intravitreal injections (for example, using established anti-VEGF therapies for treatments of macular oedema and/or RVO), including infectious endophthalmitis, sterile intraocular inflammation, and retinal vasculitis particularly with brolucizumab (Cox *et al.,* 2021, *supra)* and transient intraocular pressure (IOP) spikes (Levin et al., 2021, J Glaucoma, 30:1019-26).

In one preferred embodiment in the context of the prophylaxis or treatment of macular oedema, RVO, a condition associated with macular oedema or RVO, or any other ocular condition, any one or more of the administrations of anti-VEGF agent to the subject may be made directly to the eye, for example by intravitreal injection. Noting the numerous potential complications associated with intravitreal injections of anti-VEGF agents, it is an objective of co-therapeutic approach of the present invention to make use of the molecule comprising, or consisting of, Annexin A5 protein (such as monomeric Annexin A5 protein) to enhance the prophylactic and/or therapeutic benefit of an anti-VEGF therapy, and/or to reduce complications associated with the requirements to administer of an anti-VEGF agent, for example by reducing the number of rounds of administration of the anti-VEGF agent, reducing the frequency of administration of the anti-VEGF agent, and/or reducing the period over which the anti-VEGF agent must be administered to the subject, required to achieve the desired prophylactic and/or therapeutic benefit or endpoint.

In accordance with one preferred option of all aspects of the present invention, the molecule comprising, or consisting of, Annexin A5 protein (such as monomeric Annexin A5 protein), or the composition thereof, is administered systemically or by suprachoroidal injection. Preferably, the molecule comprising, or consisting of, Annexin A5 protein (such as monomeric Annexin A5 protein), or the composition thereof, is administered into the vascular system of the subject. Most preferably, the molecule comprising, or consisting of, Annexin A5 protein (such as monomeric Annexin A5 protein), or the composition thereof, is administered into the blood of the subject.

In one embodiment, the molecule comprising, or consisting of, Annexin A5 protein (such as monomeric Annexin A5 protein), or the composition thereof, is administered by injection, transfusion, or infusion; such as intravenously or intra-arterially (in particular, an intravenous injection or an intra-arterial injection).

In a preferred embodiment, the molecule comprising, or consisting of, Annexin A5 protein (such as monomeric Annexin A5 protein), or the composition thereof, is administered by infusion.

In an alternative preferred embodiment, the molecule comprising, or consisting of, Annexin A5 protein (such as monomeric Annexin A5 protein), or the composition thereof, is administered by intravenous injection.

In a more preferable embodiment, the molecule comprising, or consisting of, Annexin A5 protein (such as monomeric Annexin A5 protein), or the composition thereof, is administered into the blood of the subject by infusion, for example, by intravenous injection.

In another embodiment, the molecule comprising, or consisting of, Annexin A5 protein (such as monomeric Annexin A5 protein), or the composition thereof, is administered parenterally, intrathecally, intra-peritoneally, intra-jointly. intra-muscularly, subcutaneously, or locally (although, as noted above, in one embodiment, it may optionally avoid administration directly to the eye).

In another option, albeit less preferred, the molecule comprising, or consisting of, Annexin A5 protein (such as monomeric Annexin A5 protein) may be administered directly to the eye. For example, in one optionally preferred embodiment, the molecule comprising, or consisting of, Annexin A5 protein (such as monomeric Annexin A5 protein) may be administered to one, or both, of the eyes of the subject by intravitreal injection. In accordance with this embodiment, the Annexin A5 protein may be administered to the patient in the form of a sterile injectable intravitreal composition, for example as defined in accordance with the fourth aspect of the present invention.

The composition for intravitreal injection may, for example, be presented in a sterile syringe, in a form ready to inject (e.g. without the requirement for reconstitution with a carrier or diluent). Preferably the syringe contains the composition in a volume of about or less than 200 µL, preferably a volume of about or less than 100 µL, and more preferably wherein the volume is about 10 to about 90 µL, about 20 to about 80 µL, about 30 to about 70 µL, about 40 to about 60 µL or about 50 µL (wherein the term "about" in this context refers to a volume that is ± 4, 3, 2 or 1 µL of the stated value).

The composition for intravitreal injection may, for example, be presented in the form of a kit comprising the composition and one or more additional components.

For example, the kit may comprise the composition presented in a sterile syringe and, affixed thereto or included separately in the kit, one or more sterile needles. Likewise, the uses and methods of the various aspects of the present invention may include intravitreal injection of the composition that comprises an effective amount of the molecule consisting of monomeric Annexin A5 protein to one, or both, of the eyes of the subject, via a sterile needle.

Preferably, the or each needle may be a gauge selected from gauge 27, 28, 29, or more preferably 30 or 31. It may be preferred that the or each needle has a length of from about 0.5 to 0.62 inches (12.7 to 15.75 mm) in length. Longer needles may increase risk of retinal injury if the patient accidentally moves forward during the intravitreal injection procedure.

In the less preferred embodiment in which the composition is administered to the subject by intravitreal injection, then the procedure may include one or more pre-injection risk management steps selected from: 1) Apply a local anaesthetic; 2) apply an agent suitable to decrease the risk of bacterial colonisation (povidone-iodine drops and/or periocular povidone-iodine eyelid preparation may be particularly suitable); 3) insert a sterile speculum to separate the lids; and 4) optionally, reapply an agent suitable to decrease the risk of bacterial colonisation (e.g. povidone-iodine) immediately over the injection site prior to injection.

In the embodiment in which an intravitreal injection is performed with local anaesthesia, topical anaesthetic drops may be conveniently employed.

Any agent suitable to decrease the risk of bacterial colonisation may be used in the eye as part of a pre-injection risk management protocol. Povidone-iodine is one preferred option. Povidone-iodine has been shown to decrease bacterial colonization as well as the risk of endophthalmitis. Application of povidone-iodine to the conjunctival surface, eyelids, and lashes is recommended prior to the intravitreal injection, and preferably also prior to introducing a sterile speculum. (The speculum prevents the needle tip from touching the lids or lashes prior to needle insertion.) Povidone-iodine may optionally be used in a 5% to 10% solution. Studies have found that a 5 percent povidone-iodine solution is as effective as 10 percent and is less irritating to the eye. Optionally, the agent suitable to decrease the risk of bacterial colonisation (e.g. povidone-iodine) is reapplied immediately over the injection site prior to injection.

Antibiotics may be used before, during and/or after intravitreal injection. Any suitable antibiotics may be used. One exemplary embodiment is fourth-generation fluoroquinolone.

In another example, the kit may comprise one or more further compositions, each comprising one or more antibiotics, for administration to the patient for and/or after intravitreal injection.

Those skilled in the art will be able to select a suitable intravitreal injection site. For example, in one exemplary embodiment, the patient is instructed to direct their gaze away from the site of needle entry, the injection is placed about 3 to about 3.5 mm posterior to the limbus (e.g. for an aphakic or pseudophakic eye), or about 3.5 mm to about 4 mm posterior to the limbus (e.g. for a phakic eye). Injection in the inferotemporal quadrant is common, although any quadrant (e.g. the superotemporal quadrant) may be used.

In another option, the molecule comprising, or consisting of, Annexin A5 protein (such as monomeric Annexin A5) protein may be administered directly to a retinal vein (such as the central retinal vein, the branch retinal vein and/or the hemi-retinal vein) of one, or both, of the eyes of the subject, for example by catherisation

### Administrative Schedules:

In light of the disclosure of the present invention, and applying common general knowledge thereto, the skilled person is readily able to determine a suitable dosage in order to achieve the therapeutic effect desired, optionally in the range of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of a subject, and/or about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg of the molecule. For example, a suitable dosage will be typically provide and maintain an adequate amount of the molecule comprising, or consisting of, Annexin A5 protein (such as monomeric Annexin A5 protein) to treat or prevent macular oedema and/or retinal vein occlusion, or an associated condition. A suitable dosage may aim to achieve and/or maintain a level of Annexin A5 protein in the blood plasma of the subject at greater that naturally-occurring physiological levels of Annexin A5 in the blood plasma.

In other embodiments, the administration of the dose is over an extended period of time, for example where a daily dosage is delivered over a period of time of 30 or more minutes, such as: one hour or more; two hours or more, three hours or more; four hours or more; five hours or more; six hours or more; seven hours or more; eight hours or more; nine hours or more; 10 hours or more; 11 hours or more; 12 hours or more; 13 hours or more; 14 hours or more; 15 hours or more; 16 hours or more; 17 hours or more; 18 hours or more; or up to 24 hours. In a preferred embodiment, the daily dosage is delivered over the period of time by infusion.

The treatment regime may, for example, involve the continuous infusion of Annexin A5 protein to the subject, or can involve one or more administrations of a dose consisting of an amount of the molecule comprising, or consisting of, Annexin A5 protein (such as monomeric Annexin A5 protein) as defined in respect of the foregoing embodiments, for example, once, twice, three, four or more times daily.

In some cases, a therapeutically effective dose of the molecule comprising, or consisting of, Annexin A5 protein (such as monomeric Annexin A5 protein) may optionally be administered to the subject as two or more doses of escalating concentration (i.e., increasing doses), where (i) all of the doses are therapeutic doses, or where (ii) a sub-therapeutic dose (or two or more sub-therapeutic doses) is initially given and therapeutic doses are achieved by said escalation. As one non-limiting example to illustrate escalating concentration (i.e., increasing doses), a therapeutically effective dose can be administered daily, beginning with a sub-therapeutic dose, and each subsequent dose can be increased by a particular increment, or by variable increments, until a therapeutic dose is reached, at which point administration may cease or may continue (e.g., continued therapeutic doses). In some embodiments, administration of a therapeutically effective dose can be a continuous infusion and the dose can altered (e.g., escalated) over time. In some embodiments a combination therapy is also administered.

The physician in any event will determine the actual dosage of the molecule comprising, or consisting of, Annexin A5 protein (such as monomeric Annexin A5 protein) which will be most suitable for any individual patient and it will vary with the age, weight and response of the particular patient. The above dosages are exemplary of the average case. There can, of course, be individual instances where higher or lower dosage ranges are merited and such are within the scope of this invention.

For veterinary use, the Annexin A5 protein is administered as a suitably acceptable formulation in accordance with normal veterinary practice and the veterinary surgeon will determine the dosing regimen and route of administration which will be most appropriate for a particular animal.

### Treatment Periods:

The treatment regime of the molecule comprising, or consisting of, Annexin A5 protein (such as monomeric Annexin A5 protein) may be continued for a therapeutically beneficial period.

In one embodiment, the treatment may consist of a single administration of the Annexin A5 composition.

In another embodiment, multiple administrations to the subject may be made. For example, a dose consisting of an amount of the molecule comprising, or consisting of, Annexin A5 protein (such as monomeric Annexin A5 protein) as defined in respect of the foregoing embodiments may be administered once, or more, *per* day, such as: twice or more; three times or more; four times or more; five time or more; six times or more; seven times or more; eight times or more; nine times or more; or ten times or more *per* day, preferably, once *per* day.

In one embodiment, the dose of the molecule comprising, or consisting of, Annexin A5 protein (such as monomeric Annexin A5 protein) is administered once or more each two days, once or more each three days, once or more each four days, once or more each five days, once or more each six days, once or more each seven days, once or more each week, once or more each two weeks, once or more each three weeks, once or more each four weeks, once or more each month, once or more each two months, once or more each three months, once or more each four months, once or more each five months, once or more each six month, once or more each seven months, once or more each eight months, once or more each nine months, once or more each 10 months, once or more each 11 months, once or more each 12 months, once or more each two years, once or more each three years, once or more each four years, once or more each five years, or once or more each 10 years.

In one embodiment, the dose of the molecule comprising, or consisting of, Annexin A5 protein (such as monomeric Annexin A5 protein) is administered daily for a period of one day or more, such as: two consecutive days or more; three consecutive days or more; four consecutive days or more; five consecutive days or more; six consecutive days or more; seven consecutive days or more; eight consecutive days or more; nine consecutive days or more; ten consecutive days or more; 11 consecutive days or more; 12 consecutive days or more; 13 consecutive days or more; two weeks of consecutive days or more; three weeks of consecutive days or more; four weeks of consecutive days or more; two months of consecutive days or more; three months of consecutive days or more; four months of consecutive days or more; five months of consecutive days or more; six months of consecutive days or more; seven months of consecutive days or more; eight months of consecutive days or more; nine months of consecutive days or more; ten months of consecutive days or more; 11 months of consecutive days or more; 12 months of consecutive days or more; 15 months of consecutive days or more; 18 months of consecutive days or more; 21 months of consecutive days or more; 24 months of consecutive days or more; 30 months of consecutive days or more; three years of consecutive days or more; 42 months of consecutive days or more; four years of consecutive days or more; five years of consecutive days or more; six years of consecutive days or more; seven years of consecutive days or more; eight years of consecutive days or more; nine years of consecutive days or more; ten years of consecutive days of more; 11 years of consecutive days or more; 12 years of consecutive days or more; 13 years of consecutive days or more; 14 years of consecutive days or more; 15 years of consecutive days or more; 16 years of consecutive days or more; 17 years of consecutive days or more; 18 years of consecutive days or more; 19 years of consecutive days or more; 20 years of consecutive days or more; 25 years of consecutive days or more; 30 years of consecutive days or more; 35 years of consecutive days or more; 40 years of consecutive days or more; or 50 years of consecutive days or more. In a preferred embodiment, the molecule comprising, or consisting of, Annexin A5 protein (such as monomeric Annexin A5 protein) is administered daily for a period of six or more consecutive days. In an alternative preferred embodiment, the molecule comprising, or consisting of, Annexin A5 protein (such as monomeric Annexin A5 protein) is administered daily for a period of seven or more consecutive days. In a more preferred embodiment, the Annexin A5 protein is administered daily for a period of six consecutive days or seven consecutive days. In a most preferred embodiment, the Annexin A5 protein is administered daily for a period of six consecutive days. In an alternative and most preferred embodiment, the Annexin A5 protein is administered daily for a period of seven consecutive days.

In one embodiment, the molecule comprising, or consisting of, Annexin A5 protein (such as monomeric Annexin A5 protein) is administered twice or more daily for a period of one week or more: for example, two weeks or more; three weeks or more; four weeks or more; two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule comprising, or consisting of, Annexin A5 protein (such as monomeric Annexin A5 protein) is administered once weekly for a period of one week or more: for example, two weeks or more; three weeks or more; four weeks or more; two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule comprising, or consisting of, Annexin A5 protein (such as monomeric Annexin A5 protein) is administered twice or more weekly for a period of one week or more: for example, two weeks or more; three weeks or more; four weeks or more; two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule comprising, or consisting of, Annexin A5 protein (such as monomeric Annexin A5 protein) is administered once monthly for a period of one month or more: for example, two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

In one embodiment, the molecule comprising, or consisting of, Annexin A5 protein (such as monomeric Annexin A5 protein) is administered twice or more monthly for a period of one month or more: for example, two months or more; three months or more; four months or more; five months or more; six months or more; seven months or more; eight months or more; nine months or more; ten months or more; 11 months or more; 12 months or more; 15 months or more; 18 months or more; 21 months or more; 24 months or more; 30 months or more; three years or more; 42 months or more; four years or more; five years or more; six years or more; seven years or more; eight years or more; nine years or more; ten years of more; 11 years or more; 12 years or more; 13 years or more; 14 years or more; 15 years or more; 16 years or more; 17 years or more; 18 years or more; 19 years or more; 20 years or more; 25 years or more; 30 years or more; 35 years or more; 40 years or more; or 50 years or more.

### Combination therapies:

All aspects of the present invention relate to a combination therapy making use of the administration of the molecule comprising, or consisting of, Annexin A5 protein (such as monomeric Annexin A5 protein) as a co-therapy with one or more anti-VEGF agents.

Thus, a subject for treatment in accordance with the present invention can be administered the molecule comprising, or consisting of, Annexin A5 protein (monomeric Annexin A5 protein) in an amount and for a time to provide an overall therapeutic effect, as part of a co-therapy with one or more anti-VEGF agents. The molecule comprising, or consisting of, Annexin A5 protein (monomeric Annexin A5 protein A5) can be in combination with the one or more anti-VEGF agents (combination therapy), either in admixture or by separate, simultaneous or sequential administration.

Optionally, the co-therapy using the Annexin A5 protein and the anti-VEGF agent can be further supplemented with one or more additional agents. The one or more additional agents can be administered in combination with the Annexin A5 component and the one or more anti-VEGF agents, either in admixture or by separate, simultaneous or sequential administration relative to either or both of the Annexin A5 component and the one or more anti-VEGF agents. For the avoidance of doubt, the other agent is not an agent that comprises the sequence of human Annexin A5 or a biologically active fragment thereof and, more particularly, is not a dimeric Annexin A5 (commonly referred to in the art as "Diannexin"), and is not a PEGylated Annexin A5.

The amounts and times of administration of the Annexin A5 component, and the anti-VEGF component, can be those that provide, e.g., an additive or a synergistic prophylactic and/or therapeutic effect. Further, the administration of the molecule comprising, or consisting of, Annexin A5 protein, such as monomeric Annexin A5 protein, (with or without the second or further agent(s)) can be used as a primary, e.g., first line treatment, or as a secondary treatment, relative to the one or more anti-VEGF agents and/or the one or more additional agents. For example, the Annexin A5 can be administered as a secondary treatment for subjects who have an inadequate response to a previously administered therapy with anti-VEGF agent and/or one or more additional agents (i.e., to a previously-administered therapy other than one with the molecule comprising, or consisting of, Annexin A5 protein, such as monomeric Annexin A5 protein).

Accordingly, in one embodiment, the subject may be treated with the molecule comprising, or consisting of, Annexin A5 protein, such as monomeric Annexin A5 protein, therapy in addition to one or more therapeutic or prophylactic interventions with anti-VEGF agent and/or one or more additional agents. The subject may receive the molecule comprising, or consisting of, Annexin A5 protein, such as monomeric Annexin A5 protein, therapy in the same composition as, separately from, simultaneously with a separate formulation containing, or sequentially with, one or more other therapeutic or prophylactic interventions with anti-VEGF agent and/or one or more additional agents, or one or more additional therapeutic or prophylactic treatments with anti-VEGF agent and/or one or more additional agents.

In some embodiment of the present invention, the one or more other therapeutic or prophylactic interventions, or one or more additional therapeutic or prophylactic treatments comprise, or consist of one or more treatment selected from the group consisting of anti-VEGF therapy (e.g. anti-VEGF therapy delivered into the eye by intravitreal injection), corticosteroids, NSAIDs, pars plana vitrectomy, retinal laser photocoagulation, or anti-VEGF therapy that is augmented with intravitreal corticosteroid and/or retinal laser photocoagulation.

Exemplary anti-VEGF therapies can include any one or more of Ranibizumab, Aflibercept, Bevacizumab, Brolucizumab and/or faricimab.

Exemplary corticosteroids can include difluprednate, triamcinolone acetonide, triamcinolone acetonide, and dexamethasone which may be used as monotherapy, which may or may not be in combination with an immunomodulatory therapy to achieve a more rapid resolution of the oedema.

### Devices and Kits for Therapy:

Pharmaceutical and veterinary compositions that include the molecule comprising, or consisting of, Annexin A5 protein, such as monomeric Annexin A5 protein, can be administered with a medical device. The device can designed with features such as portability, room temperature storage, and ease of use so that it can be used in emergency situations, e.g., by an untrained subject or by emergency personnel in the field, removed from medical facilities and other medical equipment. The device can include, e.g., one or more housings for storing pharmaceutical preparations that include the molecule comprising, or consisting of, Annexin A5 protein, such as monomeric Annexin A5 protein, and can be configured to deliver one or more unit doses of the molecule comprising, or consisting of, Annexin A5 protein, such as monomeric Annexin A5 protein. The device can be further configured to administer a second agent such as an anti-VEGF agent, either as a single pharmaceutical composition that also includes the molecule comprising, or consisting of, Annexin A5 protein, such as monomeric Annexin A5 protein, or as two separate compositions.

The pharmaceutical and veterinary composition may be administered with a syringe. The pharmaceutical or veterinary composition can also be administered with a needleless hypodermic injection device, such as the devices disclosed in US 5,399, 163; 5,383,851; 5,312,335; 5,064,413; 4,941,880; 4,790,824; or 4,596,556. Examples of well-known implants and modules include: US 4,487,603, which discloses an implantable micro-infusion pump for dispensing medication at a controlled rate; US 4,486, 194, which discloses a therapeutic device for administering medicaments through the skin; US 4,447,233, which discloses a medication infusion pump for delivering medication at a precise infusion rate; US 4,447,224, which discloses a variable flow implantable infusion apparatus for continuous drug delivery; US 4,439, 196, which discloses an osmotic drug delivery system having multi-chamber compartments; and US 4,475,196, which discloses an osmotic drug delivery system. Many other devices, implants, delivery systems, and modules are also known.

The molecule comprising, or consisting of, Annexin A5 protein, such as monomeric Annexin A5 protein, can be provided in a kit. In one embodiment, the kit includes (a) a container that contains a composition that includes the Annexin A5 protein, and optionally (b) informational material. The informational material can be descriptive, instructional, marketing or other material that relates to the methods described herein and/or the use of the agents for therapeutic benefit.

In an embodiment, the kit also includes a second agent for treating a disorder described herein. For example, the kit includes a first container that contains a composition that includes the molecule consisting of monomeric Annexin A5 protein, and a second container that includes the second agent. Optionally, the second agent is an anti-VEGF agent.

The informational material of the kits is not limited in its form. In one embodiment, the informational material can include information about production of the compound, molecular weight of the compound, concentration, date of expiration, batch or production site information, and so forth. In one embodiment, the informational material relates to methods of administering the molecule comprising, or consisting of, Annexin A5 protein, such as monomeric Annexin A5 protein, e.g., in a suitable dose, dosage form, or mode of administration (e.g., a dose, dosage form, or mode of administration described herein), to treat a subject in need thereof as described herein. In the case of kits that include anti-VEGF agent as a second agent, the kit may also include informational material related to methods of administering the anti-VEGF agent, e.g., in a suitable dose, dosage form, or mode of administration (e.g., a dose, dosage form, or mode of administration described herein), to treat a subject in need thereof as described herein. The information can be provided in a variety of formats, include printed text, computer readable material, video recording, or audio recording, or information that provides a link or address to substantive material, e.g., on the internet. In one embodiment, the informational material provides instructions administering the molecule comprising, or consisting of, Annexin A5 protein, such as monomeric Annexin A5 protein, to the subject with a dose of about 50 µg/kg to about 100 µg/kg, about 40 µg/kg to about 80 µg/kg, about 30 µg/kg to about 60 µg/kg, about 20 µg/kg to about 40 µg/kg, about 1 µg/kg to 30 µg/kg, less than about 20 µg/kg, such as about 0.01 µg/kg to about 10 µg/kg, or about 0.01 µg/kg to about 1 µg/kg body weight of a subject, and/or administering the subject with a dose of about 5 mg, about 4 mg, about 3mg, about 2mg, less than about 2 mg, such as about 1 mg, about 0.1-1.5 mg, 0.001 mg to about 1 mg, or about 0.001 mg to about 0.1 mg of the molecule comprising, or consisting of, Annexin A5 protein, such as monomeric Annexin A5 protein.

In addition to the molecule comprising, or consisting of, Annexin A5 protein, such as monomeric Annexin A5 protein, the composition in the kit can include other ingredients, such as a solvent or buffer, a stabilizer, or a preservative. The molecule comprising, or consisting of, Annexin A5 protein, such as monomeric Annexin A5 protein, can be provided in any form, e.g., liquid, dried or lyophilized form, preferably substantially pure and/or sterile. When the molecule comprising, or consisting of, Annexin A5 protein, such as monomeric Annexin A5 protein, is provided in a liquid solution, the liquid solution preferably is an aqueous solution. When the molecule comprising, or consisting of, Annexin A5 protein, such as monomeric Annexin A5 protein, is provided as a dried form, reconstitution generally is by the addition of a suitable solvent. The solvent, e.g., sterile water or buffer, can optionally be provided in the kit.

In the case of kits that include anti-VEGF agent as a second agent, the anti-VEGF agent can be provided in any form, e.g., liquid, dried or lyophilized form, preferably substantially pure and/or sterile. When the anti-VEGF agent is provided in a liquid solution, the liquid solution preferably is an aqueous solution. When the anti-VEGF agent is provided as a dried form, reconstitution generally is by the addition of a suitable solvent. The solvent, e.g., sterile water or buffer, can optionally be provided in the kit.

The kit can include one or more containers for the composition or compositions containing the agents. In some embodiments, the kit contains separate containers, dividers or compartments for the composition and informational material. For example, the composition can be contained in a bottle, vial, or syringe, and the informational material can be contained in a plastic sleeve or packet. In other embodiments, the separate elements of the kit are contained within a single, undivided container. For example, the composition is contained in a bottle, vial or syringe that has attached thereto the informational material in the form of a label. In some embodiments, the kit includes a plurality (*e.g*., a pack) of individual containers, each containing one or more unit dosage forms (*e.g*., a dosage form described herein) of the agents. The containers can include a combination unit dosage, e.g*.*, a unit that includes both the molecule comprising, or consisting of, Annexin A5 protein, such as monomeric Annexin A5 protein, and a second agent such as the anti-VEGF agent, *e*.*g*., in a desired ratio. For example, the kit includes a plurality of syringes, ampules, foil packets, blister packs, or medical devices, *e.g*., each containing a single combination unit dose. The containers of the kits can be air tight, waterproof (*e.g*., impermeable to changes in moisture or evaporation), and/or light-tight.

The kit optionally includes one or more devices suitable for administration of the or each composition within the kit, e.g., a syringe or other suitable delivery device. The or each device can be provided pre-loaded with one or both of the compositions or can be empty, but suitable for loading.

### F. Method of calculating dosages of Annexin A5

Also described herein is a method for calculating a dosage of Annexin A5 required to treat a condition, such as macular oedema and/or RVO, comprising the steps of:
A. determining the number of Annexin A5 positive cells in a subject;
B. determining the number of Annexin A5 binding sites on the Annexin positive cells in the subject;
C. determining the number of Annexin A5 molecules required to bind to the Annexin A5 binding sites, such as by multiplying the value determined for A and the value determined for B;
D. determining the dosage, from value determined for C.

The embodiments of the invention discussed herein are applicable to this method, unless otherwise stated.

In one embodiment, the method further comprises:
C'. determining the molarity of the number of molecules required to bind to the Annexin A5 binding sites, from the value determined in C; and,
D. further comprises determining the dosage, for the value determined for C'.

Preferably, D further comprises determining the dosage by multiplying the value determined for C' with the molar mass of Annexin A5. Preferably, the molar mass of Annexin A5 that is used is about 35,700 Da. If an Annexin A5 protein variant with a different molecular mass is used, the value used in calculating step D can be adjusted accordingly.

In one embodiment, the Annexin A5 is monomeric Annexin A5, such as a molecule comprising monomeric Annexin A5 protein as defined further herein.

In one embodiment, the method comprises:
E. providing the dosage of Annexin A5 for use in administering said dose to the subject.

Preferably, the method comprises
E. providing the dosage of Annexin A5 for use in treating the condition in said subject.

In one embodiment, the method is an *in vitro* method. In another embodiment, steps A-D of the method are *in vitro* method steps.

In one embodiment, the condition is one or more selected from the list consisting of: viral haemorrhagic fever (such as a ebola virus viral haemorrhagic fever or a dengue virus viral haemorrhagic fever); a cardiovascular condition (such as stroke, atherosclerosis, central retinal vein occlusion, myocardial infarction, stenosis, or restenosis); sickle cell disease; arthritis (such as osteoarthrosis), and diabetes. Preferably, the condition is retinal vein occlusion, more preferably central retinal vein occlusion.

Also described herein is a method of calculating the dosage of Annexin A5 required to bind the Annexin A5 binding sites in a subject, comprising the steps of:
A. determining the number of Annexin A5 positive cells in the subject;
B. determining the number of Annexin A5 binding sites on the Annexin A5 positive cells in the subject;
C. determining the number of Annexin A5 molecules required to bind to the Annexin A5 binding sites, such as by multiplying the value determined for A and the value determined for B;
D. determining the dosage, from value determined for C.

The embodiments of the invention discussed herein are applicable to this method, unless otherwise stated.

In one embodiment, the method comprises
E. providing the dosage of Annexin A5 for use in administration to the subject, for prophylaxis; preferably, for prophylaxis of a condition.

The present invention will now be described with reference to one or more non-limiting examples.

### EXAMPLES

The following examples are included to demonstrate particular embodiments of the invention. It should be appreciated by those of skill in the art that the techniques disclosed in the examples which follow represent techniques discovered by the inventor to function well in the practice of the invention, and thus can be considered to constitute preferred modes for its practice.

### Example 1

### Probe Construction and Validation:

Purified recombinant monomeric human Annexin A5 protein (ANXV) was labelled with 800CW, a molecular imaging and contrast agent, to form an ANXV-800CW agent, in which it was determined that about 40% of the ANXV present was labelled with 800CW (and the remaining approximately 60% remained as unlabelled monomeric human Annexin A5 protein).

The ANXV-800CW binds specifically to Phosphatidylserine (PS), a key molecule in the pathogenesis of RVO.

A 66-year-old woman, known with systemic hypertension, was seen at the outpatient clinic. She presented with a decreased vision of her right eye in recent weeks, which was determined to be caused by a branch retinal venous occlusion (BRVO).

Using a PS-assay, performed on blood samples taken before and after systemic administration of ANXV-800CW, the presence of PS on fresh red blood cells was determined with flow cytometry using FITC labelled Annexin A5. These results showed the presence and an increased percentage of PS availability compared to a negative control **(****Figure 1****).** This increase of PS externalization was observed during all blood sampling time points, demonstrating the stable availability of the target of ANXV-800CW.

### Assessing the localization of intravenously-administered Annexin A5 to the site of a Retinal Vein Occlusion:

Next, the possibility to visualize binding of ANXV-800CW at the site of the retinal vein occlusion was assessed.

1.0 mg of ANXV-800CW was administered intravenously as a single bolus, after which Near-InfraRed (NIR) fluorescence imaging with the Heidelberg Spectralis camera system was performed at predefined time points (maximum of 4 hours after injection).

**Figure 2** shows the results of fluorescein angiography (FA), which is performed as standard of care. It shows the exact occlusion site (red arrow) and the damaged retinal area in which bleeding is present (red oval). When performing NIR fluorescence imaging we observed the likely fluorescent signal of ANXV-800CW in the vessels located the site of the affected retinal area and in the optic nerve, at 5 minutes after administration. This intensity appears to increase over time, as shown by the image made at 229 minutes after injection.

In the surrounding retinal vessels, as identified by the FA, no fluorescent signal could be detected (see **Figure 3****).** This is indicative for the absence of a distinct ANXV-800CW uptake in the vessels which are not affected by the BRVO.

These observations are indicative of site-specific accumulation of ANXV-800CW at the retinal vessels, and imply the binding of Annexin A5 at the site of the occlusion. We interpret these results as indicative of the efficacy of systemically-administered Annexin A5 (including the labelled form ANXV-800CW) in preferentially accumulating at exposed PS, its target, at the site of an occlusion in a subject with RVO.

### Example 2

**Background:** The established standard for treating retinal vein occlusion (RVO) involves long-term frequent reinjection of anti-VEGF directly into the eye by intravitreal injection (Nguyen et al., 2010, Ophthalmology, 117:2146-51; Campochiaro et al., 2010, Ophthalmology, 117:2387-94; Saint-Geniez et al, 2008, PLoS One., 3:e3554; Haydinger *et al,* 2023, *supra;* lp & Hendrick, 2018, *supra;* Cox et al., 2021, J. Clin. Med., 10(5): 981; Hattenbach et al., 2023, Ophthalmol Sci., 3(3): 100302). However, there are concerns of ocular and systemic toxicity in anti-VEGF therapy, and long-term frequent reinjection is required (Nguyen et al., 2010, Ophthalmology, 117:2146-51; Campochiaro et al., 2010, Ophthalmology, 117:2387-94; Saint-Geniez et al, 2008, PLoS One., 3:e3554). Potential complications associated with intravitreal injections of anti-VEGF therapy include infectious endophthalmitis, sterile intraocular inflammation, and retinal vasculitis particularly with brolucizumab (Cox *et al.,* 2021, *supra*) and transient intraocular pressure (IOP) spikes (Levin et al., 2021, J Glaucoma, 30:1019-26).

Phosphatidylserine (PS) is a cell membrane lipid implicated in the pathophysiology of retinal vein occlusion (RVO), diabetic retinopathy (DMR) and age-related macular degeneration (AMD). In RVO patients, PS is exposed on circulating red blood cells (RBCs) and contributes to their adhesive properties to e.g. microvascular endothelium, a feature implied as causative in RVO. ANXV, a recombinant human Annexin A5 protein, is a PS inhibitor acting as anti-inflammatory, cell protective and anti-adhesive agent.

**Methods:** All treatment-naive early onset RVO patients were eligible for the trial. A total of 15 patients have received intravenously-administered ANXV as a first line treatment, of which six patients have received daily doses of 2 mg, three patients have received 4mg and six patients 6 mg of ANXV (2 mg; n=6; 4 mg; n=3; 6mg: n=6). The ANXV doses were administered either for 5 days consecutively for some patients, or for five days within an eight day period for other patients (primarily where the weekend interrupted access to treatment of consecutive days within the treatment period). All patients were followed for adverse events (AEs), lab parameters, pharmacokinetics (PKs), anti-drug antibodies (ADAs), ophthalmic parameters and requirement of IVT anti-VEGF. Patients were (or, in the case of ongoing tests, will be) studied for at least four months, and generally, longer, after the initial treatment. At the time of writing, 8 patients have been studied for at least 4 months (4 m), 2 patients have been studied for at least 3 months (3 m), 2 patients have been studied for at least 2 months (2 m), one patient has been studied for at least one month (1 m), and one patient is in the initial stages of study of less than one month.

**Results:** A review of available safety and tolerability data from the patients who received either 2, 4 or 6 mg ANXV daily dosing and were followed 10 days after receiving the last dose of ANXV did not reveal any findings of concern.

In terms of signals of effect 8 out of 10 patients, where information is available for at least three months after the treatment, data have shown an improvement or prevention of worsening of their visual acuity (BCVA) and in several cases a reduced swelling of the retina with no or only one dose of anti-VEGF. Similarly, 10 of 12 patients, based on information available after two months have been deemed not to require any or only one anti-VEGF treatment. The decision to administer anti-VEGF is based on the presence of swelling of the retina and poor visual acuity and is taken by the patient's treating ophthalmologist.

| Subject | ANXV Daily Dose | Diagnosis | Current Study Period | Positive response to ANXV therapy, during study period | | | | Anti-VEGF used during study period? |
|---|---|---|---|---|---|---|---|---|
| | | | | 1m | 2m | 3m | 4m or longer | |
| A | 2 mg | BRVO | > 4m | Yes | Yes | Yes | Yes | One dose |
| B | 2 mg | CRVO | > 4m | No | No | No | No | One dose |
| C | 2 mg | BRVO | > 4m | Yes | Yes | Yes | Yes | One dose |
| D | 2 mg | CRVO | > 4m | No | No | No | No | Multiple doses |
| E | 2 mg | CRVO | > 4m | Yes | Yes | Yes | Yes | One dose |
| F | 2 mg | CRVO | > 4m | Yes | Yes | Yes | Yes | No |
| G | 4 mg | BRVO | > 4m | Yes | Yes | Yes | Yes | No |
| H | 4 mg | BRVO | > 4m | Yes | Yes | Yes | Yes | No |
| I | 4 mg | BRVO | 3 m | Yes | Yes | Yes | T.B.C. | No |
| J | 6 mg | BRVO | 3 m | Yes | Yes | Yes | T.B.C. | No |
| K | 6 mg | CRVO | 2 m | Yes | Yes | T.B.C. | T.B.C. | No |
| L | 6 mg | BRVO | 2m | Yes | Yes | T.B.C. | T.B.C. | No |
| M | 6 mg | HRVO | 1 m | Yes | T.B.C. | T.B.C. | T.B.C. | No |
| N | 6 mg | CRVO | 1 m | Yes | T.B.C. | T.B.C. | T.B.C. | No |
| O | 6 mg | BRVO | < 1m | T.B.C. | T.B.C. | T.B.C. | T.B.C. | No |

T.B.C.= to be confirmed. At the time of writing, the study of that particular patient is ongoing, and has not yet reached the time point indicated.

13 of the 15 tested RVO patients have responded positively (improved, or at least stabilized) by the ANXV treatment. Only two patients (subjects B and D, both of whom received the lowest texted ANXV dose) did not respond and reverted to conventional anti-VEGF treatments during the study period.

When assessed solely for visual acuity, using a BCVA test (letters gain), exemplary subjects showed the following outcomes, with improvements particularly notable from day 29 onwards:

| Subject | ANXV Dose | Visual Accuity Score, BCVA (letters gained) at indicated day | | | | | | | Signal of ANXV effect | Anti-VEGF |
|---|---|---|---|---|---|---|---|---|---|---|
| | | 0 | 1 | 15 | 29 | 60 | 90 | 120 | | |
| A | 2 mg | 50 | n.d. | 55 | 81 (+31) | 65 (+15) | stable | stable | yes | 1 dose |
| C | 2 mg | 59 | n.d. | 50 | 66 (+7) | stable | stable | stable | yes | 1 dose |
| E | 2 mg | 77 | 79 | 77 | 84 (+7) | 77 | 86 (+9) | 85 (+8) | yes | 1 dose |
| F | 2 mg | 70 | 67 | 72 | 81 (+11) | 90 (+20) | 90 (+20) | 86 (+16) | yes | no |
| G | 4 mg | 70 | 75 | 73 | 74 (+4) | 74 (+4) | 74 (+4) | 75 (+5) | yes | no |

Compared to the anticipated number of patients who would normally receive an average of four to six anti-VEGF intravitreal injections within the first six months of diagnosis of RVO (Light et al, 2020, Am. Acad. Ophthamol., 5(9): 888), the number of anti-VEGF injections deemed necessary by the treating ophthalmologist in the ANXV study group was zero or only one in 8 of 10 patients followed for 3 months and 10 of 12 followed for 2 months.

One such exemplary patient (subject F, diagnosed with CRVO, administered 5x daily doses of 2mg ANXV), was studied for 7 months and then exited the study having reached the clinical end point, having demonstrated a long-term and sustained improvement in in visual acuity (BCVA) and reduced macular retinal thickness and macular volume:
Subject F:

| Test | Day of Study (relative to ANXV treatment) | | | | | | |
|---|---|---|---|---|---|---|---|
| | 0 | 1 | 15 | 29 | 60 | 90 | 120 |
| BCVA score | 70 | 67 | 72 | 81 | 90 | 90 | 86 |
| Macular retinal thickness (µm) central subfield - RE | 418 | 371 | 276 | 267 | N.D. | N.D. | N.D. |
| Macular retinal thickness (µm) center point - RE | 416 | 350 | 255 | 238 | N.D. | N.D. | N.D. |
| Macular volume (mm³) - RE | 11.65 | 10.25 | 9.13 | 8.81 | N.D. | N.D. | N.D. |

| | | | | | | | |
|---|---|---|---|---|---|---|---|
| N.D. = not determined | | | | | | | |

A microperimetry (MMP) acquired retinal sensitivity map for subject F is shown in Fig 4A and demonstrates clear improvement using ANXV therapy at 15 days post treatment, through to complete recovery by 120 days post-treatment. Imaging of blood flow in the retinal vasculature by Fluorocein Angiography for subject F is shown in Fig 4B and, again, demonstrates clear improvement using ANXV therapy at 15 days post treatment, through to complete recovery by 29 or 120 days post-treatment.

However, three of the patients showing a signal effect to the ANXV administration (subjects A, C and E) were determined to require additional anti-VEGF treatment during the study period.

One of these subjects (Subject A) received aflibercept (Eylea^{™}) about 32 days into the study. This subject was monitored by the attending ophthalmologist for a total of 16 months from initial ANXV therapy and did not require any additional anti-VEGF treatments during the study period.

### Subject A:

| | Day of Study (relative to ANXV treatment) | | | | | | | | |
|---|---|---|---|---|---|---|---|---|---|
| | 0 | 1 | 8 | 15 | 29 | ≥32 | 60 | 90 | 120 |
| Additional anti-VEGF treatment (2mg Eylea^{™} IVT | | | | | | Yes | | | |
| BCVA score | 50 | n.d.. | 55 (+5) | 55 (+5) | 81 (+31) | 65 (+15) | 65 (+15) | stable | stable |

| | | | | | | | | | |
|---|---|---|---|---|---|---|---|---|---|
| N.D. = not determined | | | | | | | | | |

The OCT (optical coherence tomography) measurement of macular retinal thickness for subject A was determined over a period of 16 months post ANXV treatment, and is shown in Fig 5. At 16 months, visual acuity was found to be stable as 20/60, despite having received only a single anti-VEGF treatment about 32 days into the study.

**Conclusions:** The data show the positive effects of ANXV treatment on the disease course accompanied by lower than typical anti-VEGF requirements. Prior to the present study, the established standard for treating RVO involved long-term frequent reinjection of anti-VEGF, with several potential adverse side-effects associated therewith. However, the present study demonstrates that, when ANXV therapy is used then the requirement for anti-VEGF therapy could be markedly reduced. In the instances where additional anti-VEGF treatment was required, the requirement was nevertheless greatly reduced compared to the established standard, generally requiring only a single anti-VEGF injection in conjunction with ANXV therapy over a period of four or more months (e.g. up to 16 months), allowing subjects to reach a clinical endpoint and discharge from further case, without the requirement for multiple and regular anti-VEGF injections.

These data demonstrate the ability of ANXV therapy to reduce the need and/or required frequency of the administration of anti-VEGF agents. We conclude that ANXV can be used in the prophylaxis or the treatment of a disease, disorder or condition associated with VEGF in a subject, and/or the prophylaxis or the treatment of a disease, disorder or condition in a subject that involves one or more administrations of an anti-VEGF agent to the subject. We further conclude that ANXV can be used to enhance anti-VEGF therapies, and/or to reduce complications associated with administration of an anti-VEGF agent.

### Example 3

As discussed above, evidence suggests that several inflammatory cytokines, including IL-6, TNF-α, IL-1β and ICAM-1, can all act to disrupt BRB integrity and contribute to the occurrence of macular oedema and/or retinal vein occlusion (Haydinger *et al,* 2023, *supra;* Park *et al.* 2019, *supra;* Noma *et al.,* 2020, *supra*)*.*

Here we report experiments into the possibility of Annexin A5 to block the effects of such inflammatory cytokines.

**Methods:** Chondrocyte cells were cultured with IL-1β (10 ng/mL) or TNF-α (10 ng/mL) and with/without purified recombinant monomeric human Annexin A5 (ANXA5) (100ng/mL, 5µg/mL, 20 µg/mL). Cells were harvested at 3, 6, 12 and 18 hours. Cell viability was determined with propidium iodide (PI) by flow cytometry.

Expression of IL-6 was measured in IL-1β -stimulated cells. The cells were stimulated with 10ng of IL-1β and treated with different concentration of ANXA5 (0,1 µg, 5 µg and 20 µg/mL) of ANXA5 for 6 hours. Expression of genes were measured by qPCR using SYBR green reaction kit. Gene expression were normalized against GAPDH and relative expression of genes were measured using 2ΔΔCt.

**Results:** The results show that treatment with Annexin A5 reduces cell death in this ex *vivo* study.

**Figure 6** shows that in a human model, cell death promoted by application of TNFα is inhibited through treatment of Annexin A5, with cell viability of the Annexin A5 treated cells being similar to the control without TNFα treatment.

A similar result is seen with IL-1β, as shown in **Figure 7****.** In a dose dependent manner, Annexin A5 is shown to inhibit cell death, with treatment at doses of 5µg and 20µg leading to cell viability comparable to the control without IL-1β treatment. Additionally, **Figure 7** shows that treatment with Annexin A5 protein alone does not affect cell viability, when compared to the control without IL-1β treatment.

As well as inhibiting cell death, treatment with Annexin A5 also reduces key biomarkers.

As shown in **Figure 8****,** treatment with Annexin A5 reduces the expression of ICAM1 when compared to cells in which cell death and inflammation are promoted using TNFα and the control which has not been treated with TNFα.

As shown in **Figure 9****,** treatment with Annexin A5 reduces the expression of IL-6 in a dose dependent manner, when compared to cells induced using IL-1β and the control which has not been treated with IL-1β. Additionally, **Figure 9** shows that treatment with Annexin A5 alone does not have an effect on IL-6 expression.

**Conclusions:** Annexin A5 is demonstrated to inhibit the production and/or effects of several inflammatory cytokines, including IL-6, TNF-α, IL-1β and ICAM-1, which are implicated in the dysregulation of BRB integrity, and which contribute to the occurrence of macular oedema and/or retinal vein occlusion.

It is contemplated that any use, method or composition described herein can be implemented with respect to any other use, method or composition described herein.

The use of the word "a" or "an" when used in conjunction with the term "comprising" in the claims and/or the specification may mean "one," but it is also consistent with the meaning of "one or more," "at least one," and "one or more than one."

Unless stated otherwise, the term "about" as used herein may be used herein to mean a range of ±50%, ±40%, ±30%, ±20%, ±10%, ±5%, ±4%, ±3%, ±2% or ±1% of the value mentioned.

It should be understood that the foregoing description, while indicating various embodiments of the invention and numerous specific details thereof, is given by way of illustration and not of limitation. Many substitutions, modifications, additions and/or rearrangements may be made within the scope of the invention, and the invention includes all such substitutions, modifications, additions and/or rearrangements.

All of the compositions and/or methods disclosed can be made and executed without undue experimentation in light of the present disclosure. While the compositions and methods have been described in terms of preferred embodiments, it will be apparent to those of skill in the art that variations may be applied to the compositions and/or methods and in the steps or in the sequence of steps of the method described herein without departing from the scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the scope and concept of the invention as defined by the appended claims.

## Claims

1. A molecule comprising, or consisting of, Annexin A5 protein for use in the prophylaxis or the treatment of macular oedema, or a condition associated therewith, in a subject, wherein the use is as a combination therapy with an anti-vascular endothelial growth factor (anti-VEGF) agent,
wherein the condition associated with macular oedema is selected from the group consisting of: retinal vascular occlusion (RVO), dysregulation of the blood-retinal barrier (BRB), such as dysregulation of the inner BRB and/or the outer BRB, dysfunction of barriers between the vitreous and blood vessels at the ciliary body, macular edema-associated ocular diseases selected from the group consisting of diabetic retinopathy, uveitis, post-cataract surgery inflammation (pseudophakic macular edema or Irvine-Gass syndrome), retinal dystrophy, drug reactions, intraocular tumors, serous central chorioretinopathy, radiation retinopathy and other retinal vascular abnormalities including retinal arterial macroaneurysms and retinal telangiectasia, changes to subretinal neovascular membranes observed in conditions selected from the group consisting of age-related macular degeneration (AMD), metamorphopsia, micropsia, blurred vision, central scotoma and reduction of contrast or colour sensitivity.

2. The molecule comprising, or consisting of, Annexin A5 protein for use according to Claim 1, wherein:
(a) a dose of the molecule comprising, or consisting of, Annexin A5 protein is administered to the subject systemically, preferably intravenously, or by suprachoroidal injection;
(b) the dose of the molecule comprising, or consisting of, Annexin A5 protein is administered to the subject daily for two consecutive days or more, such as for three, four, five, six, seven or more consecutive days;
(c) the daily dose of the molecule comprising, or consisting of, Annexin A5 protein that is administered to the subject is:
i) a total daily dose of about 5 mg, about 4 mg, about 3 mg, about 2 mg, or less than about 2 mg, such as a total daily dose of 0.1-1.5 mg; or
ii) within a range selected from the group consisting of about 50-100 µg, about 40-80 µg, about 30-60 µg, about 20-40 µg, or less than about 20 µg, such as within the range of 1 µg to 30 µg, per kg body weight of the subject; and/or
(d) the subject has, is suspected of having, or is at risk of having, retinal vein occlusion (RVO).

3. A molecule comprising, or consisting of, Annexin A5 protein for use in the prophylaxis or the treatment of retinal vein occlusion (RVO), or a condition associated therewith, in a subject,
wherein said condition associated with RVO is selected from the group consisting of macular oedema, retinal ischemia, iris neovascularization (optionally, iris neovascularization **characterised by** an increase in cytokines, such as an increase in VEGF), retinal neovascularization (optionally, retinal neovascularization **characterised by** an increase in cytokines, such as an increase in VEGF), neovascular glaucoma, vitreous haemorrhage, rubeosis, and retinal detachment,
wherein a dose of the molecule consisting of monomeric Annexin A5 protein is administered to the subject systemically or by suprachoroidal injection, daily for two or more consecutive days,
and wherein the use is as a combination therapy with an anti-vascular endothelial growth factor (anti-VEGF) agent.

4. The molecule comprising, or consisting of, Annexin A5 protein for use according to Claim 3, wherein:
(a) a dose of the molecule comprising, or consisting of, Annexin A5 protein is administered intravenously to the subject daily for two or more consecutive days;
(b) the dose of the molecule comprising, or consisting of, Annexin A5 protein is administered systemically, preferably intravenously, or by suprachoroidal injection, to the subject daily for three, four, five, six, seven or more consecutive days;
(c) the daily dose of the molecule comprising, or consisting of, Annexin A5 protein that is administered systemically, preferably intravenously, or by suprachoroidal injection, to the subject is:
i) a total daily dose of about 5 mg, about 4 mg, about 3 mg, about 2 mg, or less than about 2 mg, such as a total daily dose of 0.1-1.5 mg; or
ii) within a range selected from the group consisting of about 50-100 µg, about 40-80 µg, about 30-60 µg, about 20-40 µg, or less than about 20 µg, such as within the range of 1 µg to 30 µg, per kg body weight of the subject.

5. The molecule comprising, or consisting of, Annexin A5 protein for use according to Claim 4 or 5,
wherein said use is for the prophylaxis or treatment of a condition in a subject that has, is suspected of having, or is at risk of having, retinal vein occlusion (RVO), and
wherein said condition is selected from the group consisting of macular oedema, retinal ischemia, iris neovascularization (optionally, iris neovascularization **characterised by** an increase in cytokines, such as an increase in VEGF), retinal neovascularization (optionally, retinal neovascularization **characterised by** an increase in cytokines, such as an increase in VEGF), neovascular glaucoma, vitreous haemorrhage, rubeosis, and retinal detachment.

6. The molecule comprising, or consisting of, Annexin A5 protein for use according to any preceding claim, wherein:
(a) the subject is a human adult and/or has a body weight of about 50 kg to about 150 kg; and/or
(b) the RVO is selected from: central retinal vein occlusion (CRVO); branch retinal vein occlusion (BRVO); or hemi-retinal vein occlusion (HRVO).

7. The molecule comprising, or consisting of, Annexin A5 protein for use according to any preceding claim, wherein:
(a) the Annexin A5 protein is monomeric Annexin A5 protein;
(b) the molecule consists of monomeric Annexin A5 protein; and/or
(c) the Annexin A5 protein (optionally a monomeric Annexin A5 protein) is selected from one or more of the following:
i. a protein comprising, consisting essentially of, or consisting of the sequence of human Annexin A5 protein (such as a human monomeric Annexin A5 protein and/or optionally, a protein comprising, or consisting of the sequence of SEQ ID NO:1, either with or without the N-terminal methionine);
ii. a recombinant human Annexin A5 protein, such as a recombinant human monomeric Annexin A5 protein;
iii. a mammalian orthologue of human Annexin A5 protein, such as of human monomeric Annexin A5 protein;
iv. an allelic or genetic variant of i, ii or iii;
v. a functional analogue or variant of a Annexin A5 protein (such as a monomeric Annexin A5 protein) which is a protein which comprises, or consists of, an amino acid sequence that is more than 50%, 60%, 70%, 75%, such as more than 80%, 85%, more than 90%, or even more preferably more than 95% or 99% identical to human Annexin A5 protein (such as a monomeric Annexin A5 protein) having the sequence defined by SEQ ID NO:1 either with, or without, the N-terminal methionine; and
vi. a biologically active fragment of any of i to v.

8. A composition that comprises a molecule comprising, or consisting of, Annexin A5 protein (such as monomeric Annexin A5 protein), for use in accordance with any preceding claim, optionally wherein:
(a) the composition also comprises one or more anti-vascular endothelial growth factor (anti-VEGF) agents,
(b) the composition is a unit dosage composition, for example wherein the unit dosage composition comprises a total dose of the molecule comprising, or consisting of, Annexin A5 protein of about 5 mg, about 4 mg, about 3 mg, about 2 mg, or less than about 2 mg, such as 0.1-1.5 mg;
(c) the composition is a pharmaceutical composition; and/or
(d) the composition further comprises one or more pharmaceutically acceptable carriers, such as wherein the composition consists of the molecule comprising, or consisting of, Annexin A5 protein and one or more pharmaceutically acceptable carriers.

9. The molecule comprising, or consisting of, Annexin A5 protein for use according to any of Claims 1 to 7, or the composition for use according to Claim 8, wherein the use is as a combination therapy with an anti-VEGF agent, and optionally also with another agent, and optionally wherein:
(a) the molecule comprising, or consisting of, Annexin A5 protein is used, with or without the anti-VEGF agent and/or the one or more other agents, as a first line of treatment; or
(b) the molecule comprising, or consisting of, Annexin A5 protein is used, after treatment with the anti-VEGF agent and/or the one or more other agents by at least one administration thereof to the subject has already commenced.

10. The molecule comprising, or consisting of, Annexin A5 protein for use according to any of Claims 1 to 7, or 9, or the composition comprising said molecule for use according to any of Claims 8 or 9, for use as a combination therapy with an anti-VEGF agent, and optionally also with another agent,
wherein the subject is administered the molecule comprising, or consisting of, Annexin A5 protein in the same composition as, separately from, simultaneously with a separate formulation containing, or sequentially with, the anti-VEGF agent, and
optionally wherein the subject is administered the molecule comprising, or consisting of, Annexin A5 protein in the same composition as, separately from, simultaneously with a separate formulation containing, or sequentially with, the also with the one or more other agents.

11. The molecule comprising, or consisting of, Annexin A5 protein for use according to any of Claims 1 to 7, 9 or 10, or the composition for use according to any of Claims 8 to 10, wherein:
(a) the period of time between sequential administrations of the anti-VEGF agent, after the subject has been administered the molecule or composition, is greater than 28 days, for example, greater than a period of time selected from 1, 2, 3, 4, 5, 6, 7, 8, 9, 10, or 11 months, or greater than 1, 1.5, 2, 2.5, 3, 3.5, 4, 4.5, or 5 years, and
optionally, wherein the anti-VEGF therapy is maintained by repeated administration to the subject over a period of about, up to, or at least 2, 3, 4, 5, 6, 7, 8, 9, 10, or 11 months, or about, up to, or at least 1, 1.5, 2, 2.5, 3, 3.5, 4, 4.5, or 5 years;
(b) the anti-VEGF agent is administered to the subject fewer than:
i. 12, 11, 10, 9, 8, 7, 6, 5, 4, 3, 2 or 1 times within a period of twelve months from the start of treatment with the molecule or composition;
ii. 10, 9, 8, 7, 6, 5, 4, 3, 2 or 1 times within a period of ten months from the start of treatment with the molecule or composition;
iii. 8, 7, 6, 5, 4, 3, 2 or 1 times within a period of eight months from the start of treatment with the molecule or composition; and/or
iv. 6, 5, 4, 3, 2 or 1 times within a period of six months from the start of treatment with the molecule or composition,
(c) the frequency of the administration of the anti-VEGF agent is reduced, after the subject has been administered the molecule or composition, compared with a suitable control, or after the subject has been administered the molecule or composition, a subsequent administration of the anti-VEGF agent is delayed (e.g. prevented) compared with a suitable control, for example wherein a suitable control is a subject who has not been treated with a molecule comprising, or consisting of, Annexin A5 protein, for example, wherein:
i. the frequency of the administration of the anti-VEGF agent is reduced by 1, 2, 3, or 4 weeks, or 1, 2, 3, 4, 5, 6, 7, 8, 9, 10, 11, 12 months, or by one or more years compared with a suitable control; and/or
ii. the need for a subsequent administration of the anti-VEGF agent is assessed based on a presence or absence of one or more symptom associated with macular oedema or RVO, or the condition associated therewith, optionally wherein the symptom is selected from the group consisting of one or more of reduced visual acuity, optionally assessed by Best-Corrected Visual Acuity (BCVA); increased macular retinal thickness; increased macular volume; and increased Retinal Area of Non-Perfusion (RANP).

12. A molecule comprising, or consisting of, Annexin A5 protein for use in:
a) the prophylaxis or the treatment of a disease, disorder or condition associated with VEGF that is selected from the group consisting of macular oedema, RVO, a retinopathy associated with a blinding eye disease selected from the group consisting of age-related macular degeneration (AMD), diabetic retinopathy and hypertensive retinopathy in a subject;
b) enhancing an anti-VEGF therapy; and/or
c) reducing complications associated with administration of an anti-VEGF agent, selected from the group consisting of reducing or preventing the subject developing a reduced response, and/or resistance, to ongoing anti-VEGF therapy; reducing or preventing ocular and/or systemic toxicity in anti-VEGF therapy; and/or reducing or preventing one or more of infectious endophthalmitis, sterile intraocular inflammation, retinal vasculitis and/or transient intraocular pressure (IOP) spikes.

13. An anti-VEGF agent for use in the prophylaxis or the treatment of macular oedema, or a condition associated therewith, in a subject, wherein the use is as a combination therapy with a molecule comprising, or consisting of, Annexin A5 protein
wherein the condition associated with macular oedema is selected from the group consisting of: retinal vascular occlusion (RVO), dysregulation of the blood-retinal barrier (BRB), such as dysregulation of the inner BRB and/or the outer BRB, dysfunction of barriers between the vitreous and blood vessels at the ciliary body, macular edema-associated ocular diseases, selected from the group consisting of diabetic retinopathy, uveitis, post-cataract surgery inflammation (pseudophakic macular edema or Irvine-Gass syndrome), retinal dystrophy, drug reactions, intraocular tumors, serous central chorioretinopathy, radiation retinopathy and other retinal vascular abnormalities including retinal arterial macroaneurysms and retinal telangiectasia, changes to subretinal neovascular membranes observed in conditions selected from the group consisting of age-related macular degeneration (AMD), metamorphopsia, micropsia, blurred vision, central scotoma and reduction of contrast or colour sensitivity, optionally, wherein the molecule consists of monomeric Annexin A5, optionally wherein:
(a) the molecule comprising, or consisting of, the Annexin A5 protein or a composition comprising said molecule, is presented in the same composition as the anti-VEGF agent; or
(b) the molecule comprising, or consisting of, the Annexin A5 protein or a composition comprising said molecule is formulated separately from the anti-VEGF agent, for example wherein the use comprises administering the molecule comprising, or consisting of, the Annexin A5 protein and the anti-VEGF agent to the subject simultaneously or sequentially.

14. An anti-VEGF agent for use in the prophylaxis or the treatment of retinal vein occlusion (RVO), or a condition associated therewith, in a subject, wherein the use is as a combination therapy with a molecule comprising, or consisting of, Annexin A5 protein,
wherein said condition associated with RVO is selected from the group consisting of macular oedema, retinal ischemia, iris neovascularization (optionally, iris neovascularization **characterised by** an increase in cytokines, such as an increase in VEGF), retinal neovascularization (optionally, retinal neovascularization **characterised by** an increase in cytokines, such as an increase in VEGF), neovascular glaucoma, vitreous haemorrhage, rubeosis, and retinal detachment, and
wherein a dose of the molecule comprising, or consisting of, Annexin A5 protein is administered to the subject systemically or by suprachoroidal injection, daily for two or more consecutive days, and
optionally, wherein:
(a) the molecule comprising, or consisting of, Annexin A5 protein or a composition comprising said molecule, is presented in the same composition as the anti-VEGF agent; or
(b) the molecule comprising, or consisting of, Annexin A5 protein or a composition comprising said molecule is formulated separately from the anti-VEGF agent, for example wherein the use comprises administering the molecule comprising, or consisting of, the Annexin A5 protein and the anti-VEGF agent to the subject simultaneously or sequentially.

15. The anti-VEGF agent for use according to Claim 13 or 14, wherein the anti-VEGF agent is selected from the group consisting of Ranibizumab, Aflibercept, Bevacizumab, Brolucizumab and Faricimab.

## Patentansprüche

1. Molekül, das das Protein von Annexin A5 umfasst oder daraus besteht, zur Verwendung bei der Prophylaxe oder Behandlung eines Makulaödems oder eines damit verbundenen Zustands bei einem Probanden, wobei die Verwendung als Kombinationstherapie mit einem anti-vaskulären endothelialen Wachstumsfaktor (Anti-VEGF)-Wirkstoff erfolgt,
wobei der mit einem Makulaödem verbundene Zustand aus der Gruppe ausgewählt ist, bestehend aus: retinaler vaskulärer Okklusion (RVO), Dysregulation der Blut-Retina-Schranke (BRB), wie einer Dysregulation der inneren BRB und/oder der äußeren BRB, Funktionsstörungen der Schranken zwischen Glaskörper und Blutgefäßen am Ziliarkörper, mit Makulaödem verbundenen Augenerkrankungen, ausgewählt aus der Gruppe, bestehend aus diabetischer Retinopathie, Uveitis, Entzündung nach Kataraktoperation (pseudophakes Makulaödem oder Irvine-Gass-Syndrom), Retinadystrophie, Arzneimittelreaktionen, intraokularen Tumoren, zentraler seröser Chorioretinopathie, Strahlenretinopathie und anderen retinalen vaskulären Anomalien, einschließlich retinaler arterieller Makroaneurysmen und retinaler Teleangiektasien, Veränderungen subretinaler neovaskulärer Membranen, die bei Zuständen beobachtet werden, die aus der Gruppe ausgewählt sind, bestehend aus altersbedingter Makuladegeneration (AMD), Metamorphopsie, Mikropsie, verschwommenem Sehen, zentralem Skotom und Verminderung der Kontrast- oder Farbsensitivität.

2. Molekül, das das Protein von Annexin A5 umfasst oder daraus besteht, zur Verwendung nach Anspruch 1, wobei:
(a) eine Dosis des Moleküls, das das Protein von Annexin A5 umfasst oder daraus besteht, dem Probanden systemisch, vorzugsweise intravenös, oder durch suprachoroidale Injektion verabreicht wird;
(b) die Dosis des Moleküls, das das Protein von Annexin A5 umfasst oder daraus besteht, dem Probanden täglich über zwei aufeinanderfolgende Tage oder länger verabreicht wird, wie etwa über drei, vier, fünf, sechs, sieben oder mehr aufeinanderfolgende Tage;
(c) die dem Probanden verabreichte tägliche Dosis des Moleküls, das das Protein von Annexin A5 umfasst oder daraus besteht:
i) eine tägliche Gesamtdosis von etwa 5 mg, etwa 4 mg, etwa 3 mg, etwa 2 mg oder weniger als etwa 2 mg, wie etwa eine tägliche Gesamtdosis von 0,1 bis 1,5 mg ist; oder
ii) innerhalb eines Bereichs, der aus der Gruppe ausgewählt ist, bestehend aus etwa 50 bis 100 µg, etwa 40 bis 80 µg, etwa 30 bis 60 µg, etwa 20 bis 40 µg oder weniger als etwa 20 µg, beispielsweise innerhalb des Bereichs von 1 µg bis 30 µg pro kg Körpergewicht des Probanden, liegt; und/oder
(d) der Proband an einer retinalen Venenokklusion (RVO) leidet, unter Verdacht steht, daran zu leiden, oder einem entsprechenden Risiko ausgesetzt ist.

3. Molekül, das das Protein von Annexin A5 umfasst oder daraus besteht, zur Verwendung bei der Prophylaxe oder Behandlung einer retinalen Venenokklusion (RVO) oder eines damit verbundenen Zustands bei einem Probanden,
wobei dieser mit einer RVO verbundene Zustand aus der Gruppe ausgewählt ist, bestehend aus Makulaödem, Retinaischämie, Irisneovaskularisation (optional einer Irisneovaskularisation **gekennzeichnet durch** einen Anstieg von Zytokinen, wie etwa einen Anstieg von VEGF), retinaler Neovaskularisation (optional einer retinalen Neovaskularisation **gekennzeichnet durch** einen Anstieg von Zytokinen, wie etwa einen Anstieg von VEGF), neovaskulärem Glaukom, Glaskörperblutung, Rubeose und Retinaablösung,
wobei eine Dosis des Moleküls, das aus dem monomeren Protein von Annexin A5 besteht, dem Probanden täglich über zwei oder mehr aufeinanderfolgende Tage systemisch oder durch suprachoroidale Injektion verabreicht wird,
und wobei die Verwendung als Kombinationstherapie mit einem anti-vaskulären endothelialen Wachstumsfaktor (Anti-VEGF)-Wirkstoff erfolgt.

4. Molekül, das das Protein von Annexin A5 umfasst oder daraus besteht, zur Verwendung nach Anspruch 3, wobei:
(a) eine Dosis des Moleküls, das das Protein von Annexin A5 umfasst oder daraus besteht, dem Probanden täglich über zwei oder mehr aufeinanderfolgende Tage intravenös verabreicht wird;
(b) die Dosis des Moleküls, das das Protein von Annexin A5 umfasst oder daraus besteht, dem Probanden täglich über drei, vier, fünf, sechs, sieben oder mehr aufeinanderfolgende Tage systemisch, vorzugsweise intravenös, oder durch suprachoroidale Injektion verabreicht wird;
(c) die dem Probanden systemisch, vorzugsweise intravenös, oder durch suprachoroidale Injektion verabreichte tägliche Dosis des Moleküls, das das Protein von Annexin A5 umfasst oder daraus besteht:
i) eine tägliche Gesamtdosis von etwa 5 mg, etwa 4 mg, etwa 3 mg, etwa 2 mg oder weniger als etwa 2 mg, wie etwa eine tägliche Gesamtdosis von 0,1 bis 1,5 mg ist; oder
ii) innerhalb eines Bereichs, der aus der Gruppe ausgewählt ist, bestehend aus etwa 50 bis 100 µg, etwa 40 bis 80 µg, etwa 30 bis 60 µg, etwa 20 bis 40 µg oder weniger als etwa 20 µg, beispielsweise innerhalb des Bereichs von 1 µg bis 30 µg pro kg Körpergewicht des Probanden, liegt.

5. Molekül, das das Protein von Annexin A5 umfasst oder daraus besteht, zur Verwendung nach Anspruch 4 oder 5,
wobei diese Verwendung der Prophylaxe oder Behandlung eines Zustands bei einem Probanden dient, der an einer retinalen Venenokklusion (RVO) leidet, unter Verdacht steht, daran zu leiden, oder einem entsprechenden Risiko ausgesetzt ist, und
wobei dieser Zustand aus der Gruppe ausgewählt ist, bestehend aus Makulaödem, Retinaischämie, Irisneovaskularisation (optional einer Irisneovaskularisation **gekennzeichnet durch** einen Anstieg von Zytokinen, wie etwa einen Anstieg von VEGF), retinaler Neovaskularisation (optional einer retinalen Neovaskularisation **gekennzeichnet durch** einen Anstieg von Zytokinen, wie etwa einen Anstieg von VEGF), neovaskulärem Glaukom, Glaskörperblutung, Rubeose und Retinaablösung.

6. Molekül, das das Protein von Annexin A5 umfasst oder daraus besteht, zur Verwendung nach einem der vorstehenden Ansprüche, wobei:
(a) der Proband ein erwachsener Mensch und/oder ein Körpergewicht von etwa 50 kg bis etwa 150 kg hat; und/oder
(b) die RVO ausgewählt ist aus: zentraler retinaler Venenokklusion (CRVO); verzweigter retinaler Venenokklusion (BRVO); oder hemi-retinaler Venenokklusion (HRVO).

7. Molekül, das das Protein von Annexin A5 umfasst oder daraus besteht, zur Verwendung nach einem der vorstehenden Ansprüche, wobei:
(a) das Protein von Annexin A5 ein monomeres Protein von Annexin A5 ist;
(b) das Molekül aus monomerem Protein von Annexin A5 besteht; und/oder
(c) das Protein von Annexin A5 (optional ein monomeres Protein von Annexin A5) aus einem oder mehreren der Folgenden ausgewählt ist:
i. einem Protein, das die Sequenz des humanen Proteins von Annexin A5 (wie etwa eines humanen monomeren Proteins von Annexin A5 und/oder optional eines Proteins, das die Sequenz von SEQ ID NO:1 umfasst oder aus dieser besteht, entweder mit oder ohne das N-terminale Methionin) umfasst, aus dieser im Wesentlichen besteht oder aus dieser besteht;
ii. einem rekombinanten humanen Proteins von Annexin A5, wie etwa einem rekombinanten humanen monomeren Protein von Annexin A5;
iii. einem Säugetier-Ortholog des humanen Proteins von Annexin A5, wie etwa des humanen monomeren Proteins von Annexin A5;
iv. einer Allel- oder Genvariante von i, ii oder iii;
v. einem funktionellen Analogon oder einer funktionellen Variante eines Proteins von Annexin A5 (wie etwa eines monomeren Proteins von Annexin A5), das ein Protein ist, das eine Aminosäuresequenz umfasst oder aus dieser besteht, die zu mehr als 50 %, 60 %, 70 %, 75 %, wie etwa mehr als 80 %, 85 %, mehr als 90 % oder noch mehr bevorzugter mehr als 95 % oder 99 % mit dem humanen Protein von Annexin A5 (wie etwa einem monomeren Protein von Annexin A5) identisch ist, das die durch SEQ ID NO:1 definierte Sequenz aufweist, entweder mit oder ohne das N-terminale Methionin; und
vi. einem biologisch aktiven Fragment eines der Punkte i bis v.

8. Zusammensetzung, die ein Molekül umfasst, das das Protein von Annexin A5 (wie etwa monomeres Protein von Annexin A5) umfasst oder daraus besteht, zur Verwendung nach einem der vorstehenden Ansprüche, wobei optional:
(a) die Zusammensetzung außerdem einen oder mehrere anti-vaskuläre endotheliale Wachstumsfaktor (Anti-VEGF)-Wirkstoffe umfasst,
(b) die Zusammensetzung eine Einzeldosis-Zusammensetzung ist, wobei beispielsweise die Einzeldosis-Zusammensetzung eine Gesamtdosis des Moleküls, das das Protein von Annexin A5 umfasst oder daraus besteht, von etwa 5 mg, etwa 4 mg, etwa 3 mg, etwa 2 mg oder weniger als etwa 2 mg, beispielsweise 0,1 bis 1,5 mg, umfasst;
(c) die Zusammensetzung eine pharmazeutische Zusammensetzung ist; und/oder
(d) die Zusammensetzung ferner einen oder mehrere pharmazeutisch verträgliche Träger umfasst, wobei beispielsweise die Zusammensetzung aus dem Molekül, das das Protein von Annexin A5 umfasst oder daraus besteht, und einem oder mehreren pharmazeutisch verträglichen Trägern besteht.

9. Molekül, das das Protein von Annexin A5 umfasst oder daraus besteht, zur Verwendung nach einem der Ansprüche 1 bis 7, oder die Zusammensetzung zur Verwendung nach Anspruch 8, wobei die Verwendung als Kombinationstherapie mit einem Anti-VEGF-Wirkstoff und optional auch mit einem weiteren Wirkstoff erfolgt, und wobei optional:
(a) das Molekül, das das Protein von Annexin A5 umfasst oder daraus besteht, mit oder ohne den Anti-VEGF-Wirkstoff und/oder den einen oder die mehreren anderen Wirkstoffe als Erstlinienbehandlung verwendet wird; oder
(b) das Molekül, das das Protein von Annexin A5 umfasst oder daraus besteht, verwendet wird, nachdem die Behandlung mit dem Anti-VEGF-Wirkstoff und/oder dem einen oder den mehreren anderen Wirkstoffen durch mindestens eine Verabreichung an den Probanden bereits begonnen hat.

10. Molekül, das das Protein von Annexin A5 umfasst oder daraus besteht, zur Verwendung nach einem der Ansprüche 1 bis 7 oder 9, oder die Zusammensetzung, die dieses Molekül umfasst, zur Verwendung nach einem der Ansprüche 8 oder 9, zur Verwendung als Kombinationstherapie mit einem Anti-VEGF-Wirkstoff und optional auch mit einem weiteren Wirkstoff,
wobei dem Probanden das Molekül, das das Protein von Annexin A5 umfasst oder daraus besteht, in derselben Zusammensetzung wie, getrennt von, gleichzeitig mit einer separaten Formulierung, die den Anti-VEGF-Wirkstoff enthält, oder nacheinander mit diesem verabreicht wird, und
wobei optional dem Probanden das Molekül, das das Protein von Annexin A5 umfasst oder daraus besteht, in derselben Zusammensetzung wie, getrennt von, gleichzeitig mit einer separaten Formulierung, die den einen oder die mehreren anderen Wirkstoffe enthält, oder nacheinander mit diesen verabreicht wird.

11. Molekül, das das Protein von Annexin A5 umfasst oder daraus besteht, zur Verwendung nach einem der Ansprüche 1 bis 7, 9 oder 10, oder die Zusammensetzung zur Verwendung nach einem der Ansprüche 8 bis 10, wobei:
(a) der Zeitraum zwischen aufeinanderfolgenden Verabreichungen des Anti-VEGF-Wirkstoffs, nachdem dem Probanden das Molekül oder die Zusammensetzung verabreicht wurde, mehr als 28 Tage beträgt, beispielsweise mehr als ein Zeitraum, der aus 1, 2, 3, 4, 5, 6, 7, 8, 9, 10 oder 11 Monaten ausgewählt wird, oder mehr als 1, 1,5, 2, 2,5, 3, 3,5, 4, 4,5 oder 5 Jahre, und
wobei optional die Anti-VEGF-Therapie durch wiederholte Verabreichung an den Probanden über einen Zeitraum von etwa, bis zu oder mindestens 2, 3, 4, 5, 6, 7, 8, 9, 10 oder 11 Monaten oder etwa, bis zu oder mindestens 1, 1,5, 2, 2,5, 3, 3,5, 4, 4,5 oder 5 Jahren aufrechterhalten wird;
(b) dem Probanden der Anti-VEGF-Wirkstoff verabreicht wird weniger als:
i. 12, 11, 10, 9, 8, 7, 6, 5, 4, 3, 2 oder 1 Mal innerhalb eines Zeitraums von zwölf Monaten ab Beginn der Behandlung mit dem Molekül oder der Zusammensetzung;
ii. 10, 9, 8, 7, 6, 5, 4, 3, 2 oder 1 Mal innerhalb eines Zeitraums von zehn Monaten ab Beginn der Behandlung mit dem Molekül oder der Zusammensetzung;
iii. 8, 7, 6, 5, 4, 3, 2 oder 1 Mal innerhalb eines Zeitraums von acht Monaten ab Beginn der Behandlung mit dem Molekül oder der Zusammensetzung; und/oder
iv. 6, 5, 4, 3, 2 oder 1 Mal innerhalb eines Zeitraums von sechs Monaten ab Beginn der Behandlung mit dem Molekül oder der Zusammensetzung,
(c) die Häufigkeit der Verabreichung des Anti-VEGF-Wirkstoffs nach der Verabreichung des Moleküls oder der Zusammensetzung an den Probanden im Vergleich zu einer geeigneten Kontrollgruppe verringert wird oder eine nachfolgende Verabreichung des Anti-VEGF-Wirkstoffs nach der Verabreichung des Moleküls oder der Zusammensetzung an den Probanden im Vergleich zu einer geeigneten Kontrollgruppe verzögert (z. B. verhindert) wird, wobei beispielsweise eine geeignete Kontrollgruppe ein Proband ist, der nicht mit einem Molekül behandelt wurde, das das Protein von Annexin A5 umfasst oder daraus besteht, wobei beispielsweise:
i. die Häufigkeit der Verabreichung des Anti-VEGF-Wirkstoffs im Vergleich zu einer geeigneten Kontrollgruppe um 1, 2, 3 oder 4 Wochen oder um 1, 2, 3, 4, 5, 6, 7, 8, 9, 10, 11, 12 Monate oder um ein oder mehrere Jahre reduziert ist; und/oder
ii. die Notwendigkeit einer nachfolgenden Verabreichung des Anti-VEGF-Wirkstoffs anhand des Vorhandenseins oder Fehlens eines oder mehrerer Symptome im Zusammenhang mit einem Makulaödem oder einer RVO oder eines damit verbundenen Zustands beurteilt wird, wobei optional das Symptom aus der Gruppe ausgewählt wird, bestehend aus einer oder mehreren der verminderten Sehschärfe, optional beurteilt anhand der bestkorrigierten Sehschärfe (BCVA); der erhöhten makulären Retinadicke; des erhöhten Makulavolumens; und des vergrößerten retinalen Nichtperfusionsbereichs (RANP).

12. Molekül, das das Protein von Annexin A5 umfasst oder daraus besteht, zur Verwendung bei:
a) der Prophylaxe oder Behandlung einer mit VEGF verbundenen Erkrankung, Störung oder eines Zustands, ausgewählt aus der Gruppe, bestehend aus Makulaödem, RVO, einer Retinopathie im Zusammenhang mit einer Blindaugenerkrankung, ausgewählt aus der Gruppe, bestehend aus altersbedingter Makuladegeneration (AMD), diabetischer Retinopathie und hypertensiver Retinopathie, bei einem Probanden;
b) der Verstärkung einer Anti-VEGF-Therapie; und/oder
c) der Verringerung von Komplikationen im Zusammenhang mit der Verabreichung eines Anti-VEGF-Wirkstoffs, ausgewählt aus der Gruppe, bestehend aus der Verringerung oder Verhinderung einer verminderten Reaktion und/oder Resistenz des Probanden gegenüber einer laufenden Anti-VEGF-Therapie; der Verringerung oder Verhinderung von okulärer und/oder systemischer Toxizität bei der Anti-VEGF-Therapie; und/oder der Verringerung oder Verhinderung einer oder mehrerer der infektiösen Endophthalmitis, sterilen intraokularen Entzündung, retinalen Vaskulitis und/oder vorübergehenden intraokularen Druckspitzen (IOD).

13. Anti-VEGF-Wirkstoff zur Verwendung bei der Prophylaxe oder Behandlung eines Makulaödems oder eines damit verbundenen Zustands bei einem Probanden, wobei die Verwendung als Kombinationstherapie mit einem Molekül erfolgt, das das Protein von Annexin A5 umfasst oder daraus besteht
wobei der mit einem Makulaödem verbundene Zustand aus der Gruppe ausgewählt ist, bestehend aus: retinaler vaskulärer Okklusion (RVO), Dysregulation der Blut-Retina-Schranke (BRB), wie einer Dysregulation der inneren BRB und/oder der äußeren BRB, Funktionsstörungen der Schranken zwischen Glaskörper und Blutgefäßen am Ziliarkörper, mit Makulaödem verbundenen Augenerkrankungen, ausgewählt aus der Gruppe, bestehend aus diabetischer Retinopathie, Uveitis, Entzündung nach Kataraktoperation (pseudophakes Makulaödem oder Irvine-Gass-Syndrom), Retinadystrophie, Arzneimittelreaktionen, intraokularen Tumoren, zentraler seröser Chorioretinopathie, Strahlenretinopathie und anderen retinalen vaskulären Anomalien, einschließlich retinaler arterieller Makroaneurysmen und retinaler Teleangiektasien, Veränderungen subretinaler neovaskulärer Membranen, die bei Zuständen beobachtet werden, die aus der Gruppe ausgewählt sind, bestehend aus altersbedingter Makuladegeneration (AMD), Metamorphopsie, Mikropsie, verschwommenem Sehen, zentralem Skotom und Verminderung der Kontrast- oder Farbsensitivität, wobei optional das Molekül aus monomerem Annexin A5 besteht, wobei optional:
(a) das Molekül, das das Protein von Annexin A5 umfasst oder daraus besteht, oder eine Zusammensetzung, die dieses Molekül umfasst, in derselben Zusammensetzung wie der Anti-VEGF-Wirkstoff vorliegt; oder
(b) das Molekül, das das Protein von Annexin A5 umfasst oder daraus besteht, oder eine Zusammensetzung, die dieses Molekül umfasst, getrennt von dem Anti-VEGF-Wirkstoff formuliert ist, wobei beispielsweise die Verwendung die gleichzeitige oder aufeinanderfolgende Verabreichung des Moleküls, das das Protein von Annexin A5 umfasst oder daraus besteht, und des Anti-VEGF-Wirkstoffs an den Probanden umfasst.

14. Anti-VEGF-Wirkstoff zur Verwendung bei der Prophylaxe oder Behandlung einer retinalen Venenokklusion (RVO) oder eines damit verbundenen Zustands bei einem Probanden, wobei die Verwendung als Kombinationstherapie mit einem Molekül erfolgt, das das Protein von Annexin A5 umfasst oder daraus besteht,
wobei dieser mit einer RVO verbundene Zustand aus der Gruppe ausgewählt ist, bestehend aus Makulaödem, Retinaischämie, Irisneovaskularisation (optional einer Irisneovaskularisation **gekennzeichnet durch** einen Anstieg von Zytokinen, wie etwa einen Anstieg von VEGF), retinaler Neovaskularisation (optional einer retinalen Neovaskularisation **gekennzeichnet durch** einen Anstieg von Zytokinen, wie etwa einen Anstieg von VEGF), neovaskulärem Glaukom, Glaskörperblutung, Rubeose und Retinaablösung, und
wobei eine Dosis des Moleküls, das das Protein von Annexin A5 umfasst oder daraus besteht, dem Probanden täglich über zwei oder mehr aufeinanderfolgende Tage systemisch oder durch suprachoroidale Injektion verabreicht wird, und
wobei optional:
(a) das Molekül, das das Protein von Annexin A5 umfasst oder daraus besteht, oder eine Zusammensetzung, die dieses Molekül umfasst, in derselben Zusammensetzung wie der Anti-VEGF-Wirkstoff vorliegt; oder
(b) das Molekül, das das Protein von Annexin A5 umfasst oder daraus besteht, oder eine Zusammensetzung, die dieses Molekül umfasst, getrennt von dem Anti-VEGF-Wirkstoff formuliert ist, wobei beispielsweise die Verwendung die gleichzeitige oder aufeinanderfolgende Verabreichung des Moleküls, das das Protein von Annexin A5 umfasst oder daraus besteht, und des Anti-VEGF-Wirkstoffs an den Probanden umfasst.

15. Anti-VEGF-Wirkstoff zur Verwendung nach Anspruch 13 oder 14, wobei der Anti-VEGF-Wirkstoff aus der Gruppe ausgewählt ist, bestehend aus Ranibizumab, Aflibercept, Bevacizumab, Brolucizumab und Faricimab.

## Revendications

1. Molécule comprenant, ou constituée de, la protéine Annexine A5, destinée à être utilisée dans la prophylaxie ou le traitement de l'œdème maculaire, ou d'une affection associée à celui-ci, chez un sujet, dans laquelle l'utilisation est sous forme d'une thérapie combinée avec un agent anti-facteur de croissance endothélial vasculaire (anti-VEGF),
dans laquelle l'affection associée à l'œdème maculaire est choisie dans le groupe constitué : de l'occlusion de la veine rétinienne (OVR), de la dysrégulation de la barrière hémato-rétinienne (BHR), notamment de la BHR interne et/ou externe, du dysfonctionnement des barrières entre le corps vitré et les vaisseaux sanguins au niveau du corps ciliaire, des maladies oculaires associées à l'œdème maculaire (rétinopathie diabétique, uvéite, inflammation post-opératoire de la cataracte [œdème maculaire pseudophaque ou syndrome d'Irvine-Gass], de la dystrophie rétinienne, des réactions médicamenteuses, des tumeurs intraoculaires, de la choriorétinopathie séreuse centrale, de la rétinopathie radique et d'autres anomalies vasculaires rétiniennes, y compris les macroanévrismes artériels rétiniens et les télangiectasies rétiniennes), des modifications des membranes néovasculaires sous-rétiniennes observées dans des conditions choisies dans le groupe constitué de la dégénérescence maculaire liée à l'âge (DMLA), de la métamorphopsie, de la micropsie, de la vision floue, du scotome central et de la diminution de la sensibilité aux contrastes ou aux couleurs.

2. Molécule comprenant, ou constituée de, la protéine Annexine A5 destinée à être utilisée selon la revendication 1, dans laquelle :
(a) une dose de la molécule comprenant, ou constituée de, la protéine Annexine A5 est administrée au sujet par voie systémique, de préférence par voie intraveineuse, ou par injection suprachoroïdienne ;
(b) la dose de la molécule comprenant, ou constituée de, la protéine Annexine A5 est administrée au sujet quotidiennement pendant deux jours consécutifs ou plus, par exemple pendant trois, quatre, cinq, six, sept jours consécutifs ou plus ;
(c) la dose journalière de la molécule comprenant, ou constituée de, la protéine Annexine A5 administrée au sujet est :
i) une dose quotidienne totale d'environ 5 mg, d'environ 4 mg, d'environ 3 mg, d'environ 2 mg ou moins de 2 mg, par exemple une dose quotidienne totale de 0,1 à 1,5 mg ; ou
ii) dans une plage choisie dans le groupe constitué d'environ 50 à 100 µg, d'environ 40 à 80 µg, d'environ 30 à 60 µg, d'environ 20 à 40 µg, ou moins d'environ 20 µg, par exemple dans la plage de 1 µg à 30 µg, par kg de poids corporel du sujet ; et/ou
(d) le sujet présente, est suspecté de présenter ou risque de présenter une occlusion de la veine rétinienne (OVR).

3. Molécule comprenant, ou constituée de, la protéine Annexine A5, destinée à être utilisée dans la prophylaxie ou le traitement de l'occlusion de la veine rétinienne (OVR), ou d'une affection associée à celle-ci, chez un sujet,
dans laquelle ladite affection associée à l'OVR est choisie dans le groupe constitué de l'œdème maculaire, de l'ischémie rétinienne, de la néovascularisation irienne (éventuellement **caractérisée par** une augmentation des cytokines, comme une augmentation du VEGF), de la néovascularisation rétinienne (éventuellement **caractérisée par** une augmentation des cytokines, comme une augmentation du VEGF), du glaucome néovasculaire, de l'hémorragie vitréenne, de la rubéose et du décollement de la rétine,
dans laquelle une dose de la molécule constituée de la protéine Annexine A5 monomérique est administrée au sujet par voie systémique ou par injection suprachoroïdienne, quotidiennement pendant deux jours consécutifs ou plus,
et dans laquelle l'utilisation est sous forme d'une thérapie combinée avec un agent anti-facteur de croissance endothélial vasculaire (anti-VEGF).

4. Molécule comprenant, ou constituée de, la protéine Annexine A5 destinée à être utilisée selon la revendication 3, dans laquelle :
(a) une dose de la molécule comprenant, ou constituée de, la protéine Annexine A5 est administrée par voie intraveineuse au sujet quotidiennement pendant deux jours consécutifs ou plus ;
(b) la dose de la molécule comprenant, ou constituée de, la protéine Annexine A5 est administrée par voie systémique, de préférence par voie intraveineuse ou par injection suprachoroïdienne, au sujet quotidiennement pendant trois, quatre, cinq, six, sept jours consécutifs ou plus ;
(c) la dose quotidienne de la molécule comprenant, ou constituée de, la protéine Annexine A5 administrée par voie systémique, de préférence par voie intraveineuse, ou par injection suprachoroïdienne, au sujet est :
i) une dose quotidienne totale d'environ 5 mg, d'environ 4 mg, d'environ 3 mg, d'environ 2 mg ou moins de 2 mg, par exemple une dose quotidienne totale de 0,1 à 1,5 mg ; ou
ii) dans une plage choisie dans le groupe constitué d'environ 50 à 100 µg, d'environ 40 à 80 µg, d'environ 30 à 60 µg, d'environ 20 à 40 µg, ou moins d'environ 20 µg, par exemple dans la plage de 1 µg à 30 µg, par kg de poids corporel du sujet.

5. Molécule comprenant, ou constituée de, la protéine Annexine A5 destinée à être utilisée selon la revendication 4 ou 5,
dans laquelle ladite utilisation est destinée à la prophylaxie ou au traitement d'une affection chez un sujet atteint, suspecté d'être atteint ou à risque d'être atteint d'une occlusion de la veine rétinienne (OVR), et
dans laquelle ladite affection est choisie dans le groupe constitué de l'œdème maculaire, de l'ischémie rétinienne, de la néovascularisation de l'iris (éventuellement, la néovascularisation de l'iris **caractérisée par** une augmentation des cytokines, telle qu'une augmentation du VEGF), de la néovascularisation rétinienne (éventuellement, la néovascularisation rétinienne **caractérisée par** une augmentation des cytokines, telle qu'une augmentation du VEGF), du glaucome néovasculaire, de l'hémorragie vitréenne, de la rubéose et du décollement de la rétine.

6. Molécule comprenant, ou constituée de, la protéine Annexine A5 destinée à être utilisée selon une quelconque revendication précédente, dans laquelle :
(a) le sujet est un adulte humain et/ou a un poids corporel d'environ 50 kg à environ 150 kg ; et/ou
(b) l'OVR est choisie parmi : l'occlusion de la veine centrale de la rétine (OVCR) ; l'occlusion de branche de la veine rétinienne (OBVR) ; ou l'occlusion de l'hémi-veine rétinienne (OHVR).

7. Molécule comprenant, ou constituée de, la protéine Annexine A5 destinée à être utilisée selon une quelconque revendication précédente, dans laquelle :
(a) la protéine Annexine A5 est une protéine Annexine A5 monomère ;
(b) la molécule est constituée de protéine Annexine A5 monomère ; et/ou
(c) la protéine Annexine A5 (éventuellement une protéine Annexine A5 monomère) est choisie parmi l'un ou plusieurs :
i. d'une protéine comprenant, constituée essentiellement de, ou constituée de la séquence de la protéine Annexine A5 humaine (telle qu'une protéine Annexine A5 monomère humaine et/ou éventuellement, une protéine comprenant, ou constituée de la séquence de SEQ ID NO:1, avec ou sans la méthionine N-terminale) ;
ii. d'une protéine Annexine A5 humaine recombinante, telle qu'une protéine Annexine A5 monomère humaine recombinante ;
iii. d'un orthologue mammifère de la protéine Annexine A5 humaine, tel que la protéine Annexine A5 monomère humaine ;
iv. d'une variante allélique ou génétique de i, ii ou iii ;
v. d'un analogue fonctionnel ou d'une variante d'une protéine Annexine A5 (telle qu'une protéine Annexine A5 monomère) qui est une protéine comprenant, ou constituée d'une séquence d'acides aminés identique à plus de 50 %, 60 %, 70 %, 75 %, par exemple à plus de 80 %, 85 %, 90 %, ou de manière davantage préférée à plus de 95 % ou 99 %, à la protéine Annexine A5 humaine (telle qu'une protéine Annexine A5 monomérique) ayant la séquence définie par SEQ ID NO:1, avec ou sans la méthionine N-terminale ; et
vi. d'un fragment biologiquement actif de l'un quelconque de i à v.

8. Composition qui comprend une molécule comprenant, ou constituée de, la protéine Annexine A5 (telle que la protéine Annexine A5 monomère), destinée à être utilisée selon une quelconque revendication précédente, éventuellement dans laquelle :
(a) la composition comprend également un ou plusieurs agents anti-facteur de croissance endothélial vasculaire (anti-VEGF),
(b) la composition est une composition à dose unitaire, par exemple dans laquelle la composition à dose unitaire comprend une dose totale de la molécule comprenant, ou constituée de, la protéine Annexine A5 d'environ 5 mg, d'environ 4 mg, d'environ 3 mg, d'environ 2 mg ou moins d'environ 2 mg, telle que 0,1 à 1,5 mg ;
(c) la composition est une composition pharmaceutique ; et/ou
(d) la composition comprend en outre un ou plusieurs supports pharmaceutiquement acceptables, par exemple dans laquelle la composition est constituée de la molécule comprenant, ou constituée de, la protéine Annexine A5 et d'un ou de plusieurs supports pharmaceutiquement acceptables.

9. Molécule comprenant, ou constituée de, la protéine Annexine A5, destinée à être utilisée selon l'une quelconque des revendications 1 à 7, ou composition destinée à être utilisée selon la revendication 8, dans laquelle l'utilisation est sous forme d'une thérapie combinée avec un agent anti-VEGF, et éventuellement avec un autre agent, et éventuellement dans laquelle :
(a) la molécule comprenant, ou constituée de, la protéine Annexine A5 est utilisée, avec ou sans l'agent anti-VEGF et/ou les un ou plusieurs autres agents, en première intention ; ou
(b) la molécule comprenant, ou constituée de, la protéine Annexine A5 est utilisée, après que le traitement par l'agent anti-VEGF et/ou les un ou plusieurs autres agents, par au moins une administration de celui-ci au sujet, a déjà commencé.

10. Molécule comprenant, ou constituée de, la protéine Annexine A5, destinée à être utilisée selon l'une quelconque des revendications 1 à 7 ou 9, ou composition comprenant ladite molécule destinée à être utilisée selon l'une quelconque des revendications 8 ou 9, destinée à être utilisée sous forme d'une thérapie combinée avec un agent anti-VEGF, et éventuellement également avec un autre agent,
dans laquelle le sujet se fait administrer la molécule comprenant, ou constituée de, la protéine Annexine A5 dans la même composition que, séparément de, simultanément avec, ou séquentiellement avec, une formulation distincte contenant l'agent anti-VEGF, et
éventuellement dans laquelle le sujet se fait administrer la molécule comprenant, ou constituée de, la protéine Annexine A5 dans la même composition que, séparément de, simultanément avec une formulation distincte contenant, ou séquentiellement avec, également avec les un ou plusieurs autres agents.

11. Molécule comprenant, ou constituée de, la protéine Annexine A5 destinée à être utilisée selon l'une quelconque des revendications 1 à 7, 9 ou 10, ou composition destinée à être utilisée selon l'une quelconque des revendications 8 à 10, dans laquelle :
(a) l'intervalle de temps entre deux administrations successives de l'agent anti-VEGF, après administration de la molécule ou de la composition au sujet, est supérieur à 28 jours, par exemple, supérieur à une période de temps choisie parmi 1, 2, 3, 4, 5, 6, 7, 8, 9, 10 ou 11 mois, ou supérieur à 1, 1,5, 2, 2,5, 3, 3,5, 4, 4,5 ou 5 ans, et
éventuellement dans laquelle le traitement anti-VEGF est maintenu par administration répétée au sujet sur une période d'environ, jusqu'à, ou au moins 2, 3, 4, 5, 6, 7, 8, 9, 10 ou 11 mois, ou d'environ, jusqu'à, ou au moins 1, 1,5, 2, 2,5, 3, 3,5, 4, 4,5 ou 5 ans ;
(b) l'agent anti-VEGF est administré au sujet moins de :
i. 12, 11, 10, 9, 8, 7, 6, 5, 4, 3, 2 ou 1 fois surune période de douze mois à compter du début du traitement avec la molécule ou la composition ;
ii. 10, 9, 8, 7, 6, 5, 4, 3, 2 ou 1 fois sur une période de dix mois à compter du début du traitement avec la molécule ou la composition ;
iii. 8, 7, 6, 5, 4, 3, 2 ou 1 fois sur une période de huit mois à compter du début du traitement par la molécule ou la composition ; et/ou
iv. 6, 5, 4, 3, 2 ou 1 fois sur une période de six mois à compter du début du traitement par la molécule ou la composition,
(c) la fréquence d'administration de l'agent anti-VEGF est réduite, après administration de la molécule ou de la composition au sujet, par rapport à un témoin approprié, ou après administration de la molécule ou de la composition au sujet, une administration ultérieure de l'agent anti-VEGF est retardée (par exemple, empêchée) par rapport à un témoin approprié, par exemple lorsque le témoin approprié est un sujet qui n'a pas été traité avec une molécule comprenant ou constituée de la protéine Annexine A5, par exemple, lorsque :
i. la fréquence d'administration de l'agent anti-VEGF est réduite de 1, 2, 3 ou 4 semaines, ou de 1, 2, 3, 4, 5, 6, 7, 8, 9, 10, 11, 12 mois, ou d'un an ou plus par rapport à un témoin approprié ; et/ou
ii. la nécessité d'une administration ultérieure de l'agent anti-VEGF est évaluée sur la base de la présence ou de l'absence d'un ou de plusieurs symptômes associés à un œdème maculaire ou à une occlusion de la veine rétinienne (OVR), ou de l'affection associée à celle-ci, éventuellement dans laquelle le symptôme est choisi dans le groupe constitué d'un ou de plusieurs des symptômes suivants : acuité visuelle réduite, éventuellement évaluée par la meilleure acuité visuelle corrigée (BCVA) ; augmentation de l'épaisseur rétinienne maculaire ; augmentation du volume maculaire ; et augmentation de la zone rétinienne de non-perfusion (RANP).

12. Molécule comprenant, ou constituée de, la protéine Annexine A5, destinée à être utilisée dans :
a) la prophylaxie ou le traitement d'une maladie, d'un trouble ou d'une affection associée au VEGF qui est choisi dans le groupe constitué de l'œdème maculaire, OVR, d'une rétinopathie associée à une maladie oculaire cécitante, choisie dans le groupe constitué de de la dégénérescence maculaire liée à l'âge (DMLA) et de la rétinopathie diabétique et de la rétinopathie hypertensive chez un sujet :
b) l'amélioration d'un traitement anti-VEGF ; et/ou
c) la réduction de complications associées à l'administration d'un agent anti-VEGF, choisie dans le groupe constitué de la réduction ou de la prévention du développement par le sujet d'une réponse réduite et/ou d'une résistance à un traitement anti-VEGF en cours ; de la réduction ou de la prévention d'une toxicité oculaire et/ou systémique lors d'un traitement anti-VEGF ; et/ou de la réduction ou de la prévention d'un ou de plusieurs des symptômes suivants : endophtalmie infectieuse, inflammation intraoculaire stérile, vascularite rétinienne et/ou pics transitoires de pression intraoculaire (PIO).

13. Agent anti-VEGF destiné à être utilisé dans la prophylaxie ou le traitement de l'œdème maculaire, ou d'une affection associée à celui-ci, chez un sujet, en association avec une molécule comprenant, ou constituée de, la protéine Annexine A5
dans lequel l'affection associée à l'œdème maculaire est choisie dans le groupe constitué : de l'occlusion de la veine rétinienne (OVR), de la dysrégulation de la barrière hémato-rétinienne (BHR), notamment de la BHR interne et/ou externe, du dysfonctionnement des barrières entre le corps vitré et les vaisseaux sanguins au niveau du corps ciliaire, des maladies oculaires associées à l'œdème maculaire (rétinopathie diabétique, uvéite, inflammation post-opératoire de la cataracte [œdème maculaire pseudophaque ou syndrome d'Irvine-Gass], de la dystrophie rétinienne, des réactions médicamenteuses, des tumeurs intraoculaires, de la choriorétinopathie séreuse centrale, de la rétinopathie radique et d'autres anomalies vasculaires rétiniennes, y compris les macroanévrismes artériels rétiniens et les télangiectasies rétiniennes), des modifications des membranes néovasculaires sous-rétiniennes observées dans des conditions choisies dans le groupe constitué de la dégénérescence maculaire liée à l'âge (DMLA), de la métamorphopsie, de la micropsie, de la vision floue, du scotome central et de la diminution de la sensibilité aux contrastes ou aux couleurs, éventuellement dans lequel la molécule est constituée de l'Annexine A5 monomère, éventuellement dans lequel :
(a) la molécule comprenant, ou constituée de, la protéine Annexine A5 ou une composition comprenant ladite molécule, est présentée dans la même composition que l'agent anti-VEGF ; ou
(b) la molécule comprenant, ou constituée de, la protéine Annexine A5 ou une composition comprenant ladite molécule est formulée séparément de l'agent anti-VEGF, par exemple dans lequel l'utilisation comprend l'administration simultanée ou séquentielle de la molécule comprenant, ou constituée de, la protéine Annexine A5 et l'agent anti-VEGF au sujet.

14. Agent anti-VEGF destiné à être utilisé dans la prophylaxie ou le traitement de l'occlusion de la veine rétinienne (OVR), ou d'une affection associée à celle-ci, chez un sujet, lorsque son utilisation est sous forme d'une thérapie combinée avec une molécule comprenant, ou constituée de, la protéine Annexine A5,
dans lequel ladite affection associée à l'OVR est choisie dans le groupe constitué de l'œdème maculaire, de l'ischémie rétinienne, de la néovascularisation irienne (éventuellement **caractérisée par** une augmentation des cytokines, comme une augmentation du VEGF), de la néovascularisation rétinienne (éventuellement **caractérisée par** une augmentation des cytokines, comme une augmentation du VEGF), du glaucome néovasculaire, de l'hémorragie vitréenne, de la rubéose et du décollement de la rétine, et
dans lequel une dose de la molécule comprenant, ou constituée de, la protéine Annexine A5 est administrée au sujet par voie systémique ou par injection suprachoroïdienne, quotidiennement pendant deux jours consécutifs ou plus, et
éventuellement dans lequel :
(a) la molécule comprenant, ou constituée de, la protéine Annexine A5 ou une composition comprenant ladite molécule, est présentée dans la même composition que l'agent anti-VEGF ; ou
(b) la molécule comprenant, ou constituée de, la protéine Annexine A5 ou une composition comprenant ladite molécule est formulée séparément de l'agent anti-VEGF, par exemple dans lequel l'utilisation comprend l'administration simultanée ou séquentielle de la molécule comprenant, ou constituée de, la protéine Annexine A5 et l'agent anti-VEGF au sujet.

15. Agent anti-VEGF destiné à être utilisé selon la revendication 13 ou 14, dans lequel l'agent anti-VEGF est choisi dans le groupe constitué de Ranibizumab, Aflibercept, Bevacizumab, Brolucizumab et Faricimab.
